(19) Europäisches Patentamt
European Patent Office
Office européen des brevets

(11) Publication number: **0 596 125 A1**

(12) **EUROPEAN PATENT APPLICATION published in accordance with Art. 158(3) EPC**

(21) Application number: **93900390.1**

(22) Date of filing: **24.12.92**

(86) International application number: **PCT/JP92/01695**

(87) International publication number: **WO 93/13105 (08.07.93 93/16)**

(51) Int. Cl.5: **C07D 495/04**, A61K 31/395

(30) Priority: **26.12.91 JP 359547/91**
**23.10.92 JP 309388/92**

(43) Date of publication of application: **11.05.94 Bulletin 94/19**

(84) Designated Contracting States: **AT BE CH DE DK ES FR GB GR IE IT LI LU MC NL PT SE**

(71) Applicant: **YOSHITOMI PHARMACEUTICAL INDUSTRIES, LTD.**
**6-9, Hiranomachi 2-chome**
**Chuo-ku**
**Osaka-shi Osaka 541(JP)**

(72) Inventor: **NAKAO, Tohru, Yoshitomi Pharmaceutical Ind. Ltd.**
**Central Research Laboratories**
**955, Oaza-koiwai**
**Yoshitomimachi Chikujo-gun, Fukuoka 871(JP)**
Inventor: **ONO, Yuji, Yoshitomi Pharmaceutical Ind., Ltd.**
**Central Research Laboratories**
**955, Oaza-koiwai**
**Yoshitomimachi Chikujo-gun, Fukuoka 871(JP)**
Inventor: **BOUGAUCHI, Masahiro Yoshitomi Pharmaceutical Ind. Ltd.**
**Central Research Lab.**
**955, Oaza-koiwai**
**Yoshitomimachi Chikujo-gun, Fukuoka 871(JP)**
Inventor: **MORIMOTO, Yasuto Yoshitomi Pharmaceutical Ind.Ltd**
**6-9, Hiranomachi 2-chome**
**Chuo-ku**
**Osaka-shi**
**Osaka 541(JP)**

(74) Representative: **VOSSIUS & PARTNER**
**Siebertstrasse 4**
**D-81675 München (DE)**

(54) **CONDENSED THIOPHENE COMPOUND AND PHARMACEUTICAL USE THEREOF.**

(57) A condensed thiophene compound represented by general formula (I) or a pharmaceutically acceptable salt thereof, wherein ring S represents a thiophene ring; $R^1$ represents hydrogen, halogen, alkyl, etc.; $R^2$ represents hydrogen, alkyl, acyl, etc.; G represents $-CH_2-$, -CH(OH)-, -CO-, etc. Q represents alkylene; T represents - $NH_2$, -NHRa (wherein Ra represents alkyl, cycloalkyl, etc.) or -NRbRc (wherein Rb and Rc represent each alkyl, etc., or alternatively Rb and Rc are combined together to form cyclic amino); D represents $-CH_2-$ or -S-; A and B represent each carbonyl or thiocarbonyl, or are null; and m and n represent each 0, 1 to 4, provided that m + n represents an integer of 4 or less. This compound is useful as an antipsychotic drug having a reduced extrapyramidal side effect or an antianxiety drug.

EP 0 596 125 A1

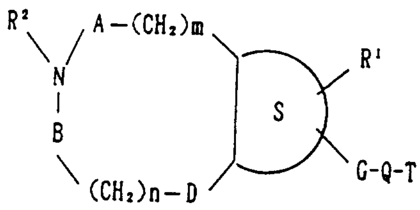

(I)

[Technical Field]

The present invention relates to novel and pharmaceutically useful condensed thiophene compounds or salts thereof and pharmaceutical uses thereof, and further synthetic intermediates for said condensed thiophene compounds.

[Background Art]

The following compounds and the method of preparation thereof are disclosed in (1) Chemical Abstracts, vol. 55, column 23488 (1961), (2) Nippon Kagaku Zassi, vol. 83-3, pp. 343-347 (1962), [Chemical Abstracts, vol. 59, column 3862 (1963)], (3) Chemical Abstracts, vol. 64, column 586 (1966), (4) Chemical Abstracts, vol. 69, 18565x (1968), (5) US Patent 4, 735, 940, column 39, lines 50-51 and (6) Journal of Heterocyclic Chemistry, vol. 13, pp. 1347-1349 (1976).

①

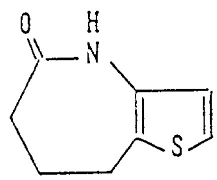

②

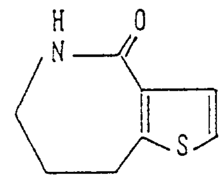

③

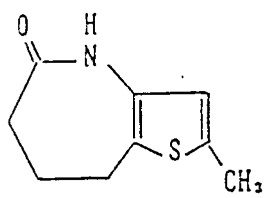

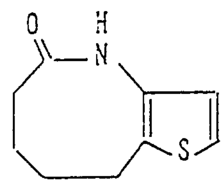

④

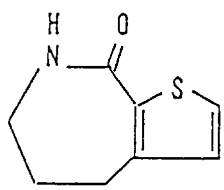

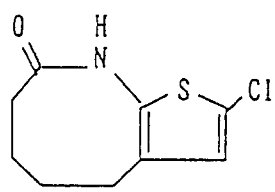

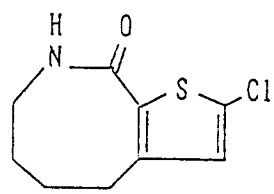

⑤

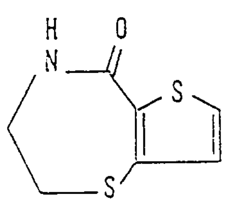

⑥

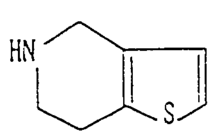

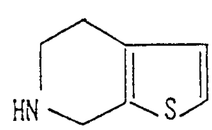

On the other hand, compounds having antipsychotic activity are known by patent publications such as JP-A S60-84282, JP-A S59-227882, JP-A S58-90583, JP-A S63-132887, JP-A S63-183576, JP-A H1-199967 and JP-A H4-308584.

Up to the present, there have been developed a large number of psychotropic agents for the therapy of schizophrenia, anxiety neurosis, mania, etc. Yet, most of the drugs for the treatment of schizophrenia accompany various extrapyramidal symptoms (excessive sedation, addiction, dyskinesia, Perkinson's syndrome) and dysendocriniasis (increase of serum prolactin level) upon continuous administration, which cause problems of unavoidable side effects in medical treatments. Further, these antipsychotic drugs are effective on so called positive symptoms like hallucination, delusion or the like as well as on psychomotor excitement but not effective on negative symptoms like apathy, abulia, disorder of cognition and so on. Accordingly, it is desired to develop antipsychotic drugs having high safety which are expected to improve negative symptoms in addition to positive symptoms, with less occurrence of extrapyramidal symptoms and reduced side effects in endocrine system. On the other hand, drugs containing benzodiazepine compounds have been widely used as antianxietic agents for the treatment of anxiety neurosis and so on. Though these compounds have potent anxiolytic action, they also exhibit side effects such as muscle-relaxation effect, sedative action and drug dependence. Consequently, the development of antianxietic drugs having high safety, no habit-forming property and no probability of abuse have been desired.

[Disclosure of Invention]

The present inventors have found that novel condensed thiophene compounds have high affinities for serotonin and dopamine receptors and are useful as potent psychotropic agents according with the above-mentioned purposes, and completed the present invention.

The present invention provides:

1. a condensed thiophene compound of the formula:

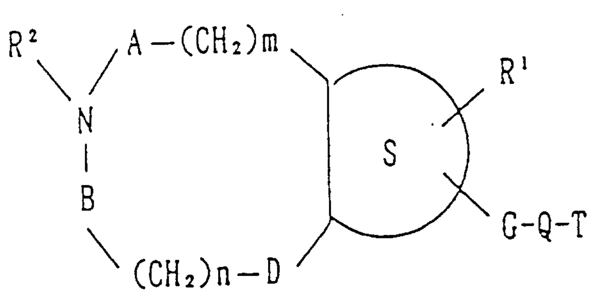

(I)

or a pharmaceutical acceptable salt thereof.

In the above formula, the ring S means following condensed thiophenes.

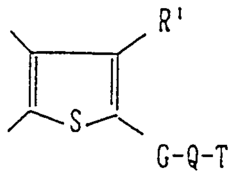 , 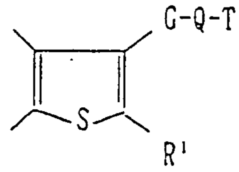

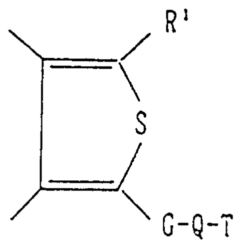 , 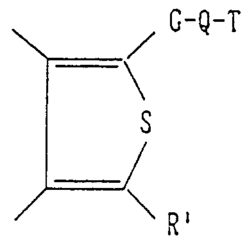

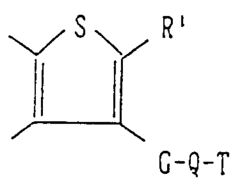 , 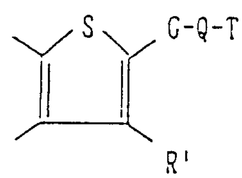

$R^1$ is hydrogen, halogen, nitro, amino, cyano, hydroxyl, formyl, alkyl, alkoxy, haloalkyl, arylalkyl, acyl, acyloxy, alkoxyalkyl, acyloxyalkyl, hydroxyalkyl, acyloxyalkanoyl, alkoxyalkanoyl, hydroxyalkanoyl, aryloxyalkanoyl, haloalkanoyl, alkylthio, alkylsulfinyl, alkylsulfonyl, arylthio, arylsulfinyl, arylsulfonyl, hydroxysulfonyl, halosulfonyl, sulfamoyl, substituted sulfamoyl, carboxyl, acylamino, alkoxycarbonyl, carbamoyl, substituted carbamoyl or substituted amino.

$R^2$ is hydrogen, alkyl, acyl, aryl or arylalkyl.

G is $-CH_2-$, $-CH(OR^3)-$ ($R^3$ is hydrogen, alkyl or acyl), -CO-, $-C(=NOR^4)-$ ($R^4$ is hydrogen, alkyl or acyl), $-CH(NH_2)-$ or -S(O)t- (t is 0, 1 or 2).

Q is straight alkylene or branched chain alkylene.

T is a primary amino, a secondary amino or a tertiary amino.

D is $-CH_2-$ or -S(O)u- (u is 0, 1 or 2).

A and B are (i) the same or different and each is carbonyl or thiocarbonyl, (ii) one is absent and the other is carbonyl or thiocarbonyl, or (iii) both absent.

When A, B are the case of above-mentioned (i) or (ii), m is 0 or 1, and n is 0, 1 or 2. When A, B are the case of above-mentioned (iii), m and n are the same or different and each is 0 or an integer of 1 to 4 with the proviso that m + n is an integer of below 4;

2. the compound of the above-mentioned item 1 or a pharmaceutically acceptable salt thereof, wherein T is a primary amino of $-NH_2$, a secondary amino of -NHRa (Ra is alkyl, cycloalkyl, arylalkyl or heteroarylalkyl), or a tertiary amino of -N(Rb)(Rc) [Rb and Rc are the same or different and each is alkyl, cycloalkyl, arylalkyl or heteroarylalkyl, or Rb and Rc together with the adjacent nitrogen atom form a cyclic amino of the formula:

(1)

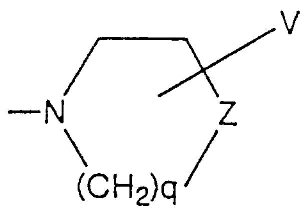

or

(2)

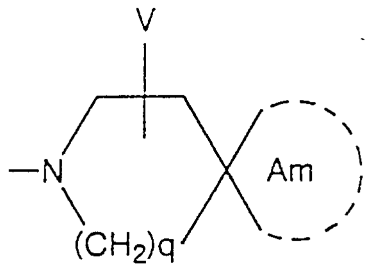

wherein q is an integer of 1 to 4, Z is methylene, oxygen atom, sulfur atom or N-$R^5$ ($R^5$ is hydrogen, carbamoyl, substituted carbamoyl, alkyl, cyanoalkyl, hydroxyalkyl, aryl, arylalkyl, alkoxycarbonyl, diarylalkyl, heteroaryl, heteroarylalkyl, cycloalkyl, cycloalkylalkyl, acyl, cinnamyl or adamantanemethyl), substituent V is hydrogen, hydroxyl, amino, carbamoyl, mono or di-substituted amino, cyclic amino, acyl, aryl, arylalkyl, arylalkylamino, alkyl, alkoxy, hydroxyalkyl, alkoxycarbonyl, heteroaryl, heteroarylalkyl, phenoxyalkyl, anilinoalkyl, alkylaminoalkyl, alkanoylaminoalkyl or bisarylmethylene and the number of V is 1 to 4. Cyclic amino of formula (1) may contain carbonyl group in the cycle and further may be condensed with aryl or heteroaryl. Ring Am of formula (2) contain amido bond in the cycle and further may contain oxygen atom, sulfur atom, carbonyl and/or N-$R^6$ ($R^6$ is hydrogen, alkyl or phenyl). Further, the ring Am can be condensed with 5 to 7-membered saturated or unsaturated ring.];

3. the compound of the above-mentioned item 1 or a pharmaceutically acceptable salt thereof, wherein $R^1$ is hydrogen, halogen, nitro, amino, alkyl, alkoxy, acyl, acyloxy, alkylthio alkylsulfinyl, alkylsulfonyl or acylamino, G is $-CH_2-$, -CH($OR^3$)- ($R^3$ is hydrogen), -CO- or -S(O)t- ( t is 0, 1 or 2), T is a primary amino of $-NH_2$, a secondary amino of -NHRa (Ra is arylalkyl or heteroarylalkyl) or a tertiary amino of -N(Rb)(Rc) [Rb and Rc are the same or different and each is alkyl, arylalkyl or heteroarylalkyl, or Rb and Rc together with the adjacent nitrogen atom form a cyclic amino of the formula:

(1)

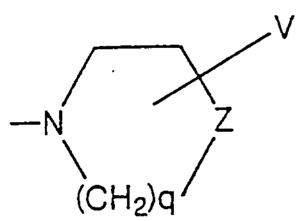

or

(2)

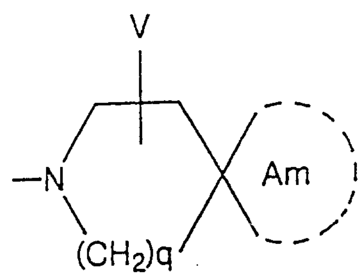

wherein q is an integer of 1 to 4, Z is methylene, oxygen atom, sulfur atom or N-$R^5$ ($R^5$ is hydrogen, carbamoyl, substituted carbamoyl alkyl, aryl, diarylalkyl, heteroaryl, heteroarylalkyl or acyl), substituent V is hydrogen, hydroxyl, heteroaryl, heteroarylalkyl or bisarylmethylene and the number of V is 1 to 4. Cyclic amino of formula (1) may contain carbonyl group in the cycle and further may be condensed with aryl or heteroaryl. Ring Am of formula (2) contain amido bond in the cycle and further may contain oxygen atom, sulfur atom, carbonyl and/or N-$R^6$ ($R^6$ is hydrogen, alkyl or phenyl). Further, the ring Am can be condensed with 5 to 7-membered saturated or unsaturated ring.];

4. the compound of the above-mentioned item 1 or a pharmaceutically acceptable salt thereof, wherein $R^1$ is hydrogen, halogen, alkyl or acyl, G is $-CH_2-$, -CH($OR^3$)- ($R^3$ is hydrogen), -CO- or -S(O)t- (t is 0, 1 or 2), T is a tertiary amino of -N(Rb)(Rc) [Rb and Rc are the same or different and each is alkyl, or Rb and Rc together with the adjacent nitrogen atom form a cyclic amino of the formula:

(1)

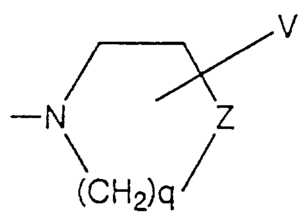

or

(2)

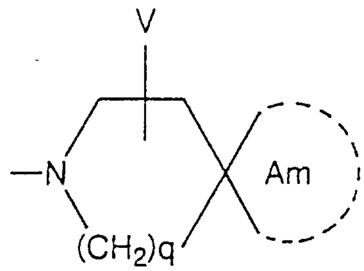

wherein q is an integer of 1 to 4, Z is methylene, or N-$R^5$ ($R^5$ is aryl, diarylalkyl, heteroaryl or heteroarylalkyl), substituent V is hydrogen, hydroxyl, heteroaryl, heteroarylalkyl or bisarylmethylene and the number of V is 1 to 4. Cyclic amino of formula (1) may contain carbonyl group in the cycle and further may be condensed with aryl or heteroaryl. Ring Am of formula (2) contain amido bond in the cycle and further may contain oxygen atom, sulfur atom, carbonyl and/or N-$R^6$ ($R^6$ is hydrogen, alkyl or phenyl). Further, the ring Am can be condensed with 5 to 7-membered saturated or unsaturated ring.];

5. preferable compound of the above-mentioned item 1 selected from the group consisting of:

2-(2-(4-(6-fluoro-1,2-benzisoxazol-3-yl)piperidin-1-yl)ethyl)-4,6,7,8-tetrahydro-5H-thieno[3,2-b]azepin-5-one,

3-(2-(4-(6-fluoro-1,2-benzisoxazol-3-yl)piperidin-1-yl)ethyl)-2-methyl-4,6,7,8- tetrahydro-5H-thieno[3,2-b]-azepin-5-one,

2-(2-(4-(6-fluoro-1,2-benzisoxazol-3-yl)piperidin-1-yl)ethyl)-4,5,6,7-tetrahydro-8H-thieno[2,3-c]azepin-8-one,

2-(4-(4-(6-fluoro-1,2-benzisoxazol-3-yl)piperidin-1-yl)butyryl)-4,5,6,7-tetrahydro-8H-thieno[2,3-c]azepin-8-one,

6-acetyl-3-(2-(4-(6-fluoro-1,2-benzisoxazol-3-yl)piperidin-1-yl)ethyl)-4,5,6,7-tetrahydro-2-methylthieno[2,3-c]pyridine,

6-acetyl-2-ethyl-3-(2-(4-(6-fluoro-1,2-benzisoxazol-3-yl)piperidin-1-yl)ethyl)-4,5,6,7-tetrahydrothieno[2,3-c]-pyridine,

6-acetyl-2-(2-(4-(6-fluoro-1,2-benzisoxazol-3-yl)piperidin-1-yl)ethyl)-4,5,6,7-tetrahydro-3-methylthieno[2,3-c]pyridine,

6-acetyl-2-(4-(4-(6-fluoro-1,2-benzisoxazol-3-yl)piperidin-1-yl)butyryl)-4,5,6,7-tetrahydrothieno[2,3-c]-pyridine,

3-(2-(4-(6-fluoro-1,2-benzisoxazol-3-yl)piperidin-1-yl)ethyl)-4,5,6,7-tetrahydro-2-methyl-6-propionylthieno-[2,3-c]pyridine,

6-cyclopropylcarbonyl-3-(2-(4-(6-fluoro-1,2-benzisoxazol-3-yl)piperidin-1-yl)ethyl)-4,5,6,7-tetrahydro-2-methylthieno[2,3-c]pyridine,

3,6-diacetyl-2-(2-(4-(6-fluoro-1,2-benzisoxazol-3-yl)piperidin-1-yl)ethyl)-4,5,6,7-tetrahydrothieno[2,3-c]-pyridine, and

6-acetyl-3-(2-(4-(6-fluorobenzo(b)thiophen-3-yl)piperidin-1-yl)ethyl)-4,5,6,7-tetrahydro-2-methylthieno[2,3-c]pyridine

or a pharmaceutically acceptable salt thereof;

6. a condensed thiophene compound of the formula:

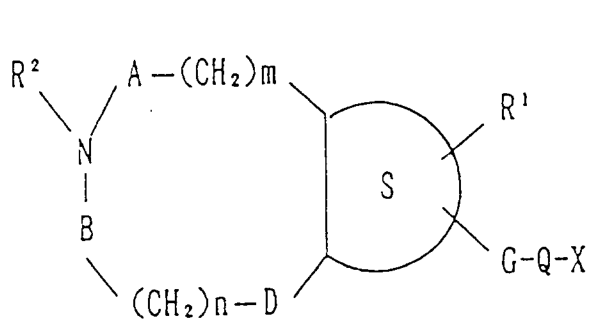

(II)

wherein X is hydroxyl, or a reactive atom or group derived from hydroxyl (halogen, methanesulfonyloxy, p-toluenesulfonyloxy and so on), and other symbols are as defined in the above-mentioned item 1;

7. a pharmaceutical composition comprising a compound of the above-mentioned item 1 and pharmaceutical additives;

8. an antipsychotic drug containing a compound of the above-mentioned item 1 as an effective ingredient;

9. an antianxietic drug containing a compound of the above-mentioned item 1 as an effective ingredient.

In the definitions of the above symbols and in the present specification, halogen means chlorine, bromine, fluorine, iodine; alkyl means, for example, methyl, ethyl, propyl, isopropyl, butyl, isobutyl, tert-butyl, pentyl, hexyl, heptyl, octyl, decyl, hexadecyl or octadecyl; alkoxy means, for example, methoxy, ethoxy, propoxy, isopropoxy, butoxy, isobutoxy, tert-butoxy, pentyloxy hexyloxy, heptyloxy or octyloxy; haloalkyl means alkyl substituted by halogen, for example, bromoethyl, chloromethyl, trifluoromethyl, 2-bromoethyl, 2-chloroethyl, difluoromethyl, 2,2-difluoroethyl, 2,2,2-trifluoroethyl, 3-bromopropyl, 3-chloropropyl or 4-fluorobutyl; arylalkyl means, for example, benzyl, 1-phenylethyl 2-phenylethyl, 3-phenylpropyl, 4-phenylbutyl, naphthylmethyl, 2-naphthylethyl, 3-naphthylpropyl, 4-naphthylbutyl, diphenylmethyl or bis (4-fluorophenyl)methyl; acyl means, for example, alkanoyl such as acetyl, propionyl, butyryl, isobutyryl, pentanoyl, hexanoyl or octanoyl, aroyl such as benzoyl or naphthoyl, heteroarylcarbonyl such as nicotinoyl, thenoyl or furoyl, or cycloalkylcarbonyl such as cyclopropylcarbonyl, cyclopentylcarbonyl, cyclohexylcarbonyl or cycloheptylcarbonyl.

Acyloxy means, for example, acetoxy, propionyloxy butyryloxy, isobutyryloxy or benzoyloxy; alkoxyalkyl means, for example, methoxymethyl, 1- or 2-methoxyethyl, 1-, 2- or 3-methoxypropyl, 1-, 2-, 3- or 4-methoxybutyl, ethoxymethyl, 1- or 2-ethoxyethyl, 1-, 2- or 3-ethoxypropyl or 1-, 2-, 3-, or 4-ethoxybutyl; acyloxyalkyl means, for example, acetoxymethyl propionyloxymethyl, 1- or 2-acetoxyethyl, 1- or 2-propionyloxyethyl, 1-, 2- or 3-acetoxypropyl, 1-, 2- or 3-propionyloxypropyl, benzoyloxymethyl, 1- or 2-benzoyloxyethyl, 1-, 2- or 3-benzoyloxypropyl or 1-, 2-, 3-, or 4-benzoyloxybutyl; hydroxyalkyl means, for example, hydroxymethyl, 1- or 2-hydroxyethyl, 1-, 2- or 3-hydroxypropyl or 1-, 2-, 3- or 4-hydroxybutyl; acyloxyalkanoyl means, for example, acetoxyacetyl, acetoxypropionyl, acetoxybutyryl, benzoyloxyacetyl, benzoyloxypropionyl or benzoyloxybutyryl; alkoxyalkanoyl means, for example, methoxyacetyl, ethoxyacetyl, propoxyacetyl, butoxyacetyl, methoxypropionyl, ethoxypropionyl, propoxypropionyl or butoxypropionyl; hydroxyalkanoyl means, for example, hydroxyacetyl, hydroxypropionyl or hydroxybutyryl; aryloxyalkanoyl means, for example, phenoxyacetyl, phenoxypropionyl or phenoxybutyryl; haloalkanoyl means, for example, bromoacetyl, chloroacetyl, bromopropionyl, chloropropionyl, bromobutyryl or chlorobutyryl; alkylthio means, for example, methylthio, ethylthio, propylthio, isopropylthio, butylthio, isobutylthio or tert-butylthio; alkylsulfonyl means, for example, methylsulfonyl, ethylsulfonyl, propylsulfonyl, isopropylsulfonyl, butylsulfonyl isobutylsulfonyl or tert-butylsulfonyl; halosulfonyl means, for example, chlorosulfonyl, bromosulfonyl or iodosulfonyl; substituted sulfamoyl means, for example, dimethylsulfamoyl, diethylsulfamoyl, dipropylsulfamoyl, dibutylsulfamoyl, piperidinosulfonyl or morpholinosulfonyl; alkylsulfinyl means, for example, methylsulfinyl, ethylsulfinyl propylsulfinyl or butylsulfinyl; arylthio means, for example, phenylthio or naphthylthio; arylsulfinyl means, for example, phenylsulfinyl or naphthylsulfinyl; arylsulfonyl means, for example, phenylsulfonyl or naphthyisulfonyl; acylamino means, for example, acetylamino, propionylamino butyrylamino or benzoylamino; alkoxycarbonyl means, for example, methoxycarbonyl, ethoxycarbonyl, propoxycarbonyl, isopropoxycarbonyl, butoxycarbonyl or isobutoxycarbonyl; substituted carbamoyl means, for example, methylcarbamoyl, ethylcarbamoyl, dimethylcarbamoyl, diethylcarbamoyl, cyclohexylcarbamoyl or piperidinocarbonyl; substituted amino means, for example, methylamino, dimethylamino, ethylamino, diethylamino, propylamino, dipropylamino, N-methyl-N-benzylamino or piperidino; straight alkylene means, for example, methylene, ethylene, trimethylene, tetramethylene, pentamethylene, hexamethylene, heptamethylene, octamethylene or decamethylene; branched chain alkylene means, for example, alkylene substituted by at least one, preferably 1 to 4 alkyl(s) such as propylene, 1-methyltrimethylene, 3-methyltrimethylene 1-methyltetramethylene, 4-methyltetramethylene 1,4-dimethyltetramethylene 6-methylhexamethylene or 4,4-dimethyltetramethylene.

In the formula (I), T is a primary amino of $-NH_2$, a secondary amino of -NHRa or a tertiary amino of -N(Rb)(Rc). Wherein Ra is alkyl (same as the above), cycloalkyl (e.g. cyclopropyl, cyclobutyl, cyclopentyl, cyclohexyl or cycloheptyl), arylalkyl (same as the above) or heteroarylalkyl (which may optionally be hydrogenated and is exemplified by pyridylmethyl furylmethyl, thienylmethyl or (1,4-benzodioxan-2-yl)-methyl) Rb and Rc are the same or different and each is alkyl (same as the above), cycloalkyl (same as the above), arylalkyl (same as the above) or heteroarylalkyl (same as the above), and -N(Rb)(Rc) is exemplified by dialkylamino (e.g. dimethylamino, diethylamino dipropylamino, diisoproylamino dibutylamino, dihexylamino, dioctylamino), N-alkyl-N-cycloalkylamino (e.g. N-methyl-N-cyclopropylamino, N-methyl-N-cyclohexylamino, N-methyl-N-cyclopentylamino, N-ethyl-N-cyclopropylamino N-ethyl-N-cyclopentylamino N-ethyl-N-cyclohexylamino, N-propyl-N-cyclopropylamino, N-propyl-N-cyclohexylamino, N-butyl-N-cyclohexylamino), N-alkyl-N-arylalkylamino (e.g. N-methyl-N-benzylamino, N-methyl-N-(2-phenylethyl)-amino, N-methyl-N-(3-phenylpropyl)amino, N-ethyl-N-benzylamino, N-ethyl-N-(2-phenylethyl)amino, N-propyl-N-benzylamino N-propyl-N-(2-phenylethyl)amino N-butyl-N-benzylamino, N-butyl-N-(2-phenylethyl)-amino) or N-alkyl-N-heteroarylalkylamino which may have a substituent on the aromatic ring (e.g. N-methyl-N-pyridylmethylamino N-methyl-N-thienylmethylamino, N-methyl-N-furylmethylamino, N-ethyl-N-pyridylmethylamino, N-ethyl-N-thienylmethylamino, N-ethyl-N-furylmethylamino, N-methyl-N- (1,4-benzodioxan-2-ylmethyl)amino), or Rb and Rc together with the adjacent nitrogen atom form a cyclic amino of the formula:

(1)

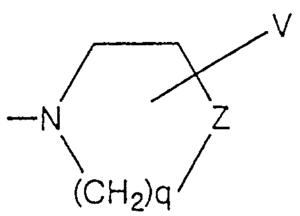

or

(2)

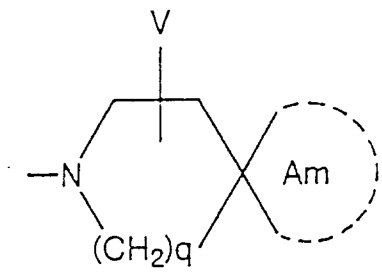

wherein q is an integer of 1 to 4, Z is methylene, oxygen atom, sulfur atom or N-$R^5$. Substituent V is hydrogen, hydroxyl amino, carbamoyl, mono or di-substituted amino (e.g. methylamino, dimethylamino, ethylamino, diethylamino anilino, N-acetylanilino N-propionylanilino or pyrrolidinylamino), cyclic amino (e.g. pyrrolidinyl, piperidino, hexamethyleneimino, morpholino, thiomorpholino, piperazinyl, homo-piperazinyl, 4-substituted-piperazinyl or 4-substituted-homopiperazinyl) acyl (same as the above), aryl (e.g. phenyl, naphthyl), arylalkyl (same as the above), arylalkylamino (e.g. benzylamino, phenylethylamino, naphthylmethylamino or naphthylethylamino), alkyl (same as the above), alkoxy (same as the above), hydroxyalkyl (same as the above), alkoxycarbonyl (same as the above), heteroaryl (e.g. pyridyl, thienyl, furyl, pyrimidinyl, 1,2-benzoisothiazol-3-yl, 1,2-benzisoxazol-3-yl, benzothiophen-2- or 3-yl, benzofuran-2- or 3-yl, quinolyl, isoquinolyl, benzoxazol-2-yl, pyrazinyl, pyridazinyl, imidazolyl, 1H-indazol-3-yl, thieno[3,2-c]pyridin-4-yl, furo[3,2-c]pyridin-4-yl, 2-oxo-1-benzimidazolyl, 2-thioxo-1-benzimidazolyl, 2,4-dioxohexahydropyrimidin-1-yl, hydantoin-1-yl), heteroarylalkyl (same as the above), phenoxyalkyl (e.g. phenoxymethyl, 2-phenoxyethyl, 3-phenoxypropyl), anilinoalkyl (e.g. anilinomethyl, 2-anilinoethyl, 3-anilinopropyl), alkylaminoalkyl (e.g. N-methylaminomethyl, N,N-dimethylaminomethyl, N,N-diethylaminomethyl, 2-(N-methylamino)ethyl, 2-(N,N-dimethylamino)ethyl), alkanoylaminoalkyl (e.g. N-acetylaminomethyl N-propionylaminomethyl, N-butyrylaminomethyl, 2-(N-acetylamino)ethyl or bisarylmethylene (e.g. bis(4-fluorophenyl)methylene, bis(4-chlorophenyl)methylene) and the number of V is 1 to 4.

$R^5$ of N-$R^5$ is hydrogen, carbamoyl, substituted carbamoyl (same as the above), alkyl (same as the above), cyanoalkyl (e.g. cyanomethyl 2-cyanoethyl, 3-cyanopropyl, 4-cyanobutyl), hydroxyalkyl (same as the above), aryl (same as the above), arylalkyl (same as the above), alkoxycarbonyl (same as the above), diarylalkyl (e.g. diphenylmethyl, bis(4-fluorophenyl)methyl, 2,2-diphenylethyl, 2,2-bis(4-fluorophenyl)ethyl), heteroaryl (same as the above), heteroarylalkyl (same as the above), cycloalkyl (same as the above), cycloalkylalkyl (e.g. cyclopropylmethyl, cyclobutylmethyl, cyclohexylmethyl, cycloheptylmethyl), acyl (same as the above), cinnamyl or admantanemethyl.

Cyclic amino of formula (I) may contain carbonyl group in the cycle and further may be fused with aryl (e.g. benzene, naphthalene) or heteroaryl (e.g. furan, thiophene, pyridine, quinoline) to form fused cyclic amino such as 1,2,3,4-tetrahydroisoquinolin-2-yl or phthalimido. Ring Am of formula (2) contain amido bond in the cycle and further may contain oxygen atom, sulfur atom, carbonyl and/or N-$R^6$ ($R^6$ is hydrogen, alkyl or phenyl). The ring Am having amido bond in the cycle includes, for example, thiazolidinone, imidazolidinone, pyrazolidinone or pyrrolidinone. Further, the ring Am can be fused with 5 to 7-membered saturated or unsaturated ring to form, for example, 2-oxo-1,2,3,5,6,7,8,8a-octahydroimidazo[1,2-a]pyridine-3-spiro-4'-piperidino.

In the above definitions, the aryl and heteroaryl may optionally be substituted by 1 to 3 substituents (e.g. halogen, nitro, amino, cyano, haloalkyl, hydroxyl, alkyl, alkoxy or alkenyl) on the aromatic ring.

Preferable embodiments of T are exemplified by (6-fluoro-1,2-benzisoxazol-3-yl)piperidin-1-yl, (5-fluoro-1,2-benzisoxazol-3-yl)piperidin-1-yl, (6-fluoro-1,2-benzisothiazol-3-yl)piperidin-1-yl, (1,2-benzisothiazol-3-yl)-piperidin-1-yl, (6-fluorobenzo(b)thiophen-3-yl)piperidin-1-yl, (6-fluoro-1H-indazol-3-yl)piperidin-1-yl, (6-fluoro-1-(4-fluorophenyl)-1H-indazol-3-yl)piperidin-1-yl, 4-(1,2-benzisoxazol-3-yl)piperazin-1-yl 4-(1,2-benzisothiazol-3-yl)piperazin-1-yl, 4-(6-chlorobenzothiazol-2-yl)piperazin-1-yl and so on.

The salt of the compound of formula (I) include an acid addition salt such as hydrochloride, hydrobromide, phosphate, sulfate, benzenesulfonate, methanesulfonate, citrate, lactate, maleate, fumarate or tartarate. When the compound of formula (I) contain carboxyl as a substituent, the salt includes a metal salt such as sodium salt, potassium salt, calcium salt, aluminum salt, or an amine salt with triethylamine, or bibasic amino acid salt with lysine etc. The pharmaceutically acceptable salts thereof are preferable The present invention also includes a hydrate and a solvate of the compound of formula (I).

The compounds of formula (I) or (II) having a chiral carbon atom can be prepared as a racemate or an optically active isomer, and the compound having at least two chiral atoms can be obtained as an individual diastereomer or a mixture thereof. The present invention embraces the mixture thereof and the individual isomers. Furthermore, the present invention embraces stereomers.

The methods for preparing the compounds of present invention are described as follows:

Method (1)

The compound of formula (I) can be synthesized by reacting the compound of formula (II) with a compound of the formula:

H-T (III)

wherein T is as defined above, or acid addition salt thereof.

The reaction is carried out in an inert solvent such as methanol, ethanol, propanol, benzene, toluene, dimethylformamide, tetrahydrofuran, acetonitrile or acetone in the presence of a suitable acid scavenger (e.g. potassium carbonate, sodium carbonate, sodium hydrogencarbonate, pyridine, triethylamine, sodium acetate or potassium acetate) at 20°C-150°C for 30 minutes to 30 hours.

When X in the compound of formula (II) is hydroxyl, the reaction is carried out in a suitable inert solvent such as dimethylformamide or benzene in the presence of an aminophosphoniurn reagent (e.g. N, N-methyl-phenylaminotriphenylphosphonium iodide) at 20°C-150°C for 30 minutes to 5 hours.

Method (2)

The compound of formula (I) can be synthesized by reacting a compound of the formula:

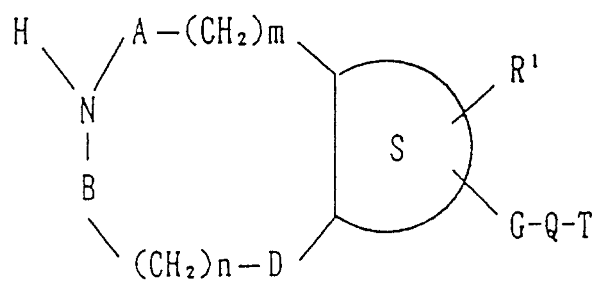

(IV)

wherein each symbol is as defined above, with a compound of the formula:

$X-R^2$ (V)

wherein each symbol is as defined above.

The reaction is carried out in an inert solvent such as methanol, ethanol, propanol, N,N-dimethylformamide benzene, toluene, acetonitrile, diethyl ether, tetrahydrofuran or n-hexane in the presence of a suitable base such as sodium methoxide, sodium ethoxide, potassium tert-butoxide, potassium hydride, sodium hydride, sodium hydroxide, potassium hydroxide, sodium carbonate or potassium carbonate at -20°C -150°C for 30 minutes to 5 hours

Method (3)

The synthetic intermediate compound of formula (II) wherein G is -CO-can be prepared by reacting a compound of the formula :

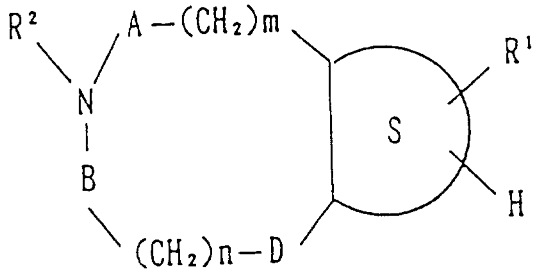

(VI)

wherein each symbol is as defined above, with a compound of the formula:

$Z^1$-CO-Q-X (VII)

wherein $Z^1$ is halogen and other symbols are as defined above.

The reaction is carried out in a suitable and inert solvent such as chloroform, dichloromethane or dichloroethane in the presence of a suitable Lewis acid (e.g. tin chloride, aluminum chloride, iron chloride, zinc chloride) at -10 ° C to 100 ° C for 30 minutes to 5 hours.

Method (4)

The synthetic intermediate compound of formula (II) wherein G is -S- can be prepared by lithiation of the compound of formula (VI) using a suitable alkyl-lithium, followed by reaction with a compound of the formula:

$X^1$-Q-$X^2$ (VIII)

wherein $X^1$ and $X^2$ are the same as the above-mentioned X, but both of them are not hydroxyl, in the presence of sulfur.

The reaction is carried out by lithiation using a suitable alkyl-lithium such as butyllithium or sec-butyllithium in a suitable and inert solvent such as diethyl ether or tetrahydrofuran at a temperature of from -78 ° C to the boiling point of the solvent employed, and followed by reaction with sulfur and the compound of formula (VIII).

Method (5)

The synthetic intermediate compound of formula (II) wherein G is -SO-or -$SO_2$- can be prepared by oxidizing the compound synthesized in Method (4) using a suitable oxidizing agent (e.g. sodium metaperiodate) in a suitable solvent.

Method (6)

The synthetic intermediate compound of formula (II) wherein G is -CH(OH)- or -$CH_2$- can be prepared by reducing the compound obtained in Method (3) wherein G is -CO- using a suitable reducing agent such as sodium borohydride.

The reaction is carried out in a suitable and inert solvent, for example, methanol, ethanol, propanol isoproyl alcohol, tetrahydrofuran, dichloromethane or dichloroethane, in the presence of a suitable reducing agent (e.g. lithium aluminum hydride, sodium borohydride, hydrogenated cyanoborohydride trifluoroboron diethylate, triethylsilane) at -10 ° C to 100 ° C for 30 minutes to 10 hours.

The compound of formula (I) wherein G is -CH (OH)- or -$CH_2$- can be prepared by reducing the compound of formula (I) wherein G is -CO- in a similar manner as the above-mentioned method.

Method (7)

The compound of formula (I) wherein G is -CH($OR^{3'}$)- ($R^{3'}$ is alkyl or acyl) can be prepared by reacting the compound of formula (I) wherein G is -CH(OH)- with a compound of the formula:

$R^{3'}$-$Z^2$ (IX)

wherein $Z^2$ is halogen and $R^{3'}$ is as defined above.

The reaction is carried out in a suitable and inert solvent such as methanol, ethanol, propanol, butanol, N,N-dimethylformamide, tetrahydrofuran, benzene or toluene in the presence of an acid scavenger (e.g. sodium hydride, sodium amide, sodium methoxide, sodium ethoxide, potassium hydroxide, sodium hydroxide) at room temperature to 150 °C for 1 to 20 hours.

Method (8)

The compound of formula (I) wherein G is -C(=NOH)- can be prepared by reacting a compound of the formula (I) wherein G is -CO- with a hydroxylamine.

The reaction is carried out by reacting hydroxylamine or its salt such as hydrochloride, hydrobromide or sulfate with the compound of formula (I) wherein G is -CO- in a suitable and inert solvent such as, preferably, alcohols such as methanol, ethanol, propanol, isopropyl alcohol or butanol, in the presence or absence of an acid scavenger such as an organic base (e.g. triethylamine, pyridine N,N-dimethylaniline) or inorganic base (e.g. sodium carbonate, potassium carbonate, sodium hydrogencarbonate). The reaction temperature and reaction time are not particularly limited, and the reaction is normally conducted at room temperature to a temperature near the boiling point of the solvent used for several to several dozens of hours.

Method (9)

The compound of formula (I) wherein G is -C(=$NOR^{4'}$)- ($R^{4'}$ is alkyl or acyl) can be prepared by reacting a compound of the formula (I) wherein G is -C(=NOH)- with a compound of the formula:

$R^{4'}$-$Z^3$ (X)

wherein $Z^3$ is halogen, and $R^{4'}$ is as defined above, in the same manner as in Method (7).

Method (10)

The compound of formula (I) wherein G is -CH($NH_2$)- can be prepared by subjecting a compound of the formula (I) wherein G is -C(=NOH)- to a reduction reaction using a suitable reducing agent.

The reduction reaction is carried out using a suitable catalyst such as palladium carbon, Raney-nickel, platinum or rhodium in a suitable and inert solvent such as alcohols (e.g. methanol, ethanol, propanol or isopropyl alcohol), or acids (e.g. formic acid, acetic acid ) by a catalytic hydrogenation under atmospheric pressure or pressurization.

The reaction temperature and reaction time are not particularly limited, and the reaction is usually conducted at room temperature to about 150 °C for several to several dozens of hours, or using a suitable reducing agent such as lithium aluminium hydride or tri-tert-butoxyaluminum hydride in a suitable and inert solvent, but preferably ethers such as diethyl ether, tetrahydrofuran, dioxane and dimethylcellosolve under cooling, at room temperature, or under heating for several to several dozens of hours.

Method (11)

The compound of formula (VI) wherein both of A and B are carbonyl groups, for example, the compound of formula:

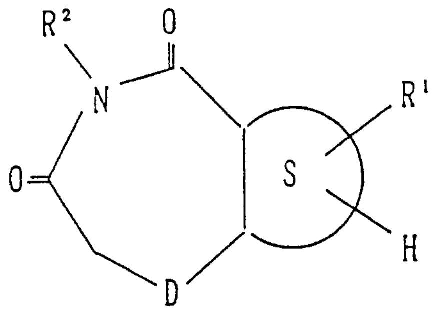

(XI)

wherein each symbol is as defined above, can be prepared by subjecting a compound of the formula:

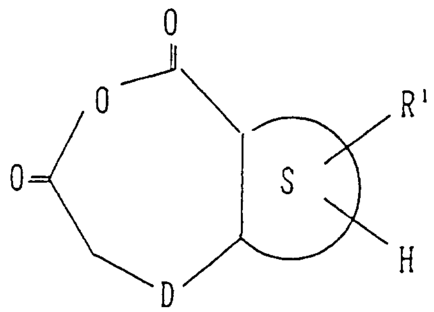

(XII)

wherein each symbol is as defined above, to a dehydrating reaction with a compound of the formula:

$H_2N-R^2$ (XIII)

wherein $R^2$ is as defined above.

The reaction is carried out in a suitable and inert solvent (e.g. acetic anhydride, toluene, benzene, chloroform, methylene chloride, pyridine, methanol, ethanol, propanol dimethylformamide, tetrahydrofuran) or without solvent at 20 °C to the boiling point of the solvent employed for 30 minutes to 10 hours.

Method (12)

The compound of formula (XII) can be prepared by reacting, for example, a compound of the formula :

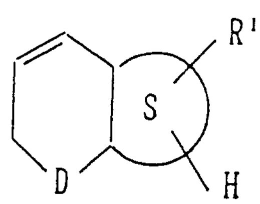

(XIV)

wherein each symbol is as defined above, with ozone and then subjecting a resulting compound of the formula:

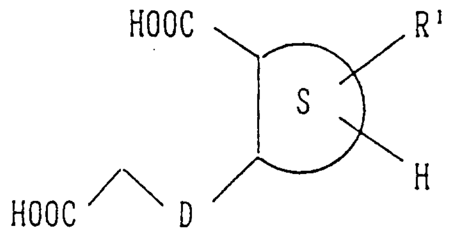

(XV)

wherein each symbol is as defined above, obtained by the oxidative treatment to a ring closure reaction with a dehydrating agent (e.g. phosphorus pentoxide, dicyclohexylcarbodiimide, N,N-carbonyldiimidazole, an acid anhydride, an acid halide, benzenesulfonyl chloride).

The reaction of the compound of formula (XII) with ozone is carried out in a suitable and inert solvent such as methanol, ethanol, propanol or tetrahydrofuran at -20 °C to 150 °C for 30 minutes to 10 hours.

The compound of formula (XII) can also be prepared by introducing a formyl group into, for example, a compound of the formula:

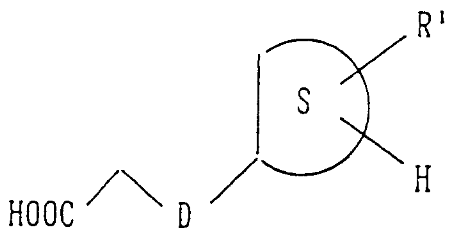

(XVI)

wherein each symbol is as defined above, by Vilsmeier's reaction or the like and oxidizing the formyl group of the compound of formula:

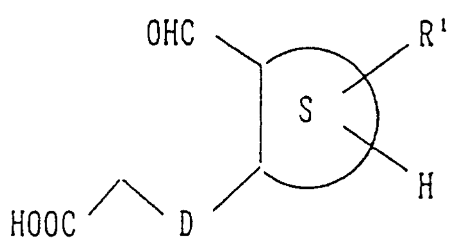

(XVII)

wherein each symbol is as defined above, by a conventional method employed in organic chemistry and then treating the resulting compound of formula (XV) in the same manner as the above-mentioned method.

The compound of formula (VI) wherein one of A and B is absent and the other is carbonyl group can be synthesized by, for example, the following methods.

Method (13)

A method which comprises subjecting a compound of the formula:

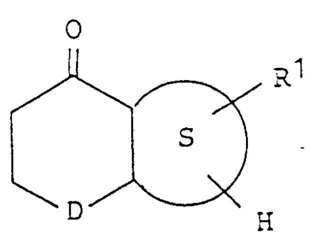

(XVIII)

wherein each symbol is as defined above, to Schmidt rearrangement.

The reaction is carried out by reacting the compound of formula (XVIII) with sodium azide in a suitable and inert solvent such as chloroform, methylene chloride, toluene or benzene or without a solvent in the presence of a suitable acid (e.g. trifluoroacetic acid, polyphosphoric acid, sulfuric acid) at 0 ° C to 150 ° C for 30 minutes to 10 hours.

Method (14)

A method which comprises subjecting a compound of the formula:

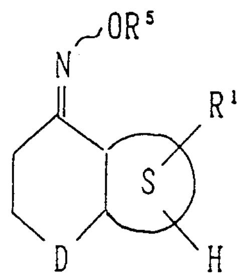

(XIX)

wherein $R^5$ is hydrogen, alkyl, methanensulfonyl group or p-toluenesulfonyl group and other symbols are as defined above, to Beckmann rearrangement.

The reaction is carried out by reacting the compound of formula (XIX) with sodium azide in a suitable and inert solvent such as benzene, toluene, dimethylformamide or diethyl ether or without solvent in the presence of a suitable acid (e.g. phosphoric acid, phosphorus oxychloride, phosphorus pentachloride, phosphorus pentoxide, polyphosphoric acid, sulfuric acid) at 0 ° C to 150 ° C for 30 minutes to 10 hours.

The compound of formula (VI) wherein both of A and B are absent can be synthesized by, for example, the following method.

Method (15)

A method which comprises reducing a compound of the formula:

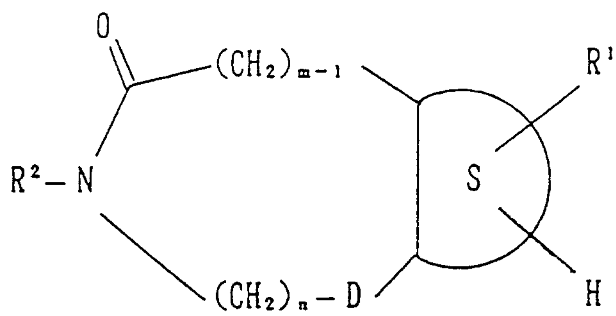

(XX)

wherein each symbol is as defined above.

The reaction is carried out using boron trifluoride-ether complex and sodium borohydride in a suitable and inert solvent such as ether or tetrahydrofuran at 0 ° C to the boiling point of the solvent employed for 30 minutes to 10 hours.

Method (16)

The synthetic intermediate compound of the formula:

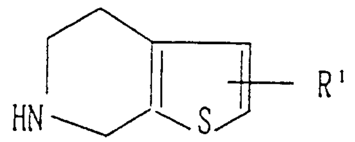

(XXI)

wherein $R^1$ is as defined above, can be prepared by the following method.

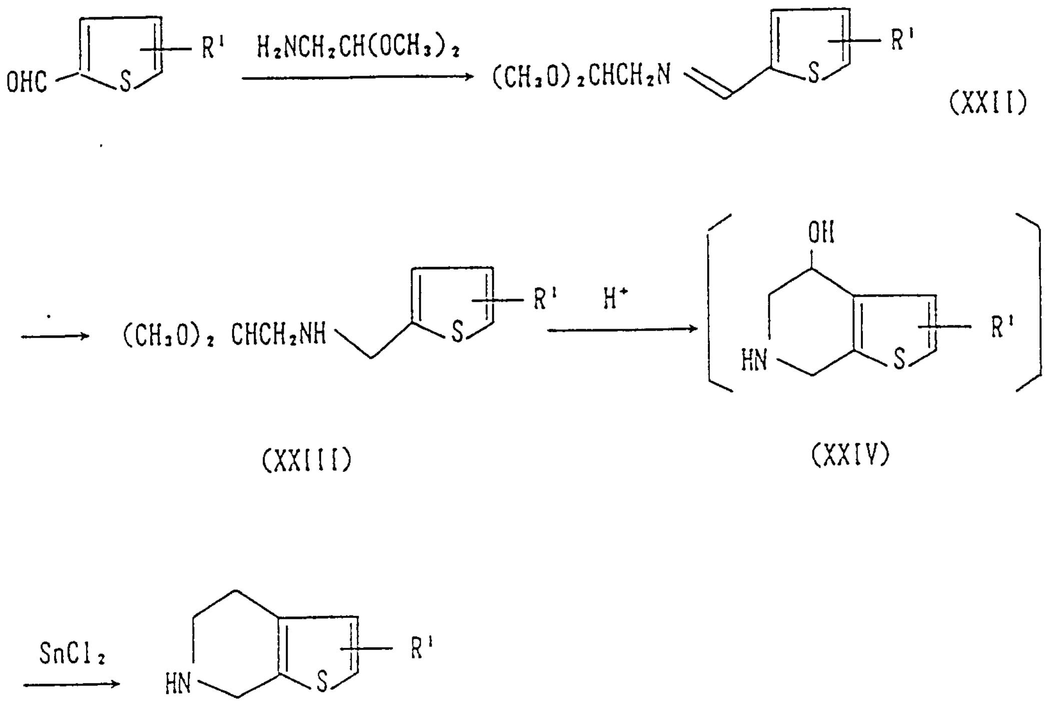


(XXII)

(XXIII)

(XXIV)

(XXI)

The compound of formula (XXII) can be prepared by a dehydration condensation of the corresponding aldehyde compound with aminoacetoaldehyde dimethyl acetal. The reaction is carried out in a suitable and inert solvent, for example, tetrahydrofuran, diethyl ether, methylene chloride, benzene or toluene, preferably without solvent at 10 ° C to 100 ° C for 30 minutes to 10 hours.

The compound of formula (XXIII) can be prepared by reducing the compound of formula (XXII) using a reducing agent such as sodium cyanoborohydride, lithium aluminum hydride, aluminum hydride or diisobutylaluminum hydride, preferably sodium borohydride in an inert solvent such as ethanol, butanol, isopropyl alcohol or tetrahydrofuran at -20 ° C to 100 ° C for 30 minutes to 15 hours.

The compound of formula (XXIV) can be prepared by reacting the compound of formula (XXIII) with an acid such as sulfuric acid, p-toluenesulfonic acid or phosphoric acid, prefebrably hydrochloric acid, in an inert solvent such as water, tetrahydrofuran, diethyl ether or dioxane, preferably in water at 0 ° C to 120 ° C for 30 minutes to 15 hours.

The compound of formula (XXI) can be prepared by reacting the compound of formula (XXIV), which usually may not be isolated and purified, with a reducing agent such as a tin (II) chloride in a solvent such as water, tetrahydrofuran, diethyl ether or dioxane, preferably in water, in the presence of an acid such as sulfuric acid, p-toluenesulfonic acid, phosphoric acid, preferably hydrochloric acid at 0 ° C to 120 ° C for 30 minutes to 15 hours.

The thus obtained compounds of the present invention can be isolated and purified by a conventional method such as recrystallization or column chromatography.

When the obtained compound is a racemate, it can be separated into desired optically active isomers, for example, by means of fractional recrystallization of a salt with an optically active acid or column filled with an optically active carrier. Individual diastereomers can be separated by the method such as fractional crystallizatin or chromatography. Such compounds can also be obtained by using an optically active starting

material. Furthermore, the stereoisomers can be isolated by recrystallization, column chromatography or the like.

The compounds exemplified in the examples and listed in the following tables, or salts thereof are encompassed in the condensed thiophene compounds of formula (I) of the present invention. In the tables, "Me" means methyl, "Et" means ethyl, "$C_6H_5$" means phenyl, and the symbols "I" to "XI" in "T" means the following amine molecules:

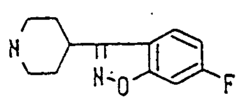

I

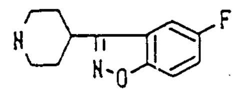

II

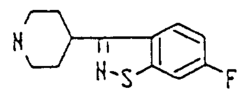

III

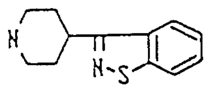

IV

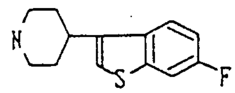

V

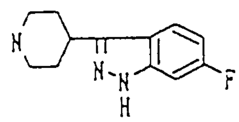

VI

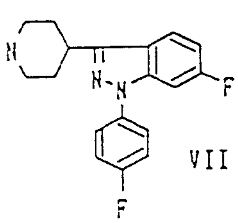

VII

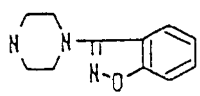

VIII

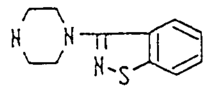

IX

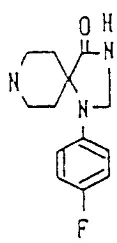

X

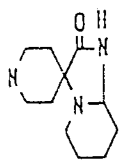

XI

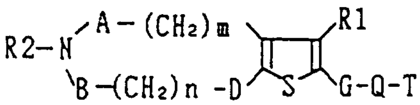

| No. | A | B | D | m | n | R1 | R2 | G | Q | T |
|---|---|---|---|---|---|---|---|---|---|---|
| 1 | - | CO | $CH_2$ | 0 | 1 | H | H | CO | $(CH_2)_3$ | I |
| 2 | - | CO | $CH_2$ | 0 | 1 | Me | H | CO | $(CH_2)_3$ | I |
| 3 | - | CO | $CH_2$ | 0 | 1 | Et | H | CO | $(CH_2)_3$ | I |
| 4 | - | CO | $CH_2$ | 0 | 1 | $COCH_3$ | H | CO | $(CH_2)_3$ | I |
| 5 | - | CO | $CH_2$ | 0 | 1 | $COC_6H_5$ | H | CO | $(CH_2)_3$ | I |
| 6 | - | CO | $CH_2$ | 0 | 1 | F | H | CO | $(CH_2)_3$ | I |
| 7 | - | CO | $CH_2$ | 0 | 1 | Cl | H | CO | $(CH_2)_3$ | I |
| 8 | - | CO | $CH_2$ | 0 | 1 | Br | H | CO | $(CH_2)_3$ | I |
| 9 | - | CO | $CH_2$ | 0 | 1 | $NO_2$ | H | CO | $(CH_2)_3$ | I |
| 10 | - | CO | $CH_2$ | 0 | 1 | $NH_2$ | H | CO | $(CH_2)_3$ | I |
| 11 | - | CO | $CH_2$ | 0 | 1 | CN | H | CO | $(CH_2)_3$ | I |
| 12 | - | CO | $CH_2$ | 0 | 1 | OH | H | CO | $(CH_2)_3$ | I |
| 13 | - | CO | $CH_2$ | 0 | 1 | CHO | H | CO | $(CH_2)_3$ | I |
| 14 | - | CO | $CH_2$ | 0 | 1 | OMe | H | CO | $(CH_2)_3$ | I |
| 15 | - | CO | $CH_2$ | 0 | 1 | $CF_3$ | H | CO | $(CH_2)_3$ | I |
| 16 | - | CO | $CH_2$ | 0 | 1 | $CH_2C_5H_5$ | H | CO | $(CH_2)_3$ | I |
| 17 | - | CO | $CH_2$ | 0 | 1 | $OCOCH_3$ | H | CO | $(CH_2)_3$ | I |
| 18 | - | CO | $CH_2$ | 0 | 1 | $CH_2OCOCH_3$ | H | CO | $(CH_2)_3$ | I |

| No. | A | B | D | m | n | R1 | R2 | G | Q | T |
|---|---|---|---|---|---|---|---|---|---|---|
| 19 | - | CO | $CH_2$ | 0 | 1 | $CH_2OCH_3$ | H | CO | $(CH_2)_3$ | I |
| 20 | - | CO | $CH_2$ | 0 | 1 | $CH_2OH$ | H | CO | $(CH_2)_3$ | I |
| 21 | - | CO | $CH_2$ | 0 | 1 | $COCH_2OCOMe$ | H | CO | $(CH_2)_3$ | I |
| 22 | - | CO | $CH_2$ | 0 | 1 | $COCH_2OMe$ | H | CO | $(CH_2)_3$ | I |
| 23 | - | CO | $CH_2$ | 0 | 1 | $COCH_2OH$ | H | CO | $(CH_2)_3$ | I |
| 24 | - | CO | $CH_2$ | 0 | 1 | $COCH_2OC_6H_5$ | H | CO | $(CH_2)_3$ | I |
| 25 | - | CO | $CH_2$ | 0 | 1 | $COCF_3$ | H | CO | $(CH_2)_3$ | I |
| 26 | - | CO | $CH_2$ | 0 | 1 | SMe | H | CO | $(CH_2)_3$ | I |
| 27 | - | CO | $CH_2$ | 0 | 1 | SOMe | H | CO | $(CH_2)_3$ | I |
| 28 | - | CO | $CH_2$ | 0 | 1 | $SO_2Me$ | H | CO | $(CH_2)_3$ | I |
| 29 | - | CO | $CH_2$ | 0 | 1 | $SC_6H_5$ | H | CO | $(CH_2)_3$ | I |
| 30 | - | CO | $CH_2$ | 0 | 1 | $SOC_6H_5$ | H | CO | $(CH_2)_3$ | I |
| 31 | - | CO | $CH_2$ | 0 | 1 | $SO_2C_6H_5$ | H | CO | $(CH_2)_3$ | I |
| 32 | - | CO | $CH_2$ | 0 | 1 | $SO_3H$ | H | CO | $(CH_2)_3$ | I |
| 33 | - | CO | $CH_2$ | 0 | 1 | $SO_2Cl$ | H | CO | $(CH_2)_3$ | I |
| 34 | - | CO | $CH_2$ | 0 | 1 | $SO_2NH_2$ | H | CO | $(CH_2)_3$ | I |
| 35 | - | CO | $CH_2$ | 0 | 1 | $SO_2NMe_2$ | H | CO | $(CH_2)_3$ | I |
| 36 | - | CO | $CH_2$ | 0 | 1 | COOH | H | CO | $(CH_2)_3$ | I |
| 37 | - | CO | $CH_2$ | 0 | 1 | $NHCOCH_3$ | H | CO | $(CH_2)_3$ | I |
| 38 | - | CO | $CH_2$ | 0 | 1 | COOMe | H | CO | $(CH_2)_3$ | I |
| 39 | - | CO | $CH_2$ | 0 | 1 | $CONH_2$ | H | CO | $(CH_2)_3$ | I |
| 40 | - | CO | $CH_2$ | 0 | 1 | $CONMe_2$ | H | CO | $(CH_2)_3$ | I |

| No. | A | B | D | m | n | R1 | R2 | G | Q | T |
|---|---|---|---|---|---|---|---|---|---|---|
| 41 | - | CO | $CH_2$ | 0 | 1 | $NMe_2$ | H | CO | $(CH_2)_3$ | I |
| 42 | - | CO | $CH_2$ | 0 | 1 | H | H | $CH_2$ | $CH_2$ | I |
| 43 | - | CO | $CH_2$ | 0 | 1 | Me | H | $CH_2$ | $CH_2$ | I |
| 44 | - | CO | $CH_2$ | 0 | 1 | Et | H | $CH_2$ | $CH_2$ | I |
| 45 | - | CO | $CH_2$ | 0 | 1 | $COCH_3$ | H | $CH_2$ | $CH_2$ | I |
| 46 | - | CO | $CH_2$ | 0 | 1 | $COC_6H_5$ | H | $CH_2$ | $CH_2$ | I |
| 47 | - | CO | $CH_2$ | 0 | 1 | F | H | $CH_2$ | $CH_2$ | I |
| 48 | - | CO | $CH_2$ | 0 | 1 | Cl | H | $CH_2$ | $CH_2$ | I |
| 49 | - | CO | $CH_2$ | 0 | 1 | Br | H | $CH_2$ | $CH_2$ | I |
| 50 | - | CO | $CH_2$ | 0 | 1 | $NO_2$ | H | $CH_2$ | $CH_2$ | I |
| 51 | - | CO | $CH_2$ | 0 | 1 | $NH_2$ | H | $CH_2$ | $CH_2$ | I |
| 52 | - | CO | $CH_2$ | 0 | 1 | CN | H | $CH_2$ | $CH_2$ | I |
| 53 | - | CO | $CH_2$ | 0 | 1 | OH | H | $CH_2$ | $CH_2$ | I |
| 54 | - | CO | $CH_2$ | 0 | 1 | CHO | H | $CH_2$ | $CH_2$ | I |
| 55 | - | CO | $CH_2$ | 0 | 1 | OMe | H | $CH_2$ | $CH_2$ | I |
| 56 | - | CO | $CH_2$ | 0 | 1 | $CF_3$ | H | $CH_2$ | $CH_2$ | I |
| 57 | - | CO | $CH_2$ | 0 | 1 | $CH_2C_6H_5$ | H | $CH_2$ | $CH_2$ | I |
| 58 | - | CO | $CH_2$ | 0 | 1 | $OCOCH_3$ | H | $CH_2$ | $CH_2$ | I |
| 59 | - | CO | $CH_2$ | 0 | 1 | $CH_2OCOCH_3$ | H | $CH_2$ | $CH_2$ | I |
| 60 | - | CO | $CH_2$ | 0 | 1 | $CH_2OCH_3$ | H | $CH_2$ | $CH_2$ | I |
| 61 | - | CO | $CH_2$ | 0 | 1 | $CH_2OH$ | H | $CH_2$ | $CH_2$ | I |
| 62 | - | CO | $CH_2$ | 0 | 1 | $COCH_2OCOMe$ | H | $CH_2$ | $CH_2$ | I |

| No. | A | B | D | m | n | R1 | R2 | G | Q | T |
|---|---|---|---|---|---|---|---|---|---|---|
| 63 | - | CO | $CH_2$ | 0 | 1 | $COCH_2OMe$ | H | $CH_2$ | $CH_2$ | I |
| 64 | - | CO | $CH_2$ | 0 | 1 | $COCH_2OH$ | H | $CH_2$ | $CH_2$ | I |
| 65 | - | CO | $CH_2$ | 0 | 1 | $COCH_2OC_6H_5$ | H | $CH_2$ | $CH_2$ | I |
| 66 | - | CO | $CH_2$ | 0 | 1 | $COCF_3$ | H | $CH_2$ | $CH_2$ | I |
| 67 | - | CO | $CH_2$ | 0 | 1 | SMe | H | $CH_2$ | $CH_2$ | I |
| 68 | - | CO | $CH_2$ | 0 | 1 | SOMe | H | $CH_2$ | $CH_2$ | I |
| 69 | - | CO | $CH_2$ | 0 | 1 | $SO_2Me$ | H | $CH_2$ | $CH_2$ | I |
| 70 | - | CO | $CH_2$ | 0 | 1 | $SC_6H_5$ | H | $CH_2$ | $CH_2$ | I |
| 71 | - | CO | $CH_2$ | 0 | 1 | $SOC_6H_5$ | H | $CH_2$ | $CH_2$ | I |
| 72 | - | CO | $CH_2$ | 0 | 1 | $SO_2C_6H_5$ | H | $CH_2$ | $CH_2$ | I |
| 73 | - | CO | $CH_2$ | 0 | 1 | $SO_3H$ | H | $CH_2$ | $CH_2$ | I |
| 74 | - | CO | $CH_2$ | 0 | 1 | $SO_2Cl$ | H | $CH_2$ | $CH_2$ | I |
| 75 | - | CO | $CH_2$ | 0 | 1 | $SO_2NH_2$ | H | $CH_2$ | $CH_2$ | I |
| 76 | - | CO | $CH_2$ | 0 | 1 | $SO_2NMe_2$ | H | $CH_2$ | $CH_2$ | I |
| 77 | - | CO | $CH_2$ | 0 | 1 | COOH | H | $CH_2$ | $CH_2$ | I |
| 78 | - | CO | $CH_2$ | 0 | 1 | $NHCOCH_3$ | H | $CH_2$ | $CH_2$ | I |
| 79 | - | CO | $CH_2$ | 0 | 1 | COOMe | H | $CH_2$ | $CH_2$ | I |
| 80 | - | CO | $CH_2$ | 0 | 1 | $CONH_2$ | H | $CH_2$ | $CH_2$ | I |
| 81 | - | CO | $CH_2$ | 0 | 1 | $CONMe_2$ | H | $CH_2$ | $CH_2$ | I |
| 82 | - | CO | $CH_2$ | 0 | 1 | $NMe_2$ | H | $CH_2$ | $CH_2$ | I |

| No. | A | B | D | m | n | R1 | R2 | G | Q | T |
|---|---|---|---|---|---|---|---|---|---|---|
| 83 | CO | - | $CH_2$ | 0 | 1 | H | H | CO | $(CH_2)_3$ | I |
| 84 | CO | - | $CH_2$ | 0 | 1 | Me | H | CO | $(CH_2)_3$ | I |
| 85 | CO | - | $CH_2$ | 0 | 1 | Et | H | CO | $(CH_2)_3$ | I |
| 86 | CO | - | $CH_2$ | 0 | 1 | $COCH_3$ | H | CO | $(CH_2)_3$ | I |
| 87 | CO | - | $CH_2$ | 0 | 1 | $COC_6H_5$ | H | CO | $(CH_2)_3$ | I |
| 88 | CO | - | $CH_2$ | 0 | 1 | F | H | CO | $(CH_2)_3$ | I |
| 89 | CO | - | $CH_2$ | 0 | 1 | Cl | H | CO | $(CH_2)_3$ | I |
| 90 | CO | - | $CH_2$ | 0 | 1 | Br | H | CO | $(CH_2)_3$ | I |
| 91 | CO | - | $CH_2$ | 0 | 1 | $NO_2$ | H | CO | $(CH_2)_3$ | I |
| 92 | CO | - | $CH_2$ | 0 | 1 | $NH_2$ | H | CO | $(CH_2)_3$ | I |
| 93 | CO | - | $CH_2$ | 0 | 1 | CN | H | CO | $(CH_2)_3$ | I |
| 94 | CO | - | $CH_2$ | 0 | 1 | OH | H | CO | $(CH_2)_3$ | I |
| 95 | CO | - | $CH_2$ | 0 | 1 | CHO | H | CO | $(CH_2)_3$ | I |
| 96 | CO | - | $CH_2$ | 0 | 1 | OMe | H | CO | $(CH_2)_3$ | I |
| 97 | CO | - | $CH_2$ | 0 | 1 | $CF_3$ | H | CO | $(CH_2)_3$ | I |
| 98 | CO | - | $CH_2$ | 0 | 1 | $CH_2C_6H_5$ | H | CO | $(CH_2)_3$ | I |
| 99 | CO | - | $CH_2$ | 0 | 1 | $OCOCH_3$ | H | CO | $(CH_2)_3$ | I |
| 100 | CO | - | $CH_2$ | 0 | 1 | $CH_2OCOCH_3$ | H | CO | $(CH_2)_3$ | I |
| 101 | CO | - | $CH_2$ | 0 | 1 | $CH_2OCH_3$ | H | CO | $(CH_2)_3$ | I |
| 102 | CO | - | $CH_2$ | 0 | 1 | $CH_2OH$ | H | CO | $(CH_2)_3$ | I |
| 103 | CO | - | $CH_2$ | 0 | 1 | $COCH_2OCOMe$ | H | CO | $(CH_2)_3$ | I |
| 104 | CO | - | $CH_2$ | 0 | 1 | $COCH_2OMe$ | H | CO | $(CH_2)_3$ | I |

| No. | A | B | D | m | n | R1 | R2 | G | Q | T |
|---|---|---|---|---|---|---|---|---|---|---|
| 105 | CO | - | $CH_2$ | 0 | 1 | $COCH_2OH$ | H | CO | $(CH_2)_3$ | I |
| 106 | CO | - | $CH_2$ | 0 | 1 | $COCH_2OC_6H_5$ | H | CO | $(CH_2)_3$ | I |
| 107 | CO | - | $CH_2$ | 0 | 1 | $COCF_3$ | H | CO | $(CH_2)_3$ | I |
| 108 | CO | - | $CH_2$ | 0 | 1 | SMe | H | CO | $(CH_2)_3$ | I |
| 109 | CO | - | $CH_2$ | 0 | 1 | SOMe | H | CO | $(CH_2)_3$ | I |
| 110 | CO | - | $CH_2$ | 0 | 1 | $SO_2Me$ | H | CO | $(CH_2)_3$ | I |
| 111 | CO | - | $CH_2$ | 0 | 1 | $SC_5H_5$ | H | CO | $(CH_2)_3$ | I |
| 112 | CO | - | $CH_2$ | 0 | 1 | $SOC_6H_5$ | H | CO | $(CH_2)_3$ | I |
| 113 | CO | - | $CH_2$ | 0 | 1 | $SO_2C_6H_5$ | H | CO | $(CH_2)_3$ | I |
| 114 | CO | - | $CH_2$ | 0 | 1 | $SO_3H$ | H | CO | $(CH_2)_3$ | I |
| 115 | CO | - | $CH_2$ | 0 | 1 | $SO_2Cl$ | H | CO | $(CH_2)_3$ | I |
| 116 | CO | - | $CH_2$ | 0 | 1 | $SO_2NH_2$ | H | CO | $(CH_2)_3$ | I |
| 117 | CO | - | $CH_2$ | 0 | 1 | $SO_2NMe_2$ | H | CO | $(CH_2)_3$ | I |
| 118 | CO | - | $CH_2$ | 0 | 1 | COOH | H | CO | $(CH_2)_3$ | I |
| 119 | CO | - | $CH_2$ | 0 | 1 | $NHCOCH_3$ | H | CO | $(CH_2)_3$ | I |
| 120 | CO | - | $CH_2$ | 0 | 1 | COOMe | H | CO | $(CH_2)_3$ | I |
| 121 | CO | - | $CH_2$ | 0 | 1 | $CONH_2$ | H | CO | $(CH_2)_3$ | I |
| 122 | CO | - | $CH_2$ | 0 | 1 | $CONMe_2$ | H | CO | $(CH_2)_3$ | I |
| 123 | CO | - | $CH_2$ | 0 | 1 | $NMe_2$ | H | CO | $(CH_2)_3$ | I |
| 124 | CO | - | $CH_2$ | 0 | 1 | H | H | $CH_2$ | $CH_2$ | I |
| 125 | CO | - | $CH_2$ | 0 | 1 | Me | H | $CH_2$ | $CH_2$ | I |
| 126 | CO | - | $CH_2$ | 0 | 1 | Et | H | $CH_2$ | $CH_2$ | I |

| No. | A | B | D | m | n | R1 | R2 | G | Q | T |
|---|---|---|---|---|---|---|---|---|---|---|
| 127 | CO | - | $CH_2$ | 0 | 1 | $COCH_3$ | H | $CH_2$ | $CH_2$ | I |
| 128 | CO | - | $CH_2$ | 0 | 1 | $COC_6H_5$ | H | $CH_2$ | $CH_2$ | I |
| 129 | CO | - | $CH_2$ | 0 | 1 | F | H | $CH_2$ | $CH_2$ | I |
| 130 | CO | - | $CH_2$ | 0 | 1 | Cl | H | $CH_2$ | $CH_2$ | I |
| 131 | CO | - | $CH_2$ | 0 | 1 | Br | H | $CH_2$ | $CH_2$ | I |
| 132 | CO | - | $CH_2$ | 0 | 1 | $NO_2$ | H | $CH_2$ | $CH_2$ | I |
| 133 | CO | - | $CH_2$ | 0 | 1 | $NH_2$ | H | $CH_2$ | $CH_2$ | I |
| 134 | CO | - | $CH_2$ | 0 | 1 | CN | H | $CH_2$ | $CH_2$ | I |
| 135 | CO | - | $CH_2$ | 0 | 1 | OH | H | $CH_2$ | $CH_2$ | I |
| 136 | CO | - | $CH_2$ | 0 | 1 | CHO | H | $CH_2$ | $CH_2$ | I |
| 137 | CO | - | $CH_2$ | 0 | 1 | OMe | H | $CH_2$ | $CH_2$ | I |
| 138 | CO | - | $CH_2$ | 0 | 1 | $CF_3$ | H | $CH_2$ | $CH_2$ | I |
| 139 | CO | - | $CH_2$ | 0 | 1 | $CH_2C_6H_5$ | H | $CH_2$ | $CH_2$ | I |
| 140 | CO | - | $CH_2$ | 0 | 1 | $OCOCH_3$ | H | $CH_2$ | $CH_2$ | I |
| 141 | CO | - | $CH_2$ | 0 | 1 | $CH_2OCOCH_3$ | H | $CH_2$ | $CH_2$ | I |
| 142 | CO | - | $CH_2$ | 0 | 1 | $CH_2OCH_3$ | H | $CH_2$ | $CH_2$ | I |
| 143 | CO | - | $CH_2$ | 0 | 1 | $CH_2OH$ | H | $CH_2$ | $CH_2$ | I |
| 144 | CO | - | $CH_2$ | 0 | 1 | $COCH_2OCOMe$ | H | $CH_2$ | $CH_2$ | I |
| 145 | CO | - | $CH_2$ | 0 | 1 | $COCH_2OMe$ | H | $CH_2$ | $CH_2$ | I |
| 146 | CO | - | $CH_2$ | 0 | 1 | $COCH_2OH$ | H | $CH_2$ | $CH_2$ | I |
| 147 | CO | - | $CH_2$ | 0 | 1 | $COCH_2OC_6H_5$ | H | $CH_2$ | $CH_2$ | I |

| No. | A | B | D | m | n | R1 | R2 | G | Q | T |
|---|---|---|---|---|---|---|---|---|---|---|
| 148 | CO | - | $CH_2$ | 0 | 1 | $COCF_3$ | H | $CH_2$ | $CH_2$ | I |
| 149 | CO | - | $CH_2$ | 0 | 1 | SMe | H | $CH_2$ | $CH_2$ | I |
| 150 | CO | - | $CH_2$ | 0 | 1 | SOMe | H | $CH_2$ | $CH_2$ | I |
| 151 | CO | - | $CH_2$ | 0 | 1 | $SO_2Me$ | H | $CH_2$ | $CH_2$ | I |
| 152 | CO | - | $CH_2$ | 0 | 1 | $SC_6H_5$ | H | $CH_2$ | $CH_2$ | I |
| 153 | CO | - | $CH_2$ | 0 | 1 | $SOC_6H_5$ | H | $CH_2$ | $CH_2$ | I |
| 154 | CO | - | $CH_2$ | 0 | 1 | $SO_2C_6H_5$ | H | $CH_2$ | $CH_2$ | I |
| 155 | CO | - | $CH_2$ | 0 | 1 | $SO_3H$ | H | $CH_2$ | $CH_2$ | I |
| 156 | CO | - | $CH_2$ | 0 | 1 | $SO_2Cl$ | H | $CH_2$ | $CH_2$ | I |
| 157 | CO | - | $CH_2$ | 0 | 1 | $SO_2NH_2$ | H | $CH_2$ | $CH_2$ | I |
| 158 | CO | - | $CH_2$ | 0 | 1 | $SO_2NMe_2$ | H | $CH_2$ | $CH_2$ | I |
| 159 | CO | - | $CH_2$ | 0 | 1 | COOH | H | $CH_2$ | $CH_2$ | I |
| 160 | CO | - | $CH_2$ | 0 | 1 | $NHCOCH_3$ | H | $CH_2$ | $CH_2$ | I |
| 161 | CO | - | $CH_2$ | 0 | 1 | COOMe | H | $CH_2$ | $CH_2$ | I |
| 162 | CO | - | $CH_2$ | 0 | 1 | $CONH_2$ | H | $CH_2$ | $CH_2$ | I |
| 163 | CO | - | $CH_2$ | 0 | 1 | $CONMe_2$ | H | $CH_2$ | $CH_2$ | I |
| 164 | CO | - | $CH_2$ | 0 | 1 | $NMe_2$ | H | $CH_2$ | $CH_2$ | I |
| 165 | - | CO | $CH_2$ | 1 | 0 | H | H | CO | $(CH_2)_3$ | I |
| 166 | - | CO | $CH_2$ | 1 | 0 | Me | H | CO | $(CH_2)_3$ | I |
| 167 | - | CO | $CH_2$ | 1 | 0 | Et | H | CO | $(CH_2)_3$ | I |
| 168 | - | CO | $CH_2$ | 1 | 0 | $COCH_3$ | H | CO | $(CH_2)_3$ | I |
| 169 | - | CO | $CH_2$ | 1 | 0 | $COC_6H_5$ | H | CO | $(CH_2)_3$ | I |

| No. | A | B | D | m | n | R1 | R2 | G | Q | T |
|---|---|---|---|---|---|---|---|---|---|---|
| 170 | - | CO | $CH_2$ | 1 | 0 | Br | H | CO | $(CH_2)_3$ | I |
| 171 | - | CO | $CH_2$ | 1 | 0 | H | H | $CH_2$ | $CH_2$ | I |
| 172 | - | CO | $CH_2$ | 1 | 0 | Me | H | $CH_2$ | $CH_2$ | I |
| 173 | - | CO | $CH_2$ | 1 | 0 | Et | H | $CH_2$ | $CH_2$ | I |
| 174 | - | CO | $CH_2$ | 1 | 0 | $COCH_3$ | H | $CH_2$ | $CH_2$ | I |
| 175 | - | CO | $CH_2$ | 1 | 0 | $COC_6H_5$ | H | $CH_2$ | $CH_2$ | I |
| 176 | - | CO | $CH_2$ | 1 | 0 | Br | H | $CH_2$ | $CH_2$ | I |
| 177 | CO | - | $CH_2$ | 1 | 0 | H | H | CO | $(CH_2)_3$ | I |
| 178 | CO | - | $CH_2$ | 1 | 0 | Me | H | CO | $(CH_2)_3$ | I |
| 179 | CO | - | $CH_2$ | 1 | 0 | Et | H | CO | $(CH_2)_3$ | I |
| 180 | CO | - | $CH_2$ | 1 | 0 | $COCH_3$ | H | CO | $(CH_2)_3$ | I |
| 181 | CO | - | $CH_2$ | 1 | 0 | $COC_6H_5$ | H | CO | $(CH_2)_3$ | I |
| 182 | CO | - | $CH_2$ | 1 | 0 | Br | H | CO | $(CH_2)_3$ | I |
| 183 | CO | - | $CH_2$ | 1 | 0 | H | H | $CH_2$ | $CH_2$ | I |
| 184 | CO | - | $CH_2$ | 1 | 0 | Me | H | $CH_2$ | $CH_2$ | I |
| 185 | CO | - | $CH_2$ | 1 | 0 | Et | H | $CH_2$ | $CH_2$ | I |
| 186 | CO | - | $CH_2$ | 1 | 0 | $COCH_3$ | H | $CH_2$ | $CH_2$ | I |
| 187 | CO | - | $CH_2$ | 1 | 0 | $COC_6H_5$ | H | $CH_2$ | $CH_2$ | I |
| 188 | CO | - | $CH_2$ | 1 | 0 | Br | H | $CH_2$ | $CH_2$ | I |
| 189 | - | CO | $CH_2$ | 0 | 2 | H | H | CO | $(CH_2)_3$ | I |
| 190 | - | CO | $CH_2$ | 0 | 2 | Me | H | CO | $(CH_2)_3$ | I |

| No. | A | B | D | m | n | R1 | R2 | G | Q | T |
|---|---|---|---|---|---|---|---|---|---|---|
| 191 | - | CO | $CH_2$ | 0 | 2 | Et | H | CO | $(CH_2)_3$ | I |
| 192 | - | CO | $CH_2$ | 0 | 2 | $COCH_3$ | H | CO | $(CH_2)_3$ | I |
| 193 | - | CO | $CH_2$ | 0 | 2 | $COC_6H_5$ | H | CO | $(CH_2)_3$ | I |
| 194 | - | CO | $CH_2$ | 0 | 2 | Br | H | CO | $(CH_2)_3$ | I |
| 195 | - | CO | $CH_2$ | 0 | 2 | H | H | $CH_2$ | $CH_2$ | I |
| 196 | - | CO | $CH_2$ | 0 | 2 | Me | H | $CH_2$ | $CH_2$ | I |
| 197 | - | CO | $CH_2$ | 0 | 2 | Et | H | $CH_2$ | $CH_2$ | I |
| 198 | - | CO | $CH_2$ | 0 | 2 | $COCH_3$ | H | $CH_2$ | $CH_2$ | I |
| 199 | - | CO | $CH_2$ | 0 | 2 | $COC_6H_5$ | H | $CH_2$ | $CH_2$ | I |
| 200 | - | CO | $CH_2$ | 0 | 2 | Br | H | $CH_2$ | $CH_2$ | I |
| 201 | CO | - | $CH_2$ | 0 | 2 | H | H | CO | $(CH_2)_3$ | I |
| 202 | CO | - | $CH_2$ | 0 | 2 | Me | H | CO | $(CH_2)_3$ | I |
| 203 | CO | - | $CH_2$ | 0 | 2 | Et | H | CO | $(CH_2)_3$ | I |
| 204 | CO | - | $CH_2$ | 0 | 2 | $COCH_3$ | H | CO | $(CH_2)_3$ | I |
| 205 | CO | - | $CH_2$ | 0 | 2 | $COC_6H_5$ | H | CO | $(CH_2)_3$ | I |
| 206 | CO | - | $CH_2$ | 0 | 2 | Br | H | CO | $(CH_2)_3$ | I |
| 207 | CO | - | $CH_2$ | 0 | 2 | H | H | $CH_2$ | $CH_2$ | I |
| 208 | CO | - | $CH_2$ | 0 | 2 | Me | H | $CH_2$ | $CH_2$ | I |
| 209 | CO | - | $CH_2$ | 0 | 2 | Et | H | $CH_2$ | $CH_2$ | I |
| 210 | CO | - | $CH_2$ | 0 | 2 | $COCH_3$ | H | $CH_2$ | $CH_2$ | I |
| 211 | CO | - | $CH_2$ | 0 | 2 | $COC_6H_5$ | H | $CH_2$ | $CH_2$ | I |
| 212 | CO | - | $CH_2$ | 0 | 2 | Br | H | $CH_2$ | $CH_2$ | I |

| No. | A | B | D | m | n | R1 | R2 | G | Q | T |
|---|---|---|---|---|---|---|---|---|---|---|
| 213 | - | CO | $CH_2$ | 1 | 1 | H | H | CO | $(CH_2)_3$ | I |
| 214 | - | CO | $CH_2$ | 1 | 1 | Me | H | CO | $(CH_2)_3$ | I |
| 215 | - | CO | $CH_2$ | 1 | 1 | Et | H | CO | $(CH_2)_3$ | I |
| 216 | - | CO | $CH_2$ | 1 | 1 | $COCH_3$ | H | CO | $(CH_2)_3$ | I |
| 217 | - | CO | $CH_2$ | 1 | 1 | $COC_6H_5$ | H | CO | $(CH_2)_3$ | I |
| 218 | - | CO | $CH_2$ | 1 | 1 | Br | H | CO | $(CH_2)_3$ | I |
| 219 | - | CO | $CH_2$ | 1 | 1 | H | H | $CH_2$ | $CH_2$ | I |
| 220 | - | CO | $CH_2$ | 1 | 1 | Me | H | $CH_2$ | $CH_2$ | I |
| 221 | - | CO | $CH_2$ | 1 | 1 | Et | H | $CH_2$ | $CH_2$ | I |
| 222 | - | CO | $CH_2$ | 1 | 1 | $COCH_3$ | H | $CH_2$ | $CH_2$ | I |
| 223 | - | CO | $CH_2$ | 1 | 1 | $COC_6H_5$ | H | $CH_2$ | $CH_2$ | I |
| 224 | - | CO | $CH_2$ | 1 | 1 | Br | H | $CH_2$ | $CH_2$ | I |
| 225 | CO | - | $CH_2$ | 1 | 1 | H | H | CO | $(CH_2)_3$ | I |
| 226 | CO | - | $CH_2$ | 1 | 1 | Me | H | CO | $(CH_2)_3$ | I |
| 227 | CO | - | $CH_2$ | 1 | 1 | Et | H | CO | $(CH_2)_3$ | I |
| 228 | CO | - | $CH_2$ | 1 | 1 | $COCH_3$ | H | CO | $(CH_2)_3$ | I |
| 229 | CO | - | $CH_2$ | 1 | 1 | $COC_5H_5$ | H | CO | $(CH_2)_3$ | I |
| 230 | CO | - | $CH_2$ | 1 | 1 | Br | H | CO | $(CH_2)_3$ | I |
| 231 | CO | - | $CH_2$ | 1 | 1 | H | H | $CH_2$ | $CH_2$ | I |
| 232 | CO | - | $CH_2$ | 1 | 1 | Me | H | $CH_2$ | $CH_2$ | I |
| 233 | CO | - | $CH_2$ | 1 | 1 | Et | H | $CH_2$ | $CH_2$ | I |
| 234 | CO | - | $CH_2$ | 1 | 1 | $COCH_3$ | H | $CH_2$ | $CH_2$ | I |

| No. | A | B | D | m | n | R1 | R2 | G | Q | T |
|---|---|---|---|---|---|---|---|---|---|---|
| 235 | CO | - | $CH_2$ | 1 | 1 | $COC_6H_5$ | H | $CH_2$ | $CH_2$ | I |
| 236 | CO | - | $CH_2$ | 1 | 1 | Br | H | $CH_2$ | $CH_2$ | I |
| 237 | - | CO | $CH_2$ | 2 | 0 | H | H | CO | $(CH_2)_3$ | I |
| 238 | - | CO | $CH_2$ | 2 | 0 | Me | H | CO | $(CH_2)_3$ | I |
| 239 | - | CO | $CH_2$ | 2 | 0 | Et | H | CO | $(CH_2)_3$ | I |
| 240 | - | CO | $CH_2$ | 2 | 0 | $COCH_3$ | H | CO | $(CH_2)_3$ | I |
| 241 | - | CO | $CH_2$ | 2 | 0 | $COC_6H_5$ | H | CO | $(CH_2)_3$ | I |
| 242 | - | CO | $CH_2$ | 2 | 0 | Br | H | CO | $(CH_2)_3$ | I |
| 243 | - | CO | $CH_2$ | 2 | 0 | H | H | $CH_2$ | $CH_2$ | I |
| 244 | - | CO | $CH_2$ | 2 | 0 | Me | H | $CH_2$ | $CH_2$ | I |
| 245 | - | CO | $CH_2$ | 2 | 0 | Et | H | $CH_2$ | $CH_2$ | I |
| 246 | - | CO | $CH_2$ | 2 | 0 | $COCH_3$ | H | $CH_2$ | $CH_2$ | I |
| 247 | - | CO | $CH_2$ | 2 | 0 | $COC_6H_5$ | H | $CH_2$ | $CH_2$ | I |
| 248 | - | CO | $CH_2$ | 2 | 0 | Br | H | $CH_2$ | $CH_2$ | I |
| 249 | CO | - | $CH_2$ | 2 | 0 | H | H | CO | $(CH_2)_3$ | I |
| 250 | CO | - | $CH_2$ | 2 | 0 | Me | H | CO | $(CH_2)_3$ | I |
| 251 | CO | - | $CH_2$ | 2 | 0 | Et | H | CO | $(CH_2)_3$ | I |
| 252 | CO | - | $CH_2$ | 2 | 0 | $COCH_3$ | H | CO | $(CH_2)_3$ | I |
| 253 | CO | - | $CH_2$ | 2 | 0 | $COC_6H_5$ | H | CO | $(CH_2)_3$ | I |
| 254 | CO | - | $CH_2$ | 2 | 0 | Br | H | CO | $(CH_2)_3$ | I |
| 255 | CO | - | $CH_2$ | 2 | 0 | H | H | $CH_2$ | $CH_2$ | I |

| No. | A | B | D | m | n | R1 | R2 | G | Q | T |
|---|---|---|---|---|---|---|---|---|---|---|
| 256 | CO | - | $CH_2$ | 2 | 0 | Me | H | $CH_2$ | $CH_2$ | I |
| 257 | CO | - | $CH_2$ | 2 | 0 | Et | H | $CH_2$ | $CH_2$ | I |
| 258 | CO | - | $CH_2$ | 2 | 0 | $COCH_3$ | H | $CH_2$ | $CH_2$ | I |
| 259 | CO | - | $CH_2$ | 2 | 0 | $COC_6H_5$ | H | $CH_2$ | $CH_2$ | I |
| 260 | CO | - | $CH_2$ | 2 | 0 | Br | H | $CH_2$ | $CH_2$ | I |
| 261 | - | CO | $CH_2$ | 0 | 3 | H | H | CO | $(CH_2)_3$ | I |
| 262 | - | CO | $CH_2$ | 0 | 3 | Me | H | CO | $(CH_2)_3$ | I |
| 263 | - | CO | $CH_2$ | 0 | 3 | Et | H | CO | $(CH_2)_3$ | I |
| 264 | - | CO | $CH_2$ | 0 | 3 | $COCH_3$ | H | CO | $(CH_2)_3$ | I |
| 265 | - | CO | $CH_2$ | 0 | 3 | $COC_5H_5$ | H | CO | $(CH_2)_3$ | I |
| 266 | - | CO | $CH_2$ | 0 | 3 | Br | H | CO | $(CH_2)_3$ | I |
| 267 | - | CO | $CH_2$ | 0 | 3 | H | H | $CH_2$ | $CH_2$ | I |
| 268 | - | CO | $CH_2$ | 0 | 3 | Me | H | $CH_2$ | $CH_2$ | I |
| 269 | - | CO | $CH_2$ | 0 | 3 | Et | H | $CH_2$ | $CH_2$ | I |
| 270 | - | CO | $CH_2$ | 0 | 3 | $COCH_3$ | H | $CH_2$ | $CH_2$ | I |
| 271 | - | CO | $CH_2$ | 0 | 3 | $COC_6H_5$ | H | $CH_2$ | $CH_2$ | I |
| 272 | - | CO | $CH_2$ | 0 | 3 | Br | H | $CH_2$ | $CH_2$ | I |
| 273 | CO | - | $CH_2$ | 0 | 3 | H | H | CO | $(CH_2)_3$ | I |
| 274 | CO | - | $CH_2$ | 0 | 3 | Me | H | CO | $(CH_2)_3$ | I |
| 275 | CO | - | $CH_2$ | 0 | 3 | Et | H | CO | $(CH_2)_3$ | I |
| 276 | CO | - | $CH_2$ | 0 | 3 | $COCH_3$ | H | CO | $(CH_2)_3$ | I |
| 277 | CO | - | $CH_2$ | 0 | 3 | $COC_6H_5$ | H | CO | $(CH_2)_3$ | I |

| No. | A | B | D | m | n | R1 | R2 | G | Q | T |
|---|---|---|---|---|---|---|---|---|---|---|
| 278 | CO | - | $CH_2$ | 0 | 3 | Br | H | CO | $(CH_2)_3$ | I |
| 279 | CO | - | $CH_2$ | 0 | 3 | H | H | $CH_2$ | $CH_2$ | I |
| 280 | CO | - | $CH_2$ | 0 | 3 | Me | H | $CH_2$ | $CH_2$ | I |
| 281 | CO | - | $CH_2$ | 0 | 3 | Et | H | $CH_2$ | $CH_2$ | I |
| 282 | CO | - | $CH_2$ | 0 | 3 | $COCH_3$ | H | $CH_2$ | $CH_2$ | I |
| 283 | CO | - | $CH_2$ | 0 | 3 | $COC_6H_5$ | H | $CH_2$ | $CH_2$ | I |
| 284 | CO | - | $CH_2$ | 0 | 3 | Br | H | $CH_2$ | $CH_2$ | I |
| 285 | - | CO | $CH_2$ | 1 | 2 | H | H | CO | $(CH_2)_3$ | I |
| 286 | - | CO | $CH_2$ | 1 | 2 | Me | H | CO | $(CH_2)_3$ | I |
| 287 | - | CO | $CH_2$ | 1 | 2 | Et | H | CO | $(CH_2)_3$ | I |
| 288 | - | CO | $CH_2$ | 1 | 2 | $COCH_3$ | H | CO | $(CH_2)_3$ | I |
| 289 | - | CO | $CH_2$ | 1 | 2 | $COC_6H_5$ | H | CO | $(CH_2)_3$ | I |
| 290 | - | CO | $CH_2$ | 1 | 2 | Br | H | CO | $(CH_2)_3$ | I |
| 291 | - | CO | $CH_2$ | 1 | 2 | H | H | $CH_2$ | $CH_2$ | I |
| 292 | - | CO | $CH_2$ | 1 | 2 | Me | H | $CH_2$ | $CH_2$ | I |
| 293 | - | CO | $CH_2$ | 1 | 2 | Et | H | $CH_2$ | $CH_2$ | I |
| 294 | - | CO | $CH_2$ | 1 | 2 | $COCH_3$ | H | $CH_2$ | $CH_2$ | I |
| 295 | - | CO | $CH_2$ | 1 | 2 | $COC_6H_5$ | H | $CH_2$ | $CH_2$ | I |
| 296 | - | CO | $CH_2$ | 1 | 2 | Br | H | $CH_2$ | $CH_2$ | I |
| 297 | CO | - | $CH_2$ | 1 | 2 | H | H | CO | $(CH_2)_3$ | I |
| 298 | CO | - | $CH_2$ | 1 | 2 | Me | H | CO | $(CH_2)_3$ | I |

| No. | A | B | D | m | n | R1 | R2 | G | Q | T |
|---|---|---|---|---|---|---|---|---|---|---|
| 299 | CO | - | $CH_2$ | 1 | 2 | Et | H | CO | $(CH_2)_3$ | I |
| 300 | CO | - | $CH_2$ | 1 | 2 | $COCH_3$ | H | CO | $(CH_2)_3$ | I |
| 301 | CO | - | $CH_2$ | 1 | 2 | $COC_6H_5$ | H | CO | $(CH_2)_3$ | I |
| 302 | CO | - | $CH_2$ | 1 | 2 | Br | H | CO | $(CH_2)_3$ | I |
| 303 | CO | - | $CH_2$ | 1 | 2 | H | H | $CH_2$ | $CH_2$ | I |
| 304 | CO | - | $CH_2$ | 1 | 2 | Me | H | $CH_2$ | $CH_2$ | I |
| 305 | CO | - | $CH_2$ | 1 | 2 | Et | H | $CH_2$ | $CH_2$ | I |
| 306 | CO | - | $CH_2$ | 1 | 2 | $COCH_3$ | H | $CH_2$ | $CH_2$ | I |
| 307 | CO | - | $CH_2$ | 1 | 2 | $COC_6H_5$ | H | $CH_2$ | $CH_2$ | I |
| 308 | CO | - | $CH_2$ | 1 | 2 | Br | H | $CH_2$ | $CH_2$ | I |
| 309 | - | CO | $CH_2$ | 2 | 1 | H | H | CO | $(CH_2)_3$ | I |
| 310 | - | CO | $CH_2$ | 2 | 1 | Me | H | CO | $(CH_2)_3$ | I |
| 311 | - | CO | $CH_2$ | 2 | 1 | Et | H | CO | $(CH_2)_3$ | I |
| 312 | - | CO | $CH_2$ | 2 | 1 | $COCH_3$ | H | CO | $(CH_2)_3$ | I |
| 313 | - | CO | $CH_2$ | 2 | 1 | $COC_5H_5$ | H | CO | $(CH_2)_3$ | I |
| 314 | - | CO | $CH_2$ | 2 | 1 | Br | H | CO | $(CH_2)_3$ | I |
| 315 | - | CO | $CH_2$ | 2 | 1 | H | H | $CH_2$ | $CH_2$ | I |
| 316 | - | CO | $CH_2$ | 2 | 1 | Me | H | $CH_2$ | $CH_2$ | I |
| 317 | - | CO | $CH_2$ | 2 | 1 | Et | H | $CH_2$ | $CH_2$ | I |
| 318 | - | CO | $CH_2$ | 2 | 1 | $COCH_3$ | H | $CH_2$ | $CH_2$ | I |
| 319 | - | CO | $CH_2$ | 2 | 1 | $COC_5H_5$ | H | $CH_2$ | $CH_2$ | I |
| 320 | - | CO | $CH_2$ | 2 | 1 | Br | H | $CH_2$ | $CH_2$ | I |

| No. | A | B | D | m | n | R1 | R2 | G | Q | T |
|---|---|---|---|---|---|---|---|---|---|---|
| 321 | CO | - | $CH_2$ | 2 | 1 | H | H | CO | $(CH_2)_3$ | I |
| 322 | CO | - | $CH_2$ | 2 | 1 | Me | H | CO | $(CH_2)_3$ | I |
| 323 | CO | - | $CH_2$ | 2 | 1 | Et | H | CO | $(CH_2)_3$ | I |
| 324 | CO | - | $CH_2$ | 2 | 1 | $COCH_3$ | H | CO | $(CH_2)_3$ | I |
| 325 | CO | - | $CH_2$ | 2 | 1 | $COC_6H_5$ | H | CO | $(CH_2)_3$ | I |
| 326 | CO | - | $CH_2$ | 2 | 1 | Br | H | CO | $(CH_2)_3$ | I |
| 327 | CO | - | $CH_2$ | 2 | 1 | H | H | $CH_2$ | $CH_2$ | I |
| 328 | CO | - | $CH_2$ | 2 | 1 | Me | H | $CH_2$ | $CH_2$ | I |
| 329 | CO | - | $CH_2$ | 2 | 1 | Et | H | $CH_2$ | $CH_2$ | I |
| 330 | CO | - | $CH_2$ | 2 | 1 | $COCH_3$ | H | $CH_2$ | $CH_2$ | I |
| 331 | CO | - | $CH_2$ | 2 | 1 | $COC_6H_5$ | H | $CH_2$ | $CH_2$ | I |
| 332 | CO | - | $CH_2$ | 2 | 1 | Br | H | $CH_2$ | $CH_2$ | I |
| 333 | - | CO | $CH_2$ | 3 | 0 | H | H | CO | $(CH_2)_3$ | I |
| 334 | - | CO | $CH_2$ | 3 | 0 | Me | H | CO | $(CH_2)_3$ | I |
| 335 | - | CO | $CH_2$ | 3 | 0 | Et | H | CO | $(CH_2)_3$ | I |
| 336 | - | CO | $CH_2$ | 3 | 0 | $COCH_3$ | H | CO | $(CH_2)_3$ | I |
| 337 | - | CO | $CH_2$ | 3 | 0 | $COC_6H_5$ | H | CO | $(CH_2)_3$ | I |
| 338 | - | CO | $CH_2$ | 3 | 0 | Br | H | CO | $(CH_2)_3$ | I |
| 339 | - | CO | $CH_2$ | 3 | 0 | H | H | $CH_2$ | $CH_2$ | I |
| 340 | - | CO | $CH_2$ | 3 | 0 | Me | H | $CH_2$ | $CH_2$ | I |
| 341 | - | CO | $CH_2$ | 3 | 0 | Et | H | $CH_2$ | $CH_2$ | I |
| 342 | - | CO | $CH_2$ | 3 | 0 | $COCH_3$ | H | $CH_2$ | $CH_2$ | I |

| No. | A | B | D | m | n | R1 | R2 | G | Q | T |
|---|---|---|---|---|---|---|---|---|---|---|
| 343 | - | CO | $CH_2$ | 3 | 0 | $COC_6H_5$ | H | $CH_2$ | $CH_2$ | I |
| 344 | - | CO | $CH_2$ | 3 | 0 | Br | H | $CH_2$ | $CH_2$ | I |
| 345 | CO | - | $CH_2$ | 3 | 0 | H | H | CO | $(CH_2)_3$ | I |
| 346 | CO | - | $CH_2$ | 3 | 0 | Me | H | CO | $(CH_2)_3$ | I |
| 347 | CO | - | $CH_2$ | 3 | 0 | Et | H | CO | $(CH_2)_3$ | I |
| 348 | CO | - | $CH_2$ | 3 | 0 | $COCH_3$ | H | CO | $(CH_2)_3$ | I |
| 349 | CO | - | $CH_2$ | 3 | 0 | $COC_6H_5$ | H | CO | $(CH_2)_3$ | I |
| 350 | CO | - | $CH_2$ | 3 | 0 | Br | H | CO | $(CH_2)_3$ | I |
| 351 | CO | - | $CH_2$ | 3 | 0 | H | H | $CH_2$ | $CH_2$ | I |
| 352 | CO | - | $CH_2$ | 3 | 0 | Me | H | $CH_2$ | $CH_2$ | I |
| 353 | CO | - | $CH_2$ | 3 | 0 | Et | H | $CH_2$ | $CH_2$ | I |
| 354 | CO | - | $CH_2$ | 3 | 0 | $COCH_3$ | H | $CH_2$ | $CH_2$ | I |
| 355 | CO | - | $CH_2$ | 3 | 0 | $COC_5H_5$ | H | $CH_2$ | $CH_2$ | I |
| 356 | CO | - | $CH_2$ | 3 | 0 | Br | H | $CH_2$ | $CH_2$ | I |

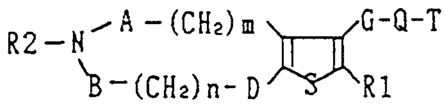

| No. | A | B | D | m | n | R1 | R2 | G | Q | T |
|---|---|---|---|---|---|---|---|---|---|---|
| 357 | - | CO | $CH_2$ | 0 | 1 | H | H | CO | $(CH_2)_3$ | I |
| 358 | - | CO | $CH_2$ | 0 | 1 | Me | H | CO | $(CH_2)_3$ | I |
| 359 | - | CO | $CH_2$ | 0 | 1 | Et | H | CO | $(CH_2)_3$ | I |
| 360 | - | CO | $CH_2$ | 0 | 1 | $COCH_3$ | H | CO | $(CH_2)_3$ | I |
| 361 | - | CO | $CH_2$ | 0 | 1 | $COC_6H_5$ | H | CO | $(CH_2)_3$ | I |
| 362 | - | CO | $CH_2$ | 0 | 1 | F | H | CO | $(CH_2)_3$ | I |
| 363 | - | CO | $CH_2$ | 0 | 1 | Cl | H | CO | $(CH_2)_3$ | I |
| 364 | - | CO | $CH_2$ | 0 | 1 | Br | H | CO | $(CH_2)_3$ | I |
| 365 | - | CO | $CH_2$ | 0 | 1 | $NO_2$ | H | CO | $(CH_2)_3$ | I |
| 366 | - | CO | $CH_2$ | 0 | 1 | $NH_2$ | H | CO | $(CH_2)_3$ | I |
| 367 | - | CO | $CH_2$ | 0 | 1 | CN | H | CO | $(CH_2)_3$ | I |
| 368 | - | CO | $CH_2$ | 0 | 1 | OH | H | CO | $(CH_2)_3$ | I |
| 369 | - | CO | $CH_2$ | 0 | 1 | CHO | H | CO | $(CH_2)_3$ | I |
| 370 | - | CO | $CH_2$ | 0 | 1 | OMe | H | CO | $(CH_2)_3$ | I |
| 371 | - | CO | $CH_2$ | 0 | 1 | $CF_3$ | H | CO | $(CH_2)_3$ | I |
| 372 | - | CO | $CH_2$ | 0 | 1 | $CH_2C_6H_5$ | H | CO | $(CH_2)_3$ | I |
| 373 | - | CO | $CH_2$ | 0 | 1 | $OCOCH_3$ | H | CO | $(CH_2)_3$ | I |
| 374 | - | CO | $CH_2$ | 0 | 1 | $CH_2OCOCH_3$ | H | CO | $(CH_2)_3$ | I |

| No. | A | B | D | m | n | R1 | R2 | G | Q | T |
|---|---|---|---|---|---|---|---|---|---|---|
| 375 | - | CO | $CH_2$ | 0 | 1 | $CH_2OCH_3$ | H | CO | $(CH_2)_3$ | I |
| 376 | - | CO | $CH_2$ | 0 | 1 | $CH_2OH$ | H | CO | $(CH_2)_3$ | I |
| 377 | - | CO | $CH_2$ | 0 | 1 | $COCH_2OCOMe$ | H | CO | $(CH_2)_3$ | I |
| 378 | - | CO | $CH_2$ | 0 | 1 | $COCH_2OMe$ | H | CO | $(CH_2)_3$ | I |
| 379 | - | CO | $CH_2$ | 0 | 1 | $COCH_2OH$ | H | CO | $(CH_2)_3$ | I |
| 380 | - | CO | $CH_2$ | 0 | 1 | $COCH_2OC_6H_5$ | H | CO | $(CH_2)_3$ | I |
| 381 | - | CO | $CH_2$ | 0 | 1 | $COCF_3$ | H | CO | $(CH_2)_3$ | I |
| 382 | - | CO | $CH_2$ | 0 | 1 | SMe | H | CO | $(CH_2)_3$ | I |
| 383 | - | CO | $CH_2$ | 0 | 1 | SOMe | H | CO | $(CH_2)_3$ | I |
| 384 | - | CO | $CH_2$ | 0 | 1 | $SO_2Me$ | H | CO | $(CH_2)_3$ | I |
| 385 | - | CO | $CH_2$ | 0 | 1 | $SC_6H_5$ | H | CO | $(CH_2)_3$ | I |
| 386 | - | CO | $CH_2$ | 0 | 1 | $SOC_6H_5$ | H | CO | $(CH_2)_3$ | I |
| 387 | - | CO | $CH_2$ | 0 | 1 | $SO_2C_6H_5$ | H | CO | $(CH_2)_3$ | I |
| 388 | - | CO | $CH_2$ | 0 | 1 | $SO_3H$ | H | CO | $(CH_2)_3$ | I |
| 389 | - | CO | $CH_2$ | 0 | 1 | $SO_2Cl$ | H | CO | $(CH_2)_3$ | I |
| 390 | - | CO | $CH_2$ | 0 | 1 | $SO_2NH_2$ | H | CO | $(CH_2)_3$ | I |
| 391 | - | CO | $CH_2$ | 0 | 1 | $SO_2NMe_2$ | H | CO | $(CH_2)_3$ | I |
| 392 | - | CO | $CH_2$ | 0 | 1 | COOH | H | CO | $(CH_2)_3$ | I |
| 393 | - | CO | $CH_2$ | 0 | 1 | $NHCOCH_3$ | H | CO | $(CH_2)_3$ | I |
| 394 | - | CO | $CH_2$ | 0 | 1 | COOMe | H | CO | $(CH_2)_3$ | I |
| 395 | - | CO | $CH_2$ | 0 | 1 | $CONH_2$ | H | CO | $(CH_2)_3$ | I |
| 396 | - | CO | $CH_2$ | 0 | 1 | $CONMe_2$ | H | CO | $(CH_2)_3$ | I |

| No. | A | B | D | m | n | R1 | R2 | G | Q | T |
|---|---|---|---|---|---|---|---|---|---|---|
| 397 | - | CO | $CH_2$ | 0 | 1 | $NMe_2$ | H | CO | $(CH_2)_3$ | I |
| 398 | - | CO | $CH_2$ | 0 | 1 | H | H | $CH_2$ | $CH_2$ | I |
| 399 | - | CO | $CH_2$ | 0 | 1 | Me | H | $CH_2$ | $CH_2$ | I |
| 400 | - | CO | $CH_2$ | 0 | 1 | Et | H | $CH_2$ | $CH_2$ | I |
| 401 | - | CO | $CH_2$ | 0 | 1 | $COCH_3$ | H | $CH_2$ | $CH_2$ | I |
| 402 | - | CO | $CH_2$ | 0 | 1 | $COC_6H_5$ | H | $CH_2$ | $CH_2$ | I |
| 403 | - | CO | $CH_2$ | 0 | 1 | F | H | $CH_2$ | $CH_2$ | I |
| 404 | - | CO | $CH_2$ | 0 | 1 | Cl | H | $CH_2$ | $CH_2$ | I |
| 405 | - | CO | $CH_2$ | 0 | 1 | Br | H | $CH_2$ | $CH_2$ | I |
| 406 | - | CO | $CH_2$ | 0 | 1 | $NO_2$ | H | $CH_2$ | $CH_2$ | I |
| 407 | - | CO | $CH_2$ | 0 | 1 | $NH_2$ | H | $CH_2$ | $CH_2$ | I |
| 408 | - | CO | $CH_2$ | 0 | 1 | CN | H | $CH_2$ | $CH_2$ | I |
| 409 | - | CO | $CH_2$ | 0 | 1 | OH | H | $CH_2$ | $CH_2$ | I |
| 410 | - | CO | $CH_2$ | 0 | 1 | CHO | H | $CH_2$ | $CH_2$ | I |
| 411 | - | CO | $CH_2$ | 0 | 1 | OMe | H | $CH_2$ | $CH_2$ | I |
| 412 | - | CO | $CH_2$ | 0 | 1 | $CF_3$ | H | $CH_2$ | $CH_2$ | I |
| 413 | - | CO | $CH_2$ | 0 | 1 | $CH_2C_6H_5$ | H | $CH_2$ | $CH_2$ | I |
| 414 | - | CO | $CH_2$ | 0 | 1 | $OCOCH_3$ | H | $CH_2$ | $CH_2$ | I |
| 415 | - | CO | $CH_2$ | 0 | 1 | $CH_2OCOCH_3$ | H | $CH_2$ | $CH_2$ | I |
| 416 | - | CO | $CH_2$ | 0 | 1 | $CH_2OCH_3$ | H | $CH_2$ | $CH_2$ | I |
| 417 | - | CO | $CH_2$ | 0 | 1 | $CH_2OH$ | H | $CH_2$ | $CH_2$ | I |

| No. | A | B | D | m | n | R1 | R2 | G | Q | T |
|---|---|---|---|---|---|---|---|---|---|---|
| 418 | - | CO | $CH_2$ | 0 | 1 | $COCH_2OCOMe$ | H | $CH_2$ | $CH_2$ | I |
| 419 | - | CO | $CH_2$ | 0 | 1 | $COCH_2OMe$ | H | $CH_2$ | $CH_2$ | I |
| 420 | - | CO | $CH_2$ | 0 | 1 | $COCH_2OH$ | H | $CH_2$ | $CH_2$ | I |
| 421 | - | CO | $CH_2$ | 0 | 1 | $COCH_2OC_6H_5$ | H | $CH_2$ | $CH_2$ | I |
| 422 | - | CO | $CH_2$ | 0 | 1 | $COCF_3$ | H | $CH_2$ | $CH_2$ | I |
| 423 | - | CO | $CH_2$ | 0 | 1 | SMe | H | $CH_2$ | $CH_2$ | I |
| 424 | - | CO | $CH_2$ | 0 | 1 | SOMe | H | $CH_2$ | $CH_2$ | I |
| 425 | - | CO | $CH_2$ | 0 | 1 | $SO_2Me$ | H | $CH_2$ | $CH_2$ | I |
| 426 | - | CO | $CH_2$ | 0 | 1 | $SC_6H_5$ | H | $CH_2$ | $CH_2$ | I |
| 427 | - | CO | $CH_2$ | 0 | 1 | $SOC_6H_5$ | H | $CH_2$ | $CH_2$ | I |
| 428 | - | CO | $CH_2$ | 0 | 1 | $SO_2C_5H_5$ | H | $CH_2$ | $CH_2$ | I |
| 429 | - | CO | $CH_2$ | 0 | 1 | $SO_3H$ | H | $CH_2$ | $CH_2$ | I |
| 430 | - | CO | $CH_2$ | 0 | 1 | $SO_2Cl$ | H | $CH_2$ | $CH_2$ | I |
| 431 | - | CO | $CH_2$ | 0 | 1 | $SO_2NH_2$ | H | $CH_2$ | $CH_2$ | I |
| 432 | - | CO | $CH_2$ | 0 | 1 | $SO_2NMe_2$ | H | $CH_2$ | $CH_2$ | I |
| 433 | - | CO | $CH_2$ | 0 | 1 | COOH | H | $CH_2$ | $CH_2$ | I |
| 434 | - | CO | $CH_2$ | 0 | 1 | $NHCOCH_3$ | H | $CH_2$ | $CH_2$ | I |
| 435 | - | CO | $CH_2$ | 0 | 1 | COOMe | H | $CH_2$ | $CH_2$ | I |
| 436 | - | CO | $CH_2$ | 0 | 1 | $CONH_2$ | H | $CH_2$ | $CH_2$ | I |
| 437 | - | CO | $CH_2$ | 0 | 1 | $CONMe_2$ | H | $CH_2$ | $CH_2$ | I |
| 438 | - | CO | $CH_2$ | 0 | 1 | $NMe_2$ | H | $CH_2$ | $CH_2$ | I |

| No. | A | B | D | m | n | R1 | R2 | G | Q | T |
|---|---|---|---|---|---|---|---|---|---|---|
| 439 | CO | - | $CH_2$ | 0 | 1 | H | H | CO | $(CH_2)_3$ | I |
| 440 | CO | - | $CH_2$ | 0 | 1 | Me | H | CO | $(CH_2)_3$ | I |
| 441 | CO | - | $CH_2$ | 0 | 1 | Et | H | CO | $(CH_2)_3$ | I |
| 442 | CO | - | $CH_2$ | 0 | 1 | $COCH_3$ | H | CO | $(CH_2)_3$ | I |
| 443 | CO | - | $CH_2$ | 0 | 1 | $COC_6H_5$ | H | CO | $(CH_2)_3$ | I |
| 444 | CO | - | $CH_2$ | 0 | 1 | F | H | CO | $(CH_2)_3$ | I |
| 445 | CO | - | $CH_2$ | 0 | 1 | Cl | H | CO | $(CH_2)_3$ | I |
| 446 | CO | - | $CH_2$ | 0 | 1 | Br | H | CO | $(CH_2)_3$ | I |
| 447 | CO | - | $CH_2$ | 0 | 1 | $NO_2$ | H | CO | $(CH_2)_3$ | I |
| 448 | CO | - | $CH_2$ | 0 | 1 | $NH_2$ | H | CO | $(CH_2)_3$ | I |
| 449 | CO | - | $CH_2$ | 0 | 1 | CN | H | CO | $(CH_2)_3$ | I |
| 450 | CO | - | $CH_2$ | 0 | 1 | OH | H | CO | $(CH_2)_3$ | I |
| 451 | CO | - | $CH_2$ | 0 | 1 | CHO | H | CO | $(CH_2)_3$ | I |
| 452 | CO | - | $CH_2$ | 0 | 1 | OMe | H | CO | $(CH_2)_3$ | I |
| 453 | CO | - | $CH_2$ | 0 | 1 | $CF_3$ | H | CO | $(CH_2)_3$ | I |
| 454 | CO | - | $CH_2$ | 0 | 1 | $CH_2C_6H_5$ | H | CO | $(CH_2)_3$ | I |
| 455 | CO | - | $CH_2$ | 0 | 1 | $OCOCH_3$ | H | CO | $(CH_2)_3$ | I |
| 456 | CO | - | $CH_2$ | 0 | 1 | $CH_2OCOCH_3$ | H | CO | $(CH_2)_3$ | I |
| 457 | CO | - | $CH_2$ | 0 | 1 | $CH_2OCH_3$ | H | CO | $(CH_2)_3$ | I |
| 458 | CO | - | $CH_2$ | 0 | 1 | $CH_2OH$ | H | CO | $(CH_2)_3$ | I |
| 459 | CO | - | $CH_2$ | 0 | 1 | $COCH_2OCOMe$ | H | CO | $(CH_2)_3$ | I |
| 460 | CO | - | $CH_2$ | 0 | 1 | $COCH_2OMe$ | H | CO | $(CH_2)_3$ | I |

| No. | A | B | D | m | n | R1 | R2 | G | Q | T |
|---|---|---|---|---|---|---|---|---|---|---|
| 461 | CO | - | $CH_2$ | 0 | 1 | $COCH_2OH$ | H | CO | $(CH_2)_3$ | I |
| 462 | CO | - | $CH_2$ | 0 | 1 | $COCH_2OC_6H_5$ | H | CO | $(CH_2)_3$ | I |
| 463 | CO | - | $CH_2$ | 0 | 1 | $COCF_3$ | H | CO | $(CH_2)_3$ | I |
| 464 | CO | - | $CH_2$ | 0 | 1 | SMe | H | CO | $(CH_2)_3$ | I |
| 465 | CO | - | $CH_2$ | 0 | 1 | SOMe | H | CO | $(CH_2)_3$ | I |
| 466 | CO | - | $CH_2$ | 0 | 1 | $SO_2Me$ | H | CO | $(CH_2)_3$ | I |
| 467 | CO | - | $CH_2$ | 0 | 1 | $SC_6H_5$ | H | CO | $(CH_2)_3$ | I |
| 468 | CO | - | $CH_2$ | 0 | 1 | $SOC_6H_5$ | H | CO | $(CH_2)_3$ | I |
| 469 | CO | - | $CH_2$ | 0 | 1 | $SO_2C_6H_5$ | H | CO | $(CH_2)_3$ | I |
| 470 | CO | - | $CH_2$ | 0 | 1 | $SO_3H$ | H | CO | $(CH_2)_3$ | I |
| 471 | CO | - | $CH_2$ | 0 | 1 | $SO_2Cl$ | H | CO | $(CH_2)_3$ | I |
| 472 | CO | - | $CH_2$ | 0 | 1 | $SO_2NH_2$ | H | CO | $(CH_2)_3$ | I |
| 473 | CO | - | $CH_2$ | 0 | 1 | $SO_2NMe_2$ | H | CO | $(CH_2)_3$ | I |
| 474 | CO | - | $CH_2$ | 0 | 1 | COOH | H | CO | $(CH_2)_3$ | I |
| 475 | CO | - | $CH_2$ | 0 | 1 | $NHCOCH_3$ | H | CO | $(CH_2)_3$ | I |
| 476 | CO | - | $CH_2$ | 0 | 1 | COOMe | H | CO | $(CH_2)_3$ | I |
| 477 | CO | - | $CH_2$ | 0 | 1 | $CONH_2$ | H | CO | $(CH_2)_3$ | I |
| 478 | CO | - | $CH_2$ | 0 | 1 | $CONMe_2$ | H | CO | $(CH_2)_3$ | I |
| 479 | CO | - | $CH_2$ | 0 | 1 | $NMe_2$ | H | CO | $(CH_2)_3$ | I |
| 480 | CO | - | $CH_2$ | 0 | 1 | H | H | $CH_2$ | $CH_2$ | I |
| 481 | CO | - | $CH_2$ | 0 | 1 | Me | H | $CH_2$ | $CH_2$ | I |

| No. | A | B | D | m | n | R1 | R2 | G | Q | T |
|---|---|---|---|---|---|---|---|---|---|---|
| 482 | CO | - | $CH_2$ | 0 | 1 | Et | H | $CH_2$ | $CH_2$ | I |
| 483 | CO | - | $CH_2$ | 0 | 1 | $COCH_3$ | H | $CH_2$ | $CH_2$ | I |
| 484 | CO | - | $CH_2$ | 0 | 1 | $COC_6H_5$ | H | $CH_2$ | $CH_2$ | I |
| 485 | CO | - | $CH_2$ | 0 | 1 | F | H | $CH_2$ | $CH_2$ | I |
| 486 | CO | - | $CH_2$ | 0 | 1 | Cl | H | $CH_2$ | $CH_2$ | I |
| 487 | CO | - | $CH_2$ | 0 | 1 | Br | H | $CH_2$ | $CH_2$ | I |
| 488 | CO | - | $CH_2$ | 0 | 1 | $NO_2$ | H | $CH_2$ | $CH_2$ | I |
| 489 | CO | - | $CH_2$ | 0 | 1 | $NH_2$ | H | $CH_2$ | $CH_2$ | I |
| 490 | CO | - | $CH_2$ | 0 | 1 | CN | H | $CH_2$ | $CH_2$ | I |
| 491 | CO | - | $CH_2$ | 0 | 1 | OH | H | $CH_2$ | $CH_2$ | I |
| 492 | CO | - | $CH_2$ | 0 | 1 | CHO | H | $CH_2$ | $CH_2$ | I |
| 493 | CO | - | $CH_2$ | 0 | 1 | OMe | H | $CH_2$ | $CH_2$ | I |
| 494 | CO | - | $CH_2$ | 0 | 1 | $CF_3$ | H | $CH_2$ | $CH_2$ | I |
| 495 | CO | - | $CH_2$ | 0 | 1 | $CH_2C_6H_5$ | H | $CH_2$ | $CH_2$ | I |
| 496 | CO | - | $CH_2$ | 0 | 1 | $OCOCH_3$ | H | $CH_2$ | $CH_2$ | I |
| 497 | CO | - | $CH_2$ | 0 | 1 | $CH_2OCOCH_3$ | H | $CH_2$ | $CH_2$ | I |
| 498 | CO | - | $CH_2$ | 0 | 1 | $CH_2OCH_3$ | H | $CH_2$ | $CH_2$ | I |
| 499 | CO | - | $CH_2$ | 0 | 1 | $CH_2OH$ | H | $CH_2$ | $CH_2$ | I |
| 500 | CO | - | $CH_2$ | 0 | 1 | $COCH_2OCOMe$ | H | $CH_2$ | $CH_2$ | I |
| 501 | CO | - | $CH_2$ | 0 | 1 | $COCH_2OMe$ | H | $CH_2$ | $CH_2$ | I |
| 502 | CO | - | $CH_2$ | 0 | 1 | $COCH_2OH$ | H | $CH_2$ | $CH_2$ | I |
| 503 | CO | - | $CH_2$ | 0 | 1 | $COCH_2OC_6H_5$ | H | $CH_2$ | $CH_2$ | I |

| No. | A | B | D | m | n | R1 | R2 | G | Q | T |
|---|---|---|---|---|---|---|---|---|---|---|
| 504 | CO | - | $CH_2$ | 0 | 1 | $COCF_3$ | H | $CH_2$ | $CH_2$ | I |
| 505 | CO | - | $CH_2$ | 0 | 1 | SMe | H | $CH_2$ | $CH_2$ | I |
| 506 | CO | - | $CH_2$ | 0 | 1 | SOMe | H | $CH_2$ | $CH_2$ | I |
| 507 | CO | - | $CH_2$ | 0 | 1 | $SO_2Me$ | H | $CH_2$ | $CH_2$ | I |
| 508 | CO | - | $CH_2$ | 0 | 1 | $SC_6H_5$ | H | $CH_2$ | $CH_2$ | I |
| 509 | CO | - | $CH_2$ | 0 | 1 | $SOC_6H_5$ | H | $CH_2$ | $CH_2$ | I |
| 510 | CO | - | $CH_2$ | 0 | 1 | $SO_2C_6H_5$ | H | $CH_2$ | $CH_2$ | I |
| 511 | CO | - | $CH_2$ | 0 | 1 | $SO_3H$ | H | $CH_2$ | $CH_2$ | I |
| 512 | CO | - | $CH_2$ | 0 | 1 | $SO_2Cl$ | H | $CH_2$ | $CH_2$ | I |
| 513 | CO | - | $CH_2$ | 0 | 1 | $SO_2NH_2$ | H | $CH_2$ | $CH_2$ | I |
| 514 | CO | - | $CH_2$ | 0 | 1 | $SO_2NMe_2$ | H | $CH_2$ | $CH_2$ | I |
| 515 | CO | - | $CH_2$ | 0 | 1 | COOH | H | $CH_2$ | $CH_2$ | I |
| 516 | CO | - | $CH_2$ | 0 | 1 | $NHCOCH_3$ | H | $CH_2$ | $CH_2$ | I |
| 517 | CO | - | $CH_2$ | 0 | 1 | COOMe | H | $CH_2$ | $CH_2$ | I |
| 518 | CO | - | $CH_2$ | 0 | 1 | $CONH_2$ | H | $CH_2$ | $CH_2$ | I |
| 519 | CO | - | $CH_2$ | 0 | 1 | $CONMe_2$ | H | $CH_2$ | $CH_2$ | I |
| 520 | CO | - | $CH_2$ | 0 | 1 | $NMe_2$ | H | $CH_2$ | $CH_2$ | I |
| 521 | - | CO | $CH_2$ | 1 | 0 | H | H | CO | $(CH_2)_3$ | I |
| 522 | - | CO | $CH_2$ | 1 | 0 | Me | H | CO | $(CH_2)_3$ | I |
| 523 | - | CO | $CH_2$ | 1 | 0 | Et | H | CO | $(CH_2)_3$ | I |
| 524 | - | CO | $CH_2$ | 1 | 0 | $COCH_3$ | H | CO | $(CH_2)_3$ | I |

| No. | A | B | D | m | n | R1 | R2 | G | Q | T |
|---|---|---|---|---|---|---|---|---|---|---|
| 525 | - | CO | $CH_2$ | 1 | 0 | $COC_6H_5$ | H | CO | $(CH_2)_3$ | I |
| 526 | - | CO | $CH_2$ | 1 | 0 | Br | H | CO | $(CH_2)_3$ | I |
| 527 | - | CO | $CH_2$ | 1 | 0 | H | H | $CH_2$ | $CH_2$ | I |
| 528 | - | CO | $CH_2$ | 1 | 0 | Me | H | $CH_2$ | $CH_2$ | I |
| 529 | - | CO | $CH_2$ | 1 | 0 | Et | H | $CH_2$ | $CH_2$ | I |
| 530 | - | CO | $CH_2$ | 1 | 0 | $COCH_3$ | H | $CH_2$ | $CH_2$ | I |
| 531 | - | CO | $CH_2$ | 1 | 0 | $COC_6H_5$ | H | $CH_2$ | $CH_2$ | I |
| 532 | - | CO | $CH_2$ | 1 | 0 | Br | H | $CH_2$ | $CH_2$ | I |
| 533 | CO | - | $CH_2$ | 1 | 0 | H | H | CO | $(CH_2)_3$ | I |
| 534 | CO | - | $CH_2$ | 1 | 0 | Me | H | CO | $(CH_2)_3$ | I |
| 535 | CO | - | $CH_2$ | 1 | 0 | Et | H | CO | $(CH_2)_3$ | I |
| 536 | CO | - | $CH_2$ | 1 | 0 | $COCH_3$ | H | CO | $(CH_2)_3$ | I |
| 537 | CO | - | $CH_2$ | 1 | 0 | $COC_6H_5$ | H | CO | $(CH_2)_3$ | I |
| 538 | CO | - | $CH_2$ | 1 | 0 | Br | H | CO | $(CH_2)_3$ | I |
| 539 | CO | - | $CH_2$ | 1 | 0 | H | H | $CH_2$ | $CH_2$ | I |
| 540 | CO | - | $CH_2$ | 1 | 0 | Me | H | $CH_2$ | $CH_2$ | I |
| 541 | CO | - | $CH_2$ | 1 | 0 | Et | H | $CH_2$ | $CH_2$ | I |
| 542 | CO | - | $CH_2$ | 1 | 0 | $COCH_3$ | H | $CH_2$ | $CH_2$ | I |
| 543 | CO | - | $CH_2$ | 1 | 0 | $COC_6H_5$ | H | $CH_2$ | $CH_2$ | I |
| 544 | CO | - | $CH_2$ | 1 | 0 | Br | H | $CH_2$ | $CH_2$ | I |

| No. | A | B | D | m | n | R1 | R2 | G | Q | T |
|---|---|---|---|---|---|---|---|---|---|---|
| 545 | - | CO | $CH_2$ | 0 | 2 | H | H | CO | $(CH_2)_3$ | I |
| 546 | - | CO | $CH_2$ | 0 | 2 | Me | H | CO | $(CH_2)_3$ | I |
| 547 | - | CO | $CH_2$ | 0 | 2 | Et | H | CO | $(CH_2)_3$ | I |
| 548 | - | CO | $CH_2$ | 0 | 2 | $COCH_3$ | H | CO | $(CH_2)_3$ | I |
| 549 | - | CO | $CH_2$ | 0 | 2 | $COC_6H_5$ | H | CO | $(CH_2)_3$ | I |
| 550 | - | CO | $CH_2$ | 0 | 2 | Br | H | CO | $(CH_2)_3$ | I |
| 551 | - | CO | $CH_2$ | 0 | 2 | H | H | $CH_2$ | $CH_2$ | I |
| 552 | - | CO | $CH_2$ | 0 | 2 | Me | H | $CH_2$ | $CH_2$ | I |
| 553 | - | CO | $CH_2$ | 0 | 2 | Et | H | $CH_2$ | $CH_2$ | I |
| 554 | - | CO | $CH_2$ | 0 | 2 | $COCH_3$ | H | $CH_2$ | $CH_2$ | I |
| 555 | - | CO | $CH_2$ | 0 | 2 | $COC_6H_5$ | H | $CH_2$ | $CH_2$ | I |
| 556 | - | CO | $CH_2$ | 0 | 2 | Br | H | $CH_2$ | $CH_2$ | I |
| 557 | CO | - | $CH_2$ | 0 | 2 | H | H | CO | $(CH_2)_3$ | I |
| 558 | CO | - | $CH_2$ | 0 | 2 | Me | H | CO | $(CH_2)_3$ | I |
| 559 | CO | - | $CH_2$ | 0 | 2 | Et | H | CO | $(CH_2)_3$ | I |
| 560 | CO | - | $CH_2$ | 0 | 2 | $COCH_3$ | H | CO | $(CH_2)_3$ | I |
| 561 | CO | - | $CH_2$ | 0 | 2 | $COC_6H_5$ | H | CO | $(CH_2)_3$ | I |
| 562 | CO | - | $CH_2$ | 0 | 2 | Br | H | CO | $(CH_2)_3$ | I |
| 563 | CO | - | $CH_2$ | 0 | 2 | H | H | $CH_2$ | $CH_2$ | I |
| 564 | CO | - | $CH_2$ | 0 | 2 | Me | H | $CH_2$ | $CH_2$ | I |
| 565 | CO | - | $CH_2$ | 0 | 2 | Et | H | $CH_2$ | $CH_2$ | I |
| 566 | CO | - | $CH_2$ | 0 | 2 | $COCH_3$ | H | $CH_2$ | $CH_2$ | I |

| No. | A | B | D | m | n | R1 | R2 | G | Q | T |
|---|---|---|---|---|---|---|---|---|---|---|
| 567 | CO | - | $CH_2$ | 0 | 2 | $COC_6H_5$ | H | $CH_2$ | $CH_2$ | I |
| 568 | CO | - | $CH_2$ | 0 | 2 | Br | H | $CH_2$ | $CH_2$ | I |
| 569 | - | CO | $CH_2$ | 1 | 1 | H | H | CO | $(CH_2)_3$ | I |
| 570 | - | CO | $CH_2$ | 1 | 1 | Me | H | CO | $(CH_2)_3$ | I |
| 571 | - | CO | $CH_2$ | 1 | 1 | Et | H | CO | $(CH_2)_3$ | I |
| 572 | - | CO | $CH_2$ | 1 | 1 | $COCH_3$ | H | CO | $(CH_2)_3$ | I |
| 573 | - | CO | $CH_2$ | 1 | 1 | $COC_6H_5$ | H | CO | $(CH_2)_3$ | I |
| 574 | - | CO | $CH_2$ | 1 | 1 | Br | H | CO | $(CH_2)_3$ | I |
| 575 | - | CO | $CH_2$ | 1 | 1 | H | H | $CH_2$ | $CH_2$ | I |
| 576 | - | CO | $CH_2$ | 1 | 1 | Me | H | $CH_2$ | $CH_2$ | I |
| 577 | - | CO | $CH_2$ | 1 | 1 | Et | H | $CH_2$ | $CH_2$ | I |
| 578 | - | CO | $CH_2$ | 1 | 1 | $COCH_3$ | H | $CH_2$ | $CH_2$ | I |
| 579 | - | CO | $CH_2$ | 1 | 1 | $COC_5H_5$ | H | $CH_2$ | $CH_2$ | I |
| 580 | - | CO | $CH_2$ | 1 | 1 | Br | H | $CH_2$ | $CH_2$ | I |
| 581 | CO | - | $CH_2$ | 1 | 1 | H | H | CO | $(CH_2)_3$ | I |
| 582 | CO | - | $CH_2$ | 1 | 1 | Me | H | CO | $(CH_2)_3$ | I |
| 583 | CO | - | $CH_2$ | 1 | 1 | Et | H | CO | $(CH_2)_3$ | I |
| 584 | CO | - | $CH_2$ | 1 | 1 | $COCH_3$ | H | CO | $(CH_2)_3$ | I |
| 585 | CO | - | $CH_2$ | 1 | 1 | $COC_6H_5$ | H | CO | $(CH_2)_3$ | I |
| 586 | CO | - | $CH_2$ | 1 | 1 | Br | H | CO | $(CH_2)_3$ | I |
| 587 | CO | - | $CH_2$ | 1 | 1 | H | H | $CH_2$ | $CH_2$ | I |

| No. | A | B | D | m | n | R1 | R2 | G | Q | T |
|---|---|---|---|---|---|---|---|---|---|---|
| 588 | CO | - | $CH_2$ | 1 | 1 | Me | H | $CH_2$ | $CH_2$ | I |
| 589 | CO | - | $CH_2$ | 1 | 1 | Et | H | $CH_2$ | $CH_2$ | I |
| 590 | CO | - | $CH_2$ | 1 | 1 | $COCH_3$ | H | $CH_2$ | $CH_2$ | I |
| 591 | CO | - | $CH_2$ | 1 | 1 | $COC_6H_5$ | H | $CH_2$ | $CH_2$ | I |
| 592 | CO | - | $CH_2$ | 1 | 1 | Br | H | $CH_2$ | $CH_2$ | I |
| 593 | - | CO | $CH_2$ | 2 | 0 | H | H | CO | $(CH_2)_3$ | I |
| 594 | - | CO | $CH_2$ | 2 | 0 | Me | H | CO | $(CH_2)_3$ | I |
| 595 | - | CO | $CH_2$ | 2 | 0 | Et | H | CO | $(CH_2)_3$ | I |
| 596 | - | CO | $CH_2$ | 2 | 0 | $COCH_3$ | H | CO | $(CH_2)_3$ | I |
| 597 | - | CO | $CH_2$ | 2 | 0 | $COC_6H_5$ | H | CO | $(CH_2)_3$ | I |
| 598 | - | CO | $CH_2$ | 2 | 0 | Br | H | CO | $(CH_2)_3$ | I |
| 599 | - | CO | $CH_2$ | 2 | 0 | H | H | $CH_2$ | $CH_2$ | I |
| 600 | - | CO | $CH_2$ | 2 | 0 | Me | H | $CH_2$ | $CH_2$ | I |
| 601 | - | CO | $CH_2$ | 2 | 0 | Et | H | $CH_2$ | $CH_2$ | I |
| 602 | - | CO | $CH_2$ | 2 | 0 | $COCH_3$ | H | $CH_2$ | $CH_2$ | I |
| 603 | - | CO | $CH_2$ | 2 | 0 | $COC_6H_5$ | H | $CH_2$ | $CH_2$ | I |
| 604 | - | CO | $CH_2$ | 2 | 0 | Br | H | $CH_2$ | $CH_2$ | I |
| 605 | CO | - | $CH_2$ | 2 | 0 | H | H | CO | $(CH_2)_3$ | I |
| 606 | CO | - | $CH_2$ | 2 | 0 | Me | H | CO | $(CH_2)_3$ | I |
| 607 | CO | - | $CH_2$ | 2 | 0 | Et | H | CO | $(CH_2)_3$ | I |
| 608 | CO | - | $CH_2$ | 2 | 0 | $COCH_3$ | H | CO | $(CH_2)_3$ | I |
| 609 | CO | - | $CH_2$ | 2 | 0 | $COC_6H_5$ | H | CO | $(CH_2)_3$ | I |

| No. | A | B | D | m | n | R1 | R2 | G | Q | T |
|---|---|---|---|---|---|---|---|---|---|---|
| 610 | CO | - | $CH_2$ | 2 | 0 | Br | H | CO | $(CH_2)_3$ | I |
| 611 | CO | - | $CH_2$ | 2 | 0 | H | H | $CH_2$ | $CH_2$ | I |
| 612 | CO | - | $CH_2$ | 2 | 0 | Me | H | $CH_2$ | $CH_2$ | I |
| 613 | CO | - | $CH_2$ | 2 | 0 | Et | H | $CH_2$ | $CH_2$ | I |
| 614 | CO | - | $CH_2$ | 2 | 0 | $COCH_3$ | H | $CH_2$ | $CH_2$ | I |
| 615 | CO | - | $CH_2$ | 2 | 0 | $COC_6H_5$ | H | $CH_2$ | $CH_2$ | I |
| 616 | CO | - | $CH_2$ | 2 | 0 | Br | H | $CH_2$ | $CH_2$ | I |
| 617 | - | CO | $CH_2$ | 0 | 3 | H | H | CO | $(CH_2)_3$ | I |
| 618 | - | CO | $CH_2$ | 0 | 3 | Me | H | CO | $(CH_2)_3$ | I |
| 619 | - | CO | $CH_2$ | 0 | 3 | Et | H | CO | $(CH_2)_3$ | I |
| 620 | - | CO | $CH_2$ | 0 | 3 | $COCH_3$ | H | CO | $(CH_2)_3$ | I |
| 621 | - | CO | $CH_2$ | 0 | 3 | $COC_6H_5$ | H | CO | $(CH_2)_3$ | I |
| 622 | - | CO | $CH_2$ | 0 | 3 | Br | H | CO | $(CH_2)_3$ | I |
| 623 | - | CO | $CH_2$ | 0 | 3 | H | H | $CH_2$ | $CH_2$ | I |
| 624 | - | CO | $CH_2$ | 0 | 3 | Me | H | $CH_2$ | $CH_2$ | I |
| 625 | - | CO | $CH_2$ | 0 | 3 | Et | H | $CH_2$ | $CH_2$ | I |
| 626 | - | CO | $CH_2$ | 0 | 3 | $COCH_3$ | H | $CH_2$ | $CH_2$ | I |
| 627 | - | CO | $CH_2$ | 0 | 3 | $COC_6H_5$ | H | $CH_2$ | $CH_2$ | I |
| 628 | - | CO | $CH_2$ | 0 | 3 | Br | H | $CH_2$ | $CH_2$ | I |
| 629 | CO | - | $CH_2$ | 0 | 3 | H | H | CO | $(CH_2)_3$ | I |
| 630 | CO | - | $CH_2$ | 0 | 3 | Me | H | CO | $(CH_2)_3$ | I |

| No. | A | B | D | m | n | R1 | R2 | G | Q | T |
|---|---|---|---|---|---|---|---|---|---|---|
| 631 | CO | - | $CH_2$ | 0 | 3 | Et | H | CO | $(CH_2)_3$ | I |
| 632 | CO | - | $CH_2$ | 0 | 3 | $COCH_3$ | H | CO | $(CH_2)_3$ | I |
| 633 | CO | - | $CH_2$ | 0 | 3 | $COC_6H_5$ | H | CO | $(CH_2)_3$ | I |
| 634 | CO | - | $CH_2$ | 0 | 3 | Br | H | CO | $(CH_2)_3$ | I |
| 635 | CO | - | $CH_2$ | 0 | 3 | H | H | $CH_2$ | $CH_2$ | I |
| 636 | CO | - | $CH_2$ | 0 | 3 | Me | H | $CH_2$ | $CH_2$ | I |
| 637 | CO | - | $CH_2$ | 0 | 3 | Et | H | $CH_2$ | $CH_2$ | I |
| 638 | CO | - | $CH_2$ | 0 | 3 | $COCH_3$ | H | $CH_2$ | $CH_2$ | I |
| 639 | CO | - | $CH_2$ | 0 | 3 | $COC_6H_5$ | H | $CH_2$ | $CH_2$ | I |
| 640 | CO | - | $CH_2$ | 0 | 3 | Br | H | $CH_2$ | $CH_2$ | I |
| 641 | - | CO | $CH_2$ | 1 | 2 | H | H | CO | $(CH_2)_3$ | I |
| 642 | - | CO | $CH_2$ | 1 | 2 | Me | H | CO | $(CH_2)_3$ | I |
| 643 | - | CO | $CH_2$ | 1 | 2 | Et | H | CO | $(CH_2)_3$ | I |
| 644 | - | CO | $CH_2$ | 1 | 2 | $COCH_3$ | H | CO | $(CH_2)_3$ | I |
| 645 | - | CO | $CH_2$ | 1 | 2 | $COC_6H_5$ | H | CO | $(CH_2)_3$ | I |
| 646 | - | CO | $CH_2$ | 1 | 2 | Br | H | CO | $(CH_2)_3$ | I |
| 647 | - | CO | $CH_2$ | 1 | 2 | H | H | $CH_2$ | $CH_2$ | I |
| 648 | - | CO | $CH_2$ | 1 | 2 | Me | H | $CH_2$ | $CH_2$ | I |
| 649 | - | CO | $CH_2$ | 1 | 2 | Et | H | $CH_2$ | $CH_2$ | I |
| 650 | - | CO | $CH_2$ | 1 | 2 | $COCH_3$ | H | $CH_2$ | $CH_2$ | I |
| 651 | - | CO | $CH_2$ | 1 | 2 | $COC_6H_5$ | H | $CH_2$ | $CH_2$ | I |
| 652 | - | CO | $CH_2$ | 1 | 2 | Br | H | $CH_2$ | $CH_2$ | I |

| No. | A | B | D | m | n | R1 | R2 | G | Q | T |
|---|---|---|---|---|---|---|---|---|---|---|
| 653 | CO | - | $CH_2$ | 1 | 2 | H | H | CO | $(CH_2)_3$ | I |
| 654 | CO | - | $CH_2$ | 1 | 2 | Me | H | CO | $(CH_2)_3$ | I |
| 655 | CO | - | $CH_2$ | 1 | 2 | Et | H | CO | $(CH_2)_3$ | I |
| 656 | CO | - | $CH_2$ | 1 | 2 | $COCH_3$ | H | CO | $(CH_2)_3$ | I |
| 657 | CO | - | $CH_2$ | 1 | 2 | $COC_6H_5$ | H | CO | $(CH_2)_3$ | I |
| 658 | CO | - | $CH_2$ | 1 | 2 | Br | H | CO | $(CH_2)_3$ | I |
| 659 | CO | - | $CH_2$ | 1 | 2 | H | H | $CH_2$ | $CH_2$ | I |
| 660 | CO | - | $CH_2$ | 1 | 2 | Me | H | $CH_2$ | $CH_2$ | I |
| 661 | CO | - | $CH_2$ | 1 | 2 | Et | H | $CH_2$ | $CH_2$ | I |
| 662 | CO | - | $CH_2$ | 1 | 2 | $COCH_3$ | H | $CH_2$ | $CH_2$ | I |
| 663 | CO | - | $CH_2$ | 1 | 2 | $COC_6H_5$ | H | $CH_2$ | $CH_2$ | I |
| 664 | CO | - | $CH_2$ | 1 | 2 | Br | H | $CH_2$ | $CH_2$ | I |
| 665 | - | CO | $CH_2$ | 2 | 1 | H | H | CO | $(CH_2)_3$ | I |
| 666 | - | CO | $CH_2$ | 2 | 1 | Me | H | CO | $(CH_2)_3$ | I |
| 667 | - | CO | $CH_2$ | 2 | 1 | Et | H | CO | $(CH_2)_3$ | I |
| 668 | - | CO | $CH_2$ | 2 | 1 | $COCH_3$ | H | CO | $(CH_2)_3$ | I |
| 669 | - | CO | $CH_2$ | 2 | 1 | $COC_6H_5$ | H | CO | $(CH_2)_3$ | I |
| 670 | - | CO | $CH_2$ | 2 | 1 | Br | H | CO | $(CH_2)_3$ | I |
| 671 | - | CO | $CH_2$ | 2 | 1 | H | H | $CH_2$ | $CH_2$ | I |
| 672 | - | CO | $CH_2$ | 2 | 1 | Me | H | $CH_2$ | $CH_2$ | I |
| 673 | - | CO | $CH_2$ | 2 | 1 | Et | H | $CH_2$ | $CH_2$ | I |
| 674 | - | CO | $CH_2$ | 2 | 1 | $COCH_3$ | H | $CH_2$ | $CH_2$ | I |

| No. | A | B | D | m | n | R1 | R2 | G | Q | T |
|---|---|---|---|---|---|---|---|---|---|---|
| 675 | - | CO | $CH_2$ | 2 | 1 | $COC_6H_5$ | H | $CH_2$ | $CH_2$ | I |
| 676 | - | CO | $CH_2$ | 2 | 1 | Br | H | $CH_2$ | $CH_2$ | I |
| 677 | CO | - | $CH_2$ | 2 | 1 | H | H | CO | $(CH_2)_3$ | I |
| 678 | CO | - | $CH_2$ | 2 | 1 | Me | H | CO | $(CH_2)_3$ | I |
| 679 | CO | - | $CH_2$ | 2 | 1 | Et | H | CO | $(CH_2)_3$ | I |
| 680 | CO | - | $CH_2$ | 2 | 1 | $COCH_3$ | H | CO | $(CH_2)_3$ | I |
| 681 | CO | - | $CH_2$ | 2 | 1 | $COC_6H_5$ | H | CO | $(CH_2)_3$ | I |
| 682 | CO | - | $CH_2$ | 2 | 1 | Br | H | CO | $(CH_2)_3$ | I |
| 683 | CO | - | $CH_2$ | 2 | 1 | H | H | $CH_2$ | $CH_2$ | I |
| 684 | CO | - | $CH_2$ | 2 | 1 | Me | H | $CH_2$ | $CH_2$ | I |
| 685 | CO | - | $CH_2$ | 2 | 1 | Et | H | $CH_2$ | $CH_2$ | I |
| 686 | CO | - | $CH_2$ | 2 | 1 | $COCH_3$ | H | $CH_2$ | $CH_2$ | I |
| 687 | CO | - | $CH_2$ | 2 | 1 | $COC_5H_5$ | H | $CH_2$ | $CH_2$ | I |
| 688 | CO | - | $CH_2$ | 2 | 1 | Br | H | $CH_2$ | $CH_2$ | I |
| 689 | - | CO | $CH_2$ | 3 | 0 | H | H | CO | $(CH_2)_3$ | I |
| 690 | - | CO | $CH_2$ | 3 | 0 | Me | H | CO | $(CH_2)_3$ | I |
| 691 | - | CO | $CH_2$ | 3 | 0 | Et | H | CO | $(CH_2)_3$ | I |
| 692 | - | CO | $CH_2$ | 3 | 0 | $COCH_3$ | H | CO | $(CH_2)_3$ | I |
| 693 | - | CO | $CH_2$ | 3 | 0 | $COC_5H_5$ | H | CO | $(CH_2)_3$ | I |
| 694 | - | CO | $CH_2$ | 3 | 0 | Br | H | CO | $(CH_2)_3$ | I |
| 695 | - | CO | $CH_2$ | 3 | 0 | H | H | $CH_2$ | $CH_2$ | I |

| No. | A | B | D | m | n | R1 | R2 | G | Q | T |
|---|---|---|---|---|---|---|---|---|---|---|
| 696 | - | CO | $CH_2$ | 3 | 0 | Me | H | $CH_2$ | $CH_2$ | I |
| 697 | - | CO | $CH_2$ | 3 | 0 | Et | H | $CH_2$ | $CH_2$ | I |
| 698 | - | CO | $CH_2$ | 3 | 0 | $COCH_3$ | H | $CH_2$ | $CH_2$ | I |
| 699 | - | CO | $CH_2$ | 3 | 0 | $COC_6H_5$ | H | $CH_2$ | $CH_2$ | I |
| 700 | - | CO | $CH_2$ | 3 | 0 | Br | H | $CH_2$ | $CH_2$ | I |
| 701 | CO | - | $CH_2$ | 3 | 0 | H | H | CO | $(CH_2)_3$ | I |
| 702 | CO | - | $CH_2$ | 3 | 0 | Me | H | CO | $(CH_2)_3$ | I |
| 703 | CO | - | $CH_2$ | 3 | 0 | Et | H | CO | $(CH_2)_3$ | I |
| 704 | CO | - | $CH_2$ | 3 | 0 | $COCH_3$ | H | CO | $(CH_2)_3$ | I |
| 705 | CO | - | $CH_2$ | 3 | 0 | $COC_6H_5$ | H | CO | $(CH_2)_3$ | I |
| 706 | CO | - | $CH_2$ | 3 | 0 | Br | H | CO | $(CH_2)_3$ | I |
| 707 | CO | - | $CH_2$ | 3 | 0 | H | H | $CH_2$ | $CH_2$ | I |
| 708 | CO | - | $CH_2$ | 3 | 0 | Me | H | $CH_2$ | $CH_2$ | I |
| 709 | CO | - | $CH_2$ | 3 | 0 | Et | H | $CH_2$ | $CH_2$ | I |
| 710 | CO | - | $CH_2$ | 3 | 0 | $COCH_3$ | H | $CH_2$ | $CH_2$ | I |
| 711 | CO | - | $CH_2$ | 3 | 0 | $COC_6H_5$ | H | $CH_2$ | $CH_2$ | I |
| 712 | CO | - | $CH_2$ | 3 | 0 | Br | H | $CH_2$ | $CH_2$ | I |

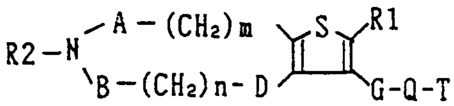

| No. | A | B | D | m | n | R1 | R2 | G | Q | T |
|---|---|---|---|---|---|---|---|---|---|---|
| 713 | - | CO | $CH_2$ | 0 | 1 | H | H | CO | $(CH_2)_3$ | I |
| 714 | - | CO | $CH_2$ | 0 | 1 | Me | H | CO | $(CH_2)_3$ | I |
| 715 | - | CO | $CH_2$ | 0 | 1 | Et | H | CO | $(CH_2)_3$ | I |
| 716 | - | CO | $CH_2$ | 0 | 1 | $COCH_3$ | H | CO | $(CH_2)_3$ | I |
| 717 | - | CO | $CH_2$ | 0 | 1 | $COC_6H_5$ | H | CO | $(CH_2)_3$ | I |
| 718 | - | CO | $CH_2$ | 0 | 1 | Br | H | CO | $(CH_2)_3$ | I |
| 719 | - | CO | $CH_2$ | 0 | 1 | H | H | $CH_2$ | $CH_2$ | I |
| 720 | - | CO | $CH_2$ | 0 | 1 | Me | H | $CH_2$ | $CH_2$ | I |
| 721 | - | CO | $CH_2$ | 0 | 1 | Et | H | $CH_2$ | $CH_2$ | I |
| 722 | - | CO | $CH_2$ | 0 | 1 | $COCH_3$ | H | $CH_2$ | $CH_2$ | I |
| 723 | - | CO | $CH_2$ | 0 | 1 | $COC_6H_5$ | H | $CH_2$ | $CH_2$ | I |
| 724 | - | CO | $CH_2$ | 0 | 1 | Br | H | $CH_2$ | $CH_2$ | I |
| 725 | CO | - | $CH_2$ | 0 | 1 | H | H | CO | $(CH_2)_3$ | I |
| 726 | CO | - | $CH_2$ | 0 | 1 | Me | H | CO | $(CH_2)_3$ | I |
| 727 | CO | - | $CH_2$ | 0 | 1 | Et | H | CO | $(CH_2)_3$ | I |
| 728 | CO | - | $CH_2$ | 0 | 1 | $COCH_3$ | H | CO | $(CH_2)_3$ | I |
| 729 | CO | - | $CH_2$ | 0 | 1 | $COC_6H_5$ | H | CO | $(CH_2)_3$ | I |
| 730 | CO | - | $CH_2$ | 0 | 1 | Br | H | CO | $(CH_2)_3$ | I |

| No. | A | B | D | m | n | R1 | R2 | G | Q | T |
|---|---|---|---|---|---|---|---|---|---|---|
| 731 | CO | - | $CH_2$ | 0 | 1 | H | H | $CH_2$ | $CH_2$ | I |
| 732 | CO | - | $CH_2$ | 0 | 1 | Me | H | $CH_2$ | $CH_2$ | I |
| 733 | CO | - | $CH_2$ | 0 | 1 | Et | H | $CH_2$ | $CH_2$ | I |
| 734 | CO | - | $CH_2$ | 0 | 1 | $COCH_3$ | H | $CH_2$ | $CH_2$ | I |
| 735 | CO | - | $CH_2$ | 0 | 1 | $COC_6H_5$ | H | $CH_2$ | $CH_2$ | I |
| 736 | CO | - | $CH_2$ | 0 | 1 | Br | H | $CH_2$ | $CH_2$ | I |
| 737 | - | CO | $CH_2$ | 0 | 2 | H | H | CO | $(CH_2)_3$ | I |
| 738 | - | CO | $CH_2$ | 0 | 2 | Me | H | CO | $(CH_2)_3$ | I |
| 739 | - | CO | $CH_2$ | 0 | 2 | Et | H | CO | $(CH_2)_3$ | I |
| 740 | - | CO | $CH_2$ | 0 | 2 | $COCH_3$ | H | CO | $(CH_2)_3$ | I |
| 741 | - | CO | $CH_2$ | 0 | 2 | $COC_6H_5$ | H | CO | $(CH_2)_3$ | I |
| 742 | - | CO | $CH_2$ | 0 | 2 | Br | H | CO | $(CH_2)_3$ | I |
| 743 | - | CO | $CH_2$ | 0 | 2 | H | H | $CH_2$ | $CH_2$ | I |
| 744 | - | CO | $CH_2$ | 0 | 2 | Me | H | $CH_2$ | $CH_2$ | I |
| 745 | - | CO | $CH_2$ | 0 | 2 | Et | H | $CH_2$ | $CH_2$ | I |
| 746 | - | CO | $CH_2$ | 0 | 2 | $COCH_3$ | H | $CH_2$ | $CH_2$ | I |
| 747 | - | CO | $CH_2$ | 0 | 2 | $COC_6H_5$ | H | $CH_2$ | $CH_2$ | I |
| 748 | - | CO | $CH_2$ | 0 | 2 | Br | H | $CH_2$ | $CH_2$ | I |
| 749 | CO | - | $CH_2$ | 0 | 2 | H | H | CO | $(CH_2)_3$ | I |
| 750 | CO | - | $CH_2$ | 0 | 2 | Me | H | CO | $(CH_2)_3$ | I |
| 751 | CO | - | $CH_2$ | 0 | 2 | Et | H | CO | $(CH_2)_3$ | I |

| No. | A | B | D | m | n | R1 | R2 | G | Q | T |
|---|---|---|---|---|---|---|---|---|---|---|
| 752 | CO | - | $CH_2$ | 0 | 2 | $COCH_3$ | H | CO | $(CH_2)_3$ | I |
| 753 | CO | - | $CH_2$ | 0 | 2 | $COC_6H_5$ | H | CO | $(CH_2)_3$ | I |
| 754 | CO | - | $CH_2$ | 0 | 2 | Br | H | CO | $(CH_2)_3$ | I |
| 755 | CO | - | $CH_2$ | 0 | 2 | H | H | $CH_2$ | $CH_2$ | I |
| 756 | CO | - | $CH_2$ | 0 | 2 | Me | H | $CH_2$ | $CH_2$ | I |
| 757 | CO | - | $CH_2$ | 0 | 2 | Et | H | $CH_2$ | $CH_2$ | I |
| 758 | CO | - | $CH_2$ | 0 | 2 | $COCH_3$ | H | $CH_2$ | $CH_2$ | I |
| 759 | CO | - | $CH_2$ | 0 | 2 | $COC_6H_5$ | H | $CH_2$ | $CH_2$ | I |
| 760 | CO | - | $CH_2$ | 0 | 2 | Br | H | $CH_2$ | $CH_2$ | I |
| 761 | - | CO | $CH_2$ | 0 | 3 | H | H | CO | $(CH_2)_3$ | I |
| 762 | - | CO | $CH_2$ | 0 | 3 | Me | H | CO | $(CH_2)_3$ | I |
| 763 | - | CO | $CH_2$ | 0 | 3 | Et | H | CO | $(CH_2)_3$ | I |
| 764 | - | CO | $CH_2$ | 0 | 3 | $COCH_3$ | H | CO | $(CH_2)_3$ | I |
| 765 | - | CO | $CH_2$ | 0 | 3 | $COC_6H_5$ | H | CO | $(CH_2)_3$ | I |
| 766 | - | CO | $CH_2$ | 0 | 3 | Br | H | CO | $(CH_2)_3$ | I |
| 767 | - | CO | $CH_2$ | 0 | 3 | H | H | $CH_2$ | $CH_2$ | I |
| 768 | - | CO | $CH_2$ | 0 | 3 | Me | H | $CH_2$ | $CH_2$ | I |
| 769 | - | CO | $CH_2$ | 0 | 3 | Et | H | $CH_2$ | $CH_2$ | I |
| 770 | - | CO | $CH_2$ | 0 | 3 | $COCH_3$ | H | $CH_2$ | $CH_2$ | I |
| 771 | - | CO | $CH_2$ | 0 | 3 | $COC_6H_5$ | H | $CH_2$ | $CH_2$ | I |
| 772 | - | CO | $CH_2$ | 0 | 3 | Br | H | $CH_2$ | $CH_2$ | I |

| No. | A | B | D | m | n | R1 | R2 | G | Q | T |
|---|---|---|---|---|---|---|---|---|---|---|
| 773 | CO | - | $CH_2$ | 0 | 3 | H | H | CO | $(CH_2)_3$ | I |
| 774 | CO | - | $CH_2$ | 0 | 3 | Me | H | CO | $(CH_2)_3$ | I |
| 775 | CO | - | $CH_2$ | 0 | 3 | Et | H | CO | $(CH_2)_3$ | I |
| 776 | CO | - | $CH_2$ | 0 | 3 | $COCH_3$ | H | CO | $(CH_2)_3$ | I |
| 777 | CO | - | $CH_2$ | 0 | 3 | $COC_6H_5$ | H | CO | $(CH_2)_3$ | I |
| 778 | CO | - | $CH_2$ | 0 | 3 | Br | H | CO | $(CH_2)_3$ | I |
| 779 | CO | - | $CH_2$ | 0 | 3 | H | H | $CH_2$ | $CH_2$ | I |
| 780 | CO | - | $CH_2$ | 0 | 3 | Me | H | $CH_2$ | $CH_2$ | I |
| 781 | CO | - | $CH_2$ | 0 | 3 | Et | H | $CH_2$ | $CH_2$ | I |
| 782 | CO | - | $CH_2$ | 0 | 3 | $COCH_3$ | H | $CH_2$ | $CH_2$ | I |
| 783 | CO | - | $CH_2$ | 0 | 3 | $COC_6H_5$ | H | $CH_2$ | $CH_2$ | I |
| 784 | CO | - | $CH_2$ | 0 | 3 | Br | H | $CH_2$ | $CH_2$ | I |

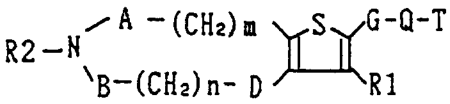

| No. | A | B | D | m | n | R1 | R2 | G | Q | T |
|---|---|---|---|---|---|---|---|---|---|---|
| 785 | - | CO | $CH_2$ | 0 | 1 | H | H | CO | $(CH_2)_3$ | I |
| 786 | - | CO | $CH_2$ | 0 | 1 | Me | H | CO | $(CH_2)_3$ | I |
| 787 | - | CO | $CH_2$ | 0 | 1 | Et | H | CO | $(CH_2)_3$ | I |
| 788 | - | CO | $CH_2$ | 0 | 1 | $COCH_3$ | H | CO | $(CH_2)_3$ | I |
| 789 | - | CO | $CH_2$ | 0 | 1 | $COC_6H_5$ | H | CO | $(CH_2)_3$ | I |
| 790 | - | CO | $CH_2$ | 0 | 1 | Br | H | CO | $(CH_2)_3$ | I |
| 791 | - | CO | $CH_2$ | 0 | 1 | H | H | $CH_2$ | $CH_2$ | I |
| 792 | - | CO | $CH_2$ | 0 | 1 | Me | H | $CH_2$ | $CH_2$ | I |
| 793 | - | CO | $CH_2$ | 0 | 1 | Et | H | $CH_2$ | $CH_2$ | I |
| 794 | - | CO | $CH_2$ | 0 | 1 | $COCH_3$ | H | $CH_2$ | $CH_2$ | I |
| 795 | - | CO | $CH_2$ | 0 | 1 | $COC_6H_5$ | H | $CH_2$ | $CH_2$ | I |
| 796 | - | CO | $CH_2$ | 0 | 1 | Br | H | $CH_2$ | $CH_2$ | I |
| 797 | CO | - | $CH_2$ | 0 | 1 | H | H | CO | $(CH_2)_3$ | I |
| 798 | CO | - | $CH_2$ | 0 | 1 | Me | H | CO | $(CH_2)_3$ | I |
| 799 | CO | - | $CH_2$ | 0 | 1 | Et | H | CO | $(CH_2)_3$ | I |
| 800 | CO | - | $CH_2$ | 0 | 1 | $COCH_3$ | H | CO | $(CH_2)_3$ | I |
| 801 | CO | - | $CH_2$ | 0 | 1 | $COC_6H_5$ | H | CO | $(CH_2)_3$ | I |
| 802 | CO | - | $CH_2$ | 0 | 1 | Br | H | CO | $(CH_2)_3$ | I |

| No. | A | B | D | m | n | R1 | R2 | G | Q | T |
|---|---|---|---|---|---|---|---|---|---|---|
| 803 | CO | - | $CH_2$ | 0 | 1 | H | H | $CH_2$ | $CH_2$ | I |
| 804 | CO | - | $CH_2$ | 0 | 1 | Me | H | $CH_2$ | $CH_2$ | I |
| 805 | CO | - | $CH_2$ | 0 | 1 | Et | H | $CH_2$ | $CH_2$ | I |
| 806 | CO | - | $CH_2$ | 0 | 1 | $COCH_3$ | H | $CH_2$ | $CH_2$ | I |
| 807 | CO | - | $CH_2$ | 0 | 1 | $COC_6H_5$ | H | $CH_2$ | $CH_2$ | I |
| 808 | CO | - | $CH_2$ | 0 | 1 | Br | H | $CH_2$ | $CH_2$ | I |
| 809 | - | CO | $CH_2$ | 0 | 2 | H | H | CO | $(CH_2)_3$ | I |
| 810 | - | CO | $CH_2$ | 0 | 2 | Me | H | CO | $(CH_2)_3$ | I |
| 811 | - | CO | $CH_2$ | 0 | 2 | Et | H | CO | $(CH_2)_3$ | I |
| 812 | - | CO | $CH_2$ | 0 | 2 | $COCH_3$ | H | CO | $(CH_2)_3$ | I |
| 813 | - | CO | $CH_2$ | 0 | 2 | $COC_6H_5$ | H | CO | $(CH_2)_3$ | I |
| 814 | - | CO | $CH_2$ | 0 | 2 | Br | H | CO | $(CH_2)_3$ | I |
| 815 | - | CO | $CH_2$ | 0 | 2 | H | H | $CH_2$ | $CH_2$ | I |
| 816 | - | CO | $CH_2$ | 0 | 2 | Me | H | $CH_2$ | $CH_2$ | I |
| 817 | - | CO | $CH_2$ | 0 | 2 | Et | H | $CH_2$ | $CH_2$ | I |
| 818 | - | CO | $CH_2$ | 0 | 2 | $COCH_3$ | H | $CH_2$ | $CH_2$ | I |
| 819 | - | CO | $CH_2$ | 0 | 2 | $COC_6H_5$ | H | $CH_2$ | $CH_2$ | I |
| 820 | - | CO | $CH_2$ | 0 | 2 | Br | H | $CH_2$ | $CH_2$ | I |
| 821 | CO | - | $CH_2$ | 0 | 2 | H | H | CO | $(CH_2)_3$ | I |
| 822 | CO | - | $CH_2$ | 0 | 2 | Me | H | CO | $(CH_2)_3$ | I |
| 823 | CO | - | $CH_2$ | 0 | 2 | Et | H | CO | $(CH_2)_3$ | I |
| 824 | CO | - | $CH_2$ | 0 | 2 | $COCH_3$ | H | CO | $(CH_2)_3$ | I |

| No. | A | B | D | m | n | R1 | R2 | G | Q | T |
|---|---|---|---|---|---|---|---|---|---|---|
| 825 | CO | - | $CH_2$ | 0 | 2 | $COC_6H_5$ | H | CO | $(CH_2)_3$ | I |
| 826 | CO | - | $CH_2$ | 0 | 2 | Br | H | CO | $(CH_2)_3$ | I |
| 827 | CO | - | $CH_2$ | 0 | 2 | H | H | $CH_2$ | $CH_2$ | I |
| 828 | CO | - | $CH_2$ | 0 | 2 | Me | H | $CH_2$ | $CH_2$ | I |
| 829 | CO | - | $CH_2$ | 0 | 2 | Et | H | $CH_2$ | $CH_2$ | I |
| 830 | CO | - | $CH_2$ | 0 | 2 | $COCH_3$ | H | $CH_2$ | $CH_2$ | I |
| 831 | CO | - | $CH_2$ | 0 | 2 | $COC_6H_5$ | H | $CH_2$ | $CH_2$ | I |
| 832 | CO | - | $CH_2$ | 0 | 2 | Br | H | $CH_2$ | $CH_2$ | I |
| 833 | - | CO | $CH_2$ | 0 | 3 | H | H | CO | $(CH_2)_3$ | I |
| 834 | - | CO | $CH_2$ | 0 | 3 | Me | H | CO | $(CH_2)_3$ | I |
| 835 | - | CO | $CH_2$ | 0 | 3 | Et | H | CO | $(CH_2)_3$ | I |
| 836 | - | CO | $CH_2$ | 0 | 3 | $COCH_3$ | H | CO | $(CH_2)_3$ | I |
| 837 | - | CO | $CH_2$ | 0 | 3 | $COC_6H_5$ | H | CO | $(CH_2)_3$ | I |
| 838 | - | CO | $CH_2$ | 0 | 3 | Br | H | CO | $(CH_2)_3$ | I |
| 839 | - | CO | $CH_2$ | 0 | 3 | H | H | $CH_2$ | $CH_2$ | I |
| 840 | - | CO | $CH_2$ | 0 | 3 | Me | H | $CH_2$ | $CH_2$ | I |
| 841 | - | CO | $CH_2$ | 0 | 3 | Et | H | $CH_2$ | $CH_2$ | I |
| 842 | - | CO | $CH_2$ | 0 | 3 | $COCH_3$ | H | $CH_2$ | $CH_2$ | I |
| 843 | - | CO | $CH_2$ | 0 | 3 | $COC_6H_5$ | H | $CH_2$ | $CH_2$ | I |
| 844 | - | CO | $CH_2$ | 0 | 3 | Br | H | $CH_2$ | $CH_2$ | I |
| 845 | CO | - | $CH_2$ | 0 | 3 | H | H | CO | $(CH_2)_3$ | I |
| 846 | CO | - | $CH_2$ | 0 | 3 | Me | H | CO | $(CH_2)_3$ | I |

| No. | A | B | D | m | n | R1 | R2 | G | Q | T |
|---|---|---|---|---|---|---|---|---|---|---|
| 847 | CO | - | $CH_2$ | 0 | 3 | Et | H | CO | $(CH_2)_3$ | I |
| 848 | CO | - | $CH_2$ | 0 | 3 | $COCH_3$ | H | CO | $(CH_2)_3$ | I |
| 849 | CO | - | $CH_2$ | 0 | 3 | $COC_6H_5$ | H | CO | $(CH_2)_3$ | I |
| 850 | CO | - | $CH_2$ | 0 | 3 | Br | H | CO | $(CH_2)_3$ | I |
| 851 | CO | - | $CH_2$ | 0 | 3 | H | H | $CH_2$ | $CH_2$ | I |
| 852 | CO | - | $CH_2$ | 0 | 3 | Me | H | $CH_2$ | $CH_2$ | I |
| 853 | CO | - | $CH_2$ | 0 | 3 | Et | H | $CH_2$ | $CH_2$ | I |
| 854 | CO | - | $CH_2$ | 0 | 3 | $COCH_3$ | H | $CH_2$ | $CH_2$ | I |
| 855 | CO | - | $CH_2$ | 0 | 3 | $COC_6H_5$ | H | $CH_2$ | $CH_2$ | I |
| 856 | CO | - | $CH_2$ | 0 | 3 | Br | H | $CH_2$ | $CH_2$ | I |

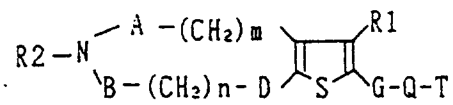

| No. | A | B | D | m | n | R1 | R2 | G | Q | T |
|---|---|---|---|---|---|---|---|---|---|---|
| 857 | - | - | $CH_2$ | 0 | 2 | H | $CH_3CO$ | CO | $(CH_2)_3$ | I |
| 858 | - | - | $CH_2$ | 0 | 2 | Me | $CH_3CO$ | CO | $(CH_2)_3$ | I |
| 859 | - | - | $CH_2$ | 0 | 2 | Et | $CH_3CO$ | CO | $(CH_2)_3$ | I |
| 860 | - | - | $CH_2$ | 0 | 2 | $COCH_3$ | $CH_3CO$ | CO | $(CH_2)_3$ | I |
| 861 | - | - | $CH_2$ | 0 | 2 | $COC_6H_5$ | $CH_3CO$ | CO | $(CH_2)_3$ | I |

| No. | A | B | D | m | n | R1 | R2 | G | Q | T |
|---|---|---|---|---|---|---|---|---|---|---|
| 862 | - | - | $CH_2$ | 0 | 2 | H | H | CO | $(CH_2)_3$ | I |
| 863 | - | - | $CH_2$ | 0 | 2 | H | Me | CO | $(CH_2)_3$ | I |
| 864 | - | - | $CH_2$ | 0 | 2 | H | $C_6H_5CO$ | CO | $(CH_2)_3$ | I |
| 865 | - | - | $CH_2$ | 0 | 2 | H | $CH_3CO$ | $CH_2$ | $CH_2$ | I |
| 866 | - | - | $CH_2$ | 0 | 2 | Me | $CH_3CO$ | $CH_2$ | $CH_2$ | I |
| 867 | - | - | $CH_2$ | 0 | 2 | Et | $CH_3CO$ | $CH_2$ | $CH_2$ | I |
| 868 | - | - | $CH_2$ | 0 | 2 | $COCH_3$ | $CH_3CO$ | $CH_2$ | $CH_2$ | I |
| 869 | - | - | $CH_2$ | 0 | 2 | $COC_6H_5$ | $CH_3CO$ | $CH_2$ | $CH_2$ | I |
| 870 | - | - | $CH_2$ | 0 | 2 | H | H | $CH_2$ | $CH_2$ | I |
| 871 | - | - | $CH_2$ | 0 | 2 | H | Me | $CH_2$ | $CH_2$ | I |
| 872 | - | - | $CH_2$ | 0 | 2 | H | $C_6H_5CO$ | $CH_2$ | $CH_2$ | I |
| 873 | - | - | $CH_2$ | 1 | 1 | H | $CH_3CO$ | CO | $(CH_2)_3$ | I |
| 874 | - | - | $CH_2$ | 1 | 1 | Me | $CH_3CO$ | CO | $(CH_2)_3$ | I |
| 875 | - | - | $CH_2$ | 1 | 1 | Et | $CH_3CO$ | CO | $(CH_2)_3$ | I |
| 876 | - | - | $CH_2$ | 1 | 1 | $COCH_3$ | $CH_3CO$ | CO | $(CH_2)_3$ | I |
| 877 | - | - | $CH_2$ | 1 | 1 | $COC_6H_5$ | $CH_3CO$ | CO | $(CH_2)_3$ | I |
| 878 | - | - | $CH_2$ | 1 | 1 | H | H | CO | $(CH_2)_3$ | I |
| 879 | - | - | $CH_2$ | 1 | 1 | H | Me | CO | $(CH_2)_3$ | I |
| 880 | - | - | $CH_2$ | 1 | 1 | H | $C_6H_5CO$ | CO | $(CH_2)_3$ | I |
| 881 | - | - | $CH_2$ | 1 | 1 | H | $CH_3CO$ | $CH_2$ | $CH_2$ | I |
| 882 | - | - | $CH_2$ | 1 | 1 | Me | $CH_3CO$ | $CH_2$ | $CH_2$ | I |
| 883 | - | - | $CH_2$ | 1 | 1 | Et | $CH_3CO$ | $CH_2$ | $CH_2$ | I |

| No. | A | B | D | m | n | R1 | R2 | G | Q | T |
|---|---|---|---|---|---|---|---|---|---|---|
| 884 | - | - | $CH_2$ | 1 | 1 | $COCH_3$ | $CH_3CO$ | $CH_2$ | $CH_2$ | I |
| 885 | - | - | $CH_2$ | 1 | 1 | $COC_6H_5$ | $CH_3CO$ | $CH_2$ | $CH_2$ | I |
| 886 | - | - | $CH_2$ | 1 | 1 | H | H | $CH_2$ | $CH_2$ | I |
| 887 | - | - | $CH_2$ | 1 | 1 | H | Me | $CH_2$ | $CH_2$ | I |
| 888 | - | - | $CH_2$ | 1 | 1 | H | $C_6H_5CO$ | $CH_2$ | $CH_2$ | I |
| 889 | - | - | $CH_2$ | 2 | 0 | H | $CH_3CO$ | CO | $(CH_2)_3$ | I |
| 890 | - | - | $CH_2$ | 2 | 0 | Me | $CH_3CO$ | CO | $(CH_2)_3$ | I |
| 891 | - | - | $CH_2$ | 2 | 0 | Et | $CH_3CO$ | CO | $(CH_2)_3$ | I |
| 892 | - | - | $CH_2$ | 2 | 0 | $COCH_3$ | $CH_3CO$ | CO | $(CH_2)_3$ | I |
| 893 | - | - | $CH_2$ | 2 | 0 | $COC_6H_5$ | $CH_3CO$ | CO | $(CH_2)_3$ | I |
| 894 | - | - | $CH_2$ | 2 | 0 | H | H | CO | $(CH_2)_3$ | I |
| 895 | - | - | $CH_2$ | 2 | 0 | H | Me | CO | $(CH_2)_3$ | I |
| 896 | - | - | $CH_2$ | 2 | 0 | H | $C_6H_5CO$ | CO | $(CH_2)_3$ | I |
| 897 | - | - | $CH_2$ | 2 | 0 | H | $CH_3CO$ | $CH_2$ | $CH_2$ | I |
| 898 | - | - | $CH_2$ | 2 | 0 | Me | $CH_3CO$ | $CH_2$ | $CH_2$ | I |
| 899 | - | - | $CH_2$ | 2 | 0 | Et | $CH_3CO$ | $CH_2$ | $CH_2$ | I |
| 900 | - | - | $CH_2$ | 2 | 0 | $COCH_3$ | $CH_3CO$ | $CH_2$ | $CH_2$ | I |
| 901 | - | - | $CH_2$ | 2 | 0 | $COC_6H_5$ | $CH_3CO$ | $CH_2$ | $CH_2$ | I |
| 902 | - | - | $CH_2$ | 2 | 0 | H | H | $CH_2$ | $CH_2$ | I |
| 903 | - | - | $CH_2$ | 2 | 0 | H | Me | $CH_2$ | $CH_2$ | I |
| 904 | - | - | $CH_2$ | 2 | 0 | H | $C_6H_5CO$ | $CH_2$ | $CH_2$ | I |

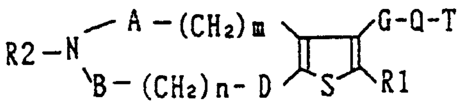

| No. | A | B | D | m | n | R1 | R2 | G | Q | T |
|---|---|---|---|---|---|---|---|---|---|---|
| 905 | - | - | $CH_2$ | 0 | 2 | H | $CH_3CO$ | CO | $(CH_2)_3$ | I |
| 906 | - | - | $CH_2$ | 0 | 2 | Me | $CH_3CO$ | CO | $(CH_2)_3$ | I |
| 907 | - | - | $CH_2$ | 0 | 2 | Et | $CH_3CO$ | CO | $(CH_2)_3$ | I |
| 908 | - | - | $CH_2$ | 0 | 2 | $COCH_3$ | $CH_3CO$ | CO | $(CH_2)_3$ | I |
| 909 | - | - | $CH_2$ | 0 | 2 | $COC_6H_5$ | $CH_3CO$ | CO | $(CH_2)_3$ | I |
| 910 | - | - | $CH_2$ | 0 | 2 | H | H | CO | $(CH_2)_3$ | I |
| 911 | - | - | $CH_2$ | 0 | 2 | H | Me | CO | $(CH_2)_3$ | I |
| 912 | - | - | $CH_2$ | 0 | 2 | H | $C_6H_5CO$ | CO | $(CH_2)_3$ | I |
| 913 | - | - | $CH_2$ | 0 | 2 | H | $CH_3CO$ | $CH_2$ | $CH_2$ | I |
| 914 | - | - | $CH_2$ | 0 | 2 | Me | $CH_3CO$ | $CH_2$ | $CH_2$ | I |
| 915 | - | - | $CH_2$ | 0 | 2 | Et | $CH_3CO$ | $CH_2$ | $CH_2$ | I |
| 916 | - | - | $CH_2$ | 0 | 2 | $COCH_3$ | $CH_3CO$ | $CH_2$ | $CH_2$ | I |
| 917 | - | - | $CH_2$ | 0 | 2 | $COC_6H_5$ | $CH_3CO$ | $CH_2$ | $CH_2$ | I |
| 918 | - | - | $CH_2$ | 0 | 2 | H | H | $CH_2$ | $CH_2$ | I |
| 919 | - | - | $CH_2$ | 0 | 2 | H | Me | $CH_2$ | $CH_2$ | I |
| 920 | - | - | $CH_2$ | 0 | 2 | H | $C_5H_5CO$ | $CH_2$ | $CH_2$ | I |
| 921 | - | - | $CH_2$ | 1 | 1 | H | $CH_3CO$ | CO | $(CH_2)_3$ | I |
| 922 | - | - | $CH_2$ | 1 | 1 | Me | $CH_3CO$ | CO | $(CH_2)_3$ | I |

| No. | A | B | D | m | n | R1 | R2 | G | Q | T |
|---|---|---|---|---|---|---|---|---|---|---|
| 923 | - | - | $CH_2$ | 1 | 1 | Et | $CH_3CO$ | CO | $(CH_2)_3$ | I |
| 924 | - | - | $CH_2$ | 1 | 1 | $COCH_3$ | $CH_3CO$ | CO | $(CH_2)_3$ | I |
| 925 | - | - | $CH_2$ | 1 | 1 | $COC_6H_5$ | $CH_3CO$ | CO | $(CH_2)_3$ | I |
| 926 | - | - | $CH_2$ | 1 | 1 | H | H | CO | $(CH_2)_3$ | I |
| 927 | - | - | $CH_2$ | 1 | 1 | H | Me | CO | $(CH_2)_3$ | I |
| 928 | - | - | $CH_2$ | 1 | 1 | H | $C_5H_5CO$ | CO | $(CH_2)_3$ | I |
| 929 | - | - | $CH_2$ | 1 | 1 | H | $CH_3CO$ | $CH_2$ | $CH_2$ | I |
| 930 | - | - | $CH_2$ | 1 | 1 | Me | $CH_3CO$ | $CH_2$ | $CH_2$ | I |
| 931 | - | - | $CH_2$ | 1 | 1 | Et | $CH_3CO$ | $CH_2$ | $CH_2$ | I |
| 932 | - | - | $CH_2$ | 1 | 1 | $COCH_3$ | $CH_3CO$ | $CH_2$ | $CH_2$ | I |
| 933 | - | - | $CH_2$ | 1 | 1 | $COC_6H_5$ | $CH_3CO$ | $CH_2$ | $CH_2$ | I |
| 934 | - | - | $CH_2$ | 1 | 1 | H | H | $CH_2$ | $CH_2$ | I |
| 935 | - | - | $CH_2$ | 1 | 1 | H | Me | $CH_2$ | $CH_2$ | I |
| 936 | - | - | $CH_2$ | 1 | 1 | H | $C_5H_5CO$ | $CH_2$ | $CH_2$ | I |
| 937 | - | - | $CH_2$ | 2 | 0 | H | $CH_3CO$ | CO | $(CH_2)_3$ | I |
| 938 | - | - | $CH_2$ | 2 | 0 | Me | $CH_3CO$ | CO | $(CH_2)_3$ | I |
| 939 | - | - | $CH_2$ | 2 | 0 | Et | $CH_3CO$ | CO | $(CH_2)_3$ | I |
| 940 | - | - | $CH_2$ | 2 | 0 | $COCH_3$ | $CH_3CO$ | CO | $(CH_2)_3$ | I |
| 941 | - | - | $CH_2$ | 2 | 0 | $COC_6H_5$ | $CH_3CO$ | CO | $(CH_2)_3$ | I |
| 942 | - | - | $CH_2$ | 2 | 0 | H | H | CO | $(CH_2)_3$ | I |
| 943 | - | - | $CH_2$ | 2 | 0 | H | Me | CO | $(CH_2)_3$ | I |
| 944 | - | - | $CH_2$ | 2 | 0 | H | $C_5H_5CO$ | CO | $(CH_2)_3$ | I |

| No. | A | B | D | m | n | R1 | R2 | G | Q | T |
|---|---|---|---|---|---|---|---|---|---|---|
| 945 | - | - | $CH_2$ | 2 | 0 | H | $CH_3CO$ | $CH_2$ | $CH_2$ | I |
| 946 | - | - | $CH_2$ | 2 | 0 | Me | $CH_3CO$ | $CH_2$ | $CH_2$ | I |
| 947 | - | - | $CH_2$ | 2 | 0 | Et | $CH_3CO$ | $CH_2$ | $CH_2$ | I |
| 948 | - | - | $CH_2$ | 2 | 0 | $COCH_3$ | $CH_3CO$ | $CH_2$ | $CH_2$ | I |
| 949 | - | - | $CH_2$ | 2 | 0 | $COC_6H_5$ | $CH_3CO$ | $CH_2$ | $CH_2$ | I |
| 950 | - | - | $CH_2$ | 2 | 0 | H | H | $CH_2$ | $CH_2$ | I |
| 951 | - | - | $CH_2$ | 2 | 0 | H | Me | $CH_2$ | $CH_2$ | I |
| 952 | - | - | $CH_2$ | 2 | 0 | H | $C_5H_5CO$ | $CH_2$ | $CH_2$ | I |

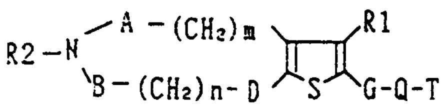

| No. | A | B | D | m | n | R1 | R2 | G | Q | T |
|---|---|---|---|---|---|---|---|---|---|---|
| 953 | - | - | $CH_2$ | 0 | 3 | H | $CH_3CO$ | CO | $(CH_2)_3$ | I |
| 954 | - | - | $CH_2$ | 0 | 3 | Me | $CH_3CO$ | CO | $(CH_2)_3$ | I |
| 955 | - | - | $CH_2$ | 0 | 3 | Et | $CH_3CO$ | CO | $(CH_2)_3$ | I |
| 956 | - | - | $CH_2$ | 0 | 3 | $COCH_3$ | $CH_3CO$ | CO | $(CH_2)_3$ | I |
| 957 | - | - | $CH_2$ | 0 | 3 | $COC_6H_5$ | $CH_3CO$ | CO | $(CH_2)_3$ | I |
| 958 | - | - | $CH_2$ | 0 | 3 | H | H | CO | $(CH_2)_3$ | I |
| 959 | - | - | $CH_2$ | 0 | 3 | H | Me | CO | $(CH_2)_3$ | I |

| No. | A | B | D | m | n | R1 | R2 | G | Q | T |
|---|---|---|---|---|---|---|---|---|---|---|
| 960 | - | - | $CH_2$ | 0 | 3 | H | $C_6H_5CO$ | CO | $(CH_2)_3$ | I |
| 961 | - | - | $CH_2$ | 0 | 3 | H | $CH_3CO$ | $CH_2$ | $CH_2$ | I |
| 962 | - | - | $CH_2$ | 0 | 3 | Me | $CH_3CO$ | $CH_2$ | $CH_2$ | I |
| 963 | - | - | $CH_2$ | 0 | 3 | Et | $CH_3CO$ | $CH_2$ | $CH_2$ | I |
| 964 | - | - | $CH_2$ | 0 | 3 | $COCH_3$ | $CH_3CO$ | $CH_2$ | $CH_2$ | I |
| 965 | - | - | $CH_2$ | 0 | 3 | $COC_6H_5$ | $CH_3CO$ | $CH_2$ | $CH_2$ | I |
| 966 | - | - | $CH_2$ | 0 | 3 | H | H | $CH_2$ | $CH_2$ | I |
| 967 | - | - | $CH_2$ | 0 | 3 | H | Me | $CH_2$ | $CH_2$ | I |
| 968 | - | - | $CH_2$ | 0 | 3 | H | $C_6H_5CO$ | $CH_2$ | $CH_2$ | I |
| 969 | - | - | $CH_2$ | 1 | 2 | H | $CH_3CO$ | CO | $(CH_2)_3$ | I |
| 970 | - | - | $CH_2$ | 1 | 2 | Me | $CH_3CO$ | CO | $(CH_2)_3$ | I |
| 971 | - | - | $CH_2$ | 1 | 2 | Et | $CH_3CO$ | CO | $(CH_2)_3$ | I |
| 972 | - | - | $CH_2$ | 1 | 2 | $COCH_3$ | $CH_3CO$ | CO | $(CH_2)_3$ | I |
| 973 | - | - | $CH_2$ | 1 | 2 | COC6H5 | $CH_3CO$ | CO | $(CH_2)_3$ | I |
| 974 | - | - | $CH_2$ | 1 | 2 | H | H | CO | $(CH_2)_3$ | I |
| 975 | - | - | $CH_2$ | 1 | 2 | H | Me | CO | $(CH_2)_3$ | I |
| 976 | - | - | $CH_2$ | 1 | 2 | H | $C_5H_5CO$ | CO | $(CH_2)_3$ | I |
| 977 | - | - | $CH_2$ | 1 | 2 | H | $CH_3CO$ | $CH_2$ | $CH_2$ | I |
| 978 | - | - | $CH_2$ | 1 | 2 | Me | $CH_3CO$ | $CH_2$ | $CH_2$ | I |
| 979 | - | - | $CH_2$ | 1 | 2 | Et | $CH_3CO$ | $CH_2$ | $CH_2$ | I |
| 980 | - | - | $CH_2$ | 1 | 2 | $COCH_3$ | $CH_3CO$ | $CH_2$ | $CH_2$ | I |
| 981 | - | - | $CH_2$ | 1 | 2 | $COC_6H_5$ | $CH_3CO$ | $CH_2$ | $CH_2$ | I |

| No. | A | B | D | m | n | R1 | R2 | G | Q | T |
|---|---|---|---|---|---|---|---|---|---|---|
| 982 | - | - | $CH_2$ | 1 | 2 | H | H | $CH_2$ | $CH_2$ | I |
| 983 | - | - | $CH_2$ | 1 | 2 | H | Me | $CH_2$ | $CH_2$ | I |
| 984 | - | - | $CH_2$ | 1 | 2 | H | $C_6H_5CO$ | $CH_2$ | $CH_2$ | I |
| 985 | - | - | $CH_2$ | 2 | 1 | H | $CH_3CO$ | CO | $(CH_2)_3$ | I |
| 986 | - | - | $CH_2$ | 2 | 1 | Me | $CH_3CO$ | CO | $(CH_2)_3$ | I |
| 987 | - | - | $CH_2$ | 2 | 1 | Et | $CH_3CO$ | CO | $(CH_2)_3$ | I |
| 988 | - | - | $CH_2$ | 2 | 1 | $COCH_3$ | $CH_3CO$ | CO | $(CH_2)_3$ | I |
| 989 | - | - | $CH_2$ | 2 | 1 | $COC_6H_5$ | $CH_3CO$ | CO | $(CH_2)_3$ | I |
| 990 | - | - | $CH_2$ | 2 | 1 | H | H | CO | $(CH_2)_3$ | I |
| 991 | - | - | $CH_2$ | 2 | 1 | H | Me | CO | $(CH_2)_3$ | I |
| 992 | - | - | $CH_2$ | 2 | 1 | H | $C_6H_5CO$ | CO | $(CH_2)_3$ | I |
| 993 | - | - | $CH_2$ | 2 | 1 | H | $CH_3CO$ | $CH_2$ | $CH_2$ | I |
| 994 | - | - | $CH_2$ | 2 | 1 | Me | $CH_3CO$ | $CH_2$ | $CH_2$ | I |
| 995 | - | - | $CH_2$ | 2 | 1 | Et | $CH_3CO$ | $CH_2$ | $CH_2$ | I |
| 996 | - | - | $CH_2$ | 2 | 1 | $COCH_3$ | $CH_3CO$ | $CH_2$ | $CH_2$ | I |
| 997 | - | - | $CH_2$ | 2 | 1 | $COC_6H_5$ | $CH_3CO$ | $CH_2$ | $CH_2$ | I |
| 998 | - | - | $CH_2$ | 2 | 1 | H | H | $CH_2$ | $CH_2$ | I |
| 999 | - | - | $CH_2$ | 2 | 1 | H | Me | $CH_2$ | $CH_2$ | I |
| 1000 | - | - | $CH_2$ | 2 | 1 | H | $C_6H_5CO$ | $CH_2$ | $CH_2$ | I |
| 1001 | - | - | $CH_2$ | 3 | 0 | H | $CH_3CO$ | CO | $(CH_2)_3$ | I |
| 1002 | - | - | $CH_2$ | 3 | 0 | Me | $CH_3CO$ | CO | $(CH_2)_3$ | I |
| 1003 | - | - | $CH_2$ | 3 | 0 | Et | $CH_3CO$ | CO | $(CH_2)_3$ | I |

| No. | A | B | D | m | n | R1 | R2 | G | Q | T |
|---|---|---|---|---|---|---|---|---|---|---|
| 1004 | - | - | $CH_2$ | 3 | 0 | $COCH_3$ | $CH_3CO$ | CO | $(CH_2)_3$ | I |
| 1005 | - | - | $CH_2$ | 3 | 0 | $COC_6H_5$ | $CH_3CO$ | CO | $(CH_2)_3$ | I |
| 1006 | - | - | $CH_2$ | 3 | 0 | H | H | CO | $(CH_2)_3$ | I |
| 1007 | - | - | $CH_2$ | 3 | 0 | H | Me | CO | $(CH_2)_3$ | I |
| 1008 | - | - | $CH_2$ | 3 | 0 | H | $C_6H_5CO$ | CO | $(CH_2)_3$ | I |
| 1009 | - | - | $CH_2$ | 3 | 0 | H | $CH_3CO$ | $CH_2$ | $CH_2$ | I |
| 1010 | - | - | $CH_2$ | 3 | 0 | Me | $CH_3CO$ | $CH_2$ | $CH_2$ | I |
| 1011 | - | - | $CH_2$ | 3 | 0 | Et | $CH_3CO$ | $CH_2$ | $CH_2$ | I |
| 1012 | - | - | $CH_2$ | 3 | 0 | $COCH_3$ | $CH_3CO$ | $CH_2$ | $CH_2$ | I |
| 1013 | - | - | $CH_2$ | 3 | 0 | $COC_6H_5$ | $CH_3CO$ | $CH_2$ | $CH_2$ | I |
| 1014 | - | - | $CH_2$ | 3 | 0 | H | H | $CH_2$ | $CH_2$ | I |
| 1015 | - | - | $CH_2$ | 3 | 0 | H | Me | $CH_2$ | $CH_2$ | I |
| 1016 | - | - | $CH_2$ | 3 | 0 | H | $C_6H_5CO$ | $CH_2$ | $CH_2$ | I |

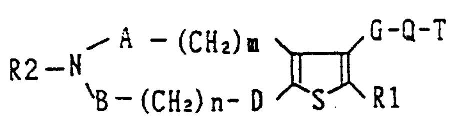

| No. | A | B | D | m | n | R1 | R2 | G | Q | T |
|---|---|---|---|---|---|---|---|---|---|---|
| 1017 | - | - | $CH_2$ | 0 | 3 | H | $CH_3CO$ | CO | $(CH_2)_3$ | I |
| 1018 | - | - | $CH_2$ | 0 | 3 | Me | $CH_3CO$ | CO | $(CH_2)_3$ | I |

| No. | A | B | D | m | n | R1 | R2 | G | Q | T |
|---|---|---|---|---|---|---|---|---|---|---|
| 1019 | - | - | $CH_2$ | 0 | 3 | Et | $CH_3CO$ | CO | $(CH_2)_3$ | I |
| 1020 | - | - | $CH_2$ | 0 | 3 | $COCH_3$ | $CH_3CO$ | CO | $(CH_2)_3$ | I |
| 1021 | - | - | $CH_2$ | 0 | 3 | $COC_6H_5$ | $CH_3CO$ | CO | $(CH_2)_3$ | I |
| 1022 | - | - | $CH_2$ | 0 | 3 | H | H | CO | $(CH_2)_3$ | I |
| 1023 | - | - | $CH_2$ | 0 | 3 | H | Me | CO | $(CH_2)_3$ | I |
| 1024 | - | - | $CH_2$ | 0 | 3 | H | $C_6H_5CO$ | CO | $(CH_2)_3$ | I |
| 1025 | - | - | $CH_2$ | 0 | 3 | H | $CH_3CO$ | $CH_2$ | $CH_2$ | I |
| 1026 | - | - | $CH_2$ | 0 | 3 | Me | $CH_3CO$ | $CH_2$ | $CH_2$ | I |
| 1027 | - | - | $CH_2$ | 0 | 3 | Et | $CH_3CO$ | $CH_2$ | $CH_2$ | I |
| 1028 | - | - | $CH_2$ | 0 | 3 | $COCH_3$ | $CH_3CO$ | $CH_2$ | $CH_2$ | I |
| 1029 | - | - | $CH_2$ | 0 | 3 | $COC_6H_5$ | $CH_3CO$ | $CH_2$ | $CH_2$ | I |
| 1030 | - | - | $CH_2$ | 0 | 3 | H | H | $CH_2$ | $CH_2$ | I |
| 1031 | - | - | $CH_2$ | 0 | 3 | H | Me | $CH_2$ | $CH_2$ | I |
| 1032 | - | - | $CH_2$ | 0 | 3 | H | $C_5H_5CO$ | $CH_2$ | $CH_2$ | I |
| 1033 | - | - | $CH_2$ | 1 | 2 | H | $CH_3CO$ | CO | $(CH_2)_3$ | I |
| 1034 | - | - | $CH_2$ | 1 | 2 | Me | $CH_3CO$ | CO | $(CH_2)_3$ | I |
| 1035 | - | - | $CH_2$ | 1 | 2 | Et | $CH_3CO$ | CO | $(CH_2)_3$ | I |
| 1036 | - | - | $CH_2$ | 1 | 2 | $COCH_3$ | $CH_3CO$ | CO | $(CH_2)_3$ | I |
| 1037 | - | - | $CH_2$ | 1 | 2 | $COC_6H_5$ | $CH_3CO$ | CO | $(CH_2)_3$ | I |
| 1038 | - | - | $CH_2$ | 1 | 2 | H | H | CO | $(CH_2)_3$ | I |
| 1039 | - | - | $CH_2$ | 1 | 2 | H | Me | CO | $(CH_2)_3$ | I |
| 1040 | - | - | $CH_2$ | 1 | 2 | H | $C_5H_5CO$ | CO | $(CH_2)_3$ | I |

| No. | A | B | D | m | n | R1 | R2 | G | Q | T |
|---|---|---|---|---|---|---|---|---|---|---|
| 1041 | - | - | $CH_2$ | 1 | 2 | H | $CH_3CO$ | $CH_2$ | $CH_2$ | I |
| 1042 | - | - | $CH_2$ | 1 | 2 | Me | $CH_3CO$ | $CH_2$ | $CH_2$ | I |
| 1043 | - | - | $CH_2$ | 1 | 2 | Et | $CH_3CO$ | $CH_2$ | $CH_2$ | I |
| 1044 | - | - | $CH_2$ | 1 | 2 | $COCH_3$ | $CH_3CO$ | $CH_2$ | $CH_2$ | I |
| 1045 | - | - | $CH_2$ | 1 | 2 | $COC_6H_5$ | $CH_3CO$ | $CH_2$ | $CH_2$ | I |
| 1046 | - | - | $CH_2$ | 1 | 2 | H | H | $CH_2$ | $CH_2$ | I |
| 1047 | - | - | $CH_2$ | 1 | 2 | H | Me | $CH_2$ | $CH_2$ | I |
| 1048 | - | - | $CH_2$ | 1 | 2 | H | $C_5H_5CO$ | $CH_2$ | $CH_2$ | I |
| 1049 | - | - | $CH_2$ | 2 | 1 | H | $CH_3CO$ | CO | $(CH_2)_3$ | I |
| 1050 | - | - | $CH_2$ | 2 | 1 | Me | $CH_3CO$ | CO | $(CH_2)_3$ | I |
| 1051 | - | - | $CH_2$ | 2 | 1 | Et | $CH_3CO$ | CO | $(CH_2)_3$ | I |
| 1052 | - | - | $CH_2$ | 2 | 1 | $COCH_3$ | $CH_3CO$ | CO | $(CH_2)_3$ | I |
| 1053 | - | - | $CH_2$ | 2 | 1 | $COC_6H_5$ | $CH_3CO$ | CO | $(CH_2)_3$ | I |
| 1054 | - | - | $CH_2$ | 2 | 1 | H | H | CO | $(CH_2)_3$ | I |
| 1055 | - | - | $CH_2$ | 2 | 1 | H | Me | CO | $(CH_2)_3$ | I |
| 1056 | - | - | $CH_2$ | 2 | 1 | H | $C_5H_5CO$ | CO | $(CH_2)_3$ | I |
| 1057 | - | - | $CH_2$ | 2 | 1 | H | $CH_3CO$ | $CH_2$ | $CH_2$ | I |
| 1058 | - | - | $CH_2$ | 2 | 1 | Me | $CH_3CO$ | $CH_2$ | $CH_2$ | I |
| 1059 | - | - | $CH_2$ | 2 | 1 | Et | $CH_3CO$ | $CH_2$ | $CH_2$ | I |
| 1060 | - | - | $CH_2$ | 2 | 1 | $COCH_3$ | $CH_3CO$ | $CH_2$ | $CH_2$ | I |
| 1061 | - | - | $CH_2$ | 2 | 1 | $COC_6H_5$ | $CH_3CO$ | $CH_2$ | $CH_2$ | I |
| 1062 | - | - | $CH_2$ | 2 | 1 | H | H | $CH_2$ | $CH_2$ | I |

| No. | A | B | D | m | n | R1 | R2 | G | Q | T |
|---|---|---|---|---|---|---|---|---|---|---|
| 1063 | - | - | $CH_2$ | 2 | 1 | H | Me | $CH_2$ | $CH_2$ | I |
| 1064 | - | - | $CH_2$ | 2 | 1 | H | $C_6H_5CO$ | $CH_2$ | $CH_2$ | I |
| 1065 | - | - | $CH_2$ | 3 | 0 | H | $CH_3CO$ | CO | $(CH_2)_3$ | I |
| 1066 | - | - | $CH_2$ | 3 | 0 | Me | $CH_3CO$ | CO | $(CH_2)_3$ | I |
| 1067 | - | - | $CH_2$ | 3 | 0 | Et | $CH_3CO$ | CO | $(CH_2)_3$ | I |
| 1068 | - | - | $CH_2$ | 3 | 0 | $COCH_3$ | $CH_3CO$ | CO | $(CH_2)_3$ | I |
| 1069 | - | - | $CH_2$ | 3 | 0 | $COC_6H_5$ | $CH_3CO$ | CO | $(CH_2)_3$ | I |
| 1070 | - | - | $CH_2$ | 3 | 0 | H | H | CO | $(CH_2)_3$ | I |
| 1071 | - | - | $CH_2$ | 3 | 0 | H | Me | CO | $(CH_2)_3$ | I |
| 1072 | - | - | $CH_2$ | 3 | 0 | H | $C_6H_5CO$ | CO | $(CH_2)_3$ | I |
| 1073 | - | - | $CH_2$ | 3 | 0 | H | $CH_3CO$ | $CH_2$ | $CH_2$ | I |
| 1074 | - | - | $CH_2$ | 3 | 0 | Me | $CH_3CO$ | $CH_2$ | $CH_2$ | I |
| 1075 | - | - | $CH_2$ | 3 | 0 | Et | $CH_3CO$ | $CH_2$ | $CH_2$ | I |
| 1076 | - | - | $CH_2$ | 3 | 0 | $COCH_3$ | $CH_3CO$ | $CH_2$ | $CH_2$ | I |
| 1077 | - | - | $CH_2$ | 3 | 0 | $COC_6H_5$ | $CH_3CO$ | $CH_2$ | $CH_2$ | I |
| 1078 | - | - | $CH_2$ | 3 | 0 | H | H | $CH_2$ | $CH_2$ | I |
| 1079 | - | - | $CH_2$ | 3 | 0 | H | Me | $CH_2$ | $CH_2$ | I |
| 1080 | - | - | $CH_2$ | 3 | 0 | H | $C_6H_5CO$ | $CH_2$ | $CH_2$ | I |

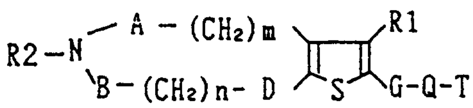

| No. | A | B | D | m | n | R1 | R2 | G | Q | T |
|---|---|---|---|---|---|---|---|---|---|---|
| 1081 | - | - | $CH_2$ | 0 | 4 | H | $CH_3CO$ | CO | $(CH_2)_3$ | I |
| 1082 | - | - | $CH_2$ | 0 | 4 | Me | $CH_3CO$ | CO | $(CH_2)_3$ | I |
| 1083 | - | - | $CH_2$ | 0 | 4 | Et | $CH_3CO$ | CO | $(CH_2)_3$ | I |
| 1084 | - | - | $CH_2$ | 0 | 4 | $COCH_3$ | $CH_3CO$ | CO | $(CH_2)_3$ | I |
| 1085 | - | - | $CH_2$ | 0 | 4 | $COC_6H_5$ | $CH_3CO$ | CO | $(CH_2)_3$ | I |
| 1086 | - | - | $CH_2$ | 0 | 4 | H | H | CO | $(CH_2)_3$ | I |
| 1087 | - | - | $CH_2$ | 0 | 4 | H | Me | CO | $(CH_2)_3$ | I |
| 1088 | - | - | $CH_2$ | 0 | 4 | H | $C_5H_5CO$ | CO | $(CH_2)_3$ | I |
| 1089 | - | - | $CH_2$ | 0 | 4 | H | $CH_3CO$ | $CH_2$ | $CH_2$ | I |
| 1090 | - | - | $CH_2$ | 0 | 4 | Me | $CH_3CO$ | $CH_2$ | $CH_2$ | I |
| 1091 | - | - | $CH_2$ | 0 | 4 | Et | $CH_3CO$ | $CH_2$ | $CH_2$ | I |
| 1092 | - | - | $CH_2$ | 0 | 4 | $COCH_3$ | $CH_3CO$ | $CH_2$ | $CH_2$ | I |
| 1093 | - | - | $CH_2$ | 0 | 4 | $COC_6H_5$ | $CH_3CO$ | $CH_2$ | $CH_2$ | I |
| 1094 | - | - | $CH_2$ | 0 | 4 | H | H | $CH_2$ | $CH_2$ | I |
| 1095 | - | - | $CH_2$ | 0 | 4 | H | Me | $CH_2$ | $CH_2$ | I |
| 1096 | - | - | $CH_2$ | 0 | 4 | H | $C_6H_5CO$ | $CH_2$ | $CH_2$ | I |
| 1097 | - | - | $CH_2$ | 1 | 3 | H | $CH_3CO$ | CO | $(CH_2)_3$ | I |
| 1098 | - | - | $CH_2$ | 1 | 3 | Me | $CH_3CO$ | CO | $(CH_2)_3$ | I |

| No. | A | B | D | m | n | R1 | R2 | G | Q | T |
|---|---|---|---|---|---|---|---|---|---|---|
| 1099 | - | - | $CH_2$ | 1 | 3 | Et | $CH_3CO$ | CO | $(CH_2)_3$ | I |
| 1100 | - | - | $CH_2$ | 1 | 3 | $COCH_3$ | $CH_3CO$ | CO | $(CH_2)_3$ | I |
| 1101 | - | - | $CH_2$ | 1 | 3 | $COC_6H_5$ | $CH_3CO$ | CO | $(CH_2)_3$ | I |
| 1102 | - | - | $CH_2$ | 1 | 3 | H | H | CO | $(CH_2)_3$ | I |
| 1103 | - | - | $CH_2$ | 1 | 3 | H | Me | CO | $(CH_2)_3$ | I |
| 1104 | - | - | $CH_2$ | 1 | 3 | H | $C_5H_5CO$ | CO | $(CH_2)_3$ | I |
| 1105 | - | - | $CH_2$ | 1 | 3 | H | $CH_3CO$ | $CH_2$ | $CH_2$ | I |
| 1106 | - | - | $CH_2$ | 1 | 3 | Me | $CH_3CO$ | $CH_2$ | $CH_2$ | I |
| 1107 | - | - | $CH_2$ | 1 | 3 | Et | $CH_3CO$ | $CH_2$ | $CH_2$ | I |
| 1108 | - | - | $CH_2$ | 1 | 3 | $COCH_3$ | $CH_3CO$ | $CH_2$ | $CH_2$ | I |
| 1109 | - | - | $CH_2$ | 1 | 3 | $COC_5H_5$ | $CH_3CO$ | $CH_2$ | $CH_2$ | I |
| 1110 | - | - | $CH_2$ | 1 | 3 | H | H | $CH_2$ | $CH_2$ | I |
| 1111 | - | - | $CH_2$ | 1 | 3 | H | Me | $CH_2$ | $CH_2$ | I |
| 1112 | - | - | $CH_2$ | 1 | 3 | H | $C_5H_5CO$ | $CH_2$ | $CH_2$ | I |
| 1113 | - | - | $CH_2$ | 2 | 2 | H | $CH_3CO$ | CO | $(CH_2)_3$ | I |
| 1114 | - | - | $CH_2$ | 2 | 2 | Me | $CH_3CO$ | CO | $(CH_2)_3$ | I |
| 1115 | - | - | $CH_2$ | 2 | 2 | Et | $CH_3CO$ | CO | $(CH_2)_3$ | I |
| 1116 | - | - | $CH_2$ | 2 | 2 | $COCH_3$ | $CH_3CO$ | CO | $(CH_2)_3$ | I |
| 1117 | - | - | $CH_2$ | 2 | 2 | $COC_6H_5$ | $CH_3CO$ | CO | $(CH_2)_3$ | I |
| 1118 | - | - | $CH_2$ | 2 | 2 | H | H | CO | $(CH_2)_3$ | I |
| 1119 | - | - | $CH_2$ | 2 | 2 | H | Me | CO | $(CH_2)_3$ | I |
| 1120 | - | - | $CH_2$ | 2 | 2 | H | $C_5H_5CO$ | CO | $(CH_2)_3$ | I |

| No. | A | B | D | m | n | R1 | R2 | G | Q | T |
|---|---|---|---|---|---|---|---|---|---|---|
| 1121 | - | - | $CH_2$ | 2 | 2 | H | $CH_3CO$ | $CH_2$ | $CH_2$ | I |
| 1122 | - | - | $CH_2$ | 2 | 2 | Me | $CH_3CO$ | $CH_2$ | $CH_2$ | I |
| 1123 | - | - | $CH_2$ | 2 | 2 | Et | $CH_3CO$ | $CH_2$ | $CH_2$ | I |
| 1124 | - | - | $CH_2$ | 2 | 2 | $COCH_3$ | $CH_3CO$ | $CH_2$ | $CH_2$ | I |
| 1125 | - | - | $CH_2$ | 2 | 2 | $COC_6H_5$ | $CH_3CO$ | $CH_2$ | $CH_2$ | I |
| 1126 | - | - | $CH_2$ | 2 | 2 | H | H | $CH_2$ | $CH_2$ | I |
| 1127 | - | - | $CH_2$ | 2 | 2 | H | Me | $CH_2$ | $CH_2$ | I |
| 1128 | - | - | $CH_2$ | 2 | 2 | H | $C_6H_5CO$ | $CH_2$ | $CH_2$ | I |
| 1129 | - | - | $CH_2$ | 3 | 1 | H | $CH_3CO$ | CO | $(CH_2)_3$ | I |
| 1130 | - | - | $CH_2$ | 3 | 1 | Me | $CH_3CO$ | CO | $(CH_2)_3$ | I |
| 1131 | - | - | $CH_2$ | 3 | 1 | Et | $CH_3CO$ | CO | $(CH_2)_3$ | I |
| 1132 | - | - | $CH_2$ | 3 | 1 | $COCH_3$ | $CH_3CO$ | CO | $(CH_2)_3$ | I |
| 1133 | - | - | $CH_2$ | 3 | 1 | $COC_6H_5$ | $CH_3CO$ | CO | $(CH_2)_3$ | I |
| 1134 | - | - | $CH_2$ | 3 | 1 | H | H | CO | $(CH_2)_3$ | I |
| 1135 | - | - | $CH_2$ | 3 | 1 | H | Me | CO | $(CH_2)_3$ | I |
| 1136 | - | - | $CH_2$ | 3 | 1 | H | $C_5H_5CO$ | CO | $(CH_2)_3$ | I |
| 1137 | - | - | $CH_2$ | 3 | 1 | H | $CH_3CO$ | $CH_2$ | $CH_2$ | I |
| 1138 | - | - | $CH_2$ | 3 | 1 | Me | $CH_3CO$ | $CH_2$ | $CH_2$ | I |
| 1139 | - | - | $CH_2$ | 3 | 1 | Et | $CH_3CO$ | $CH_2$ | $CH_2$ | I |
| 1140 | - | - | $CH_2$ | 3 | 1 | $COCH_3$ | $CH_3CO$ | $CH_2$ | $CH_2$ | I |
| 1141 | - | - | $CH_2$ | 3 | 1 | $COC_6H_5$ | $CH_3CO$ | $CH_2$ | $CH_2$ | I |
| 1142 | - | - | $CH_2$ | 3 | 1 | H | H | $CH_2$ | $CH_2$ | I |

| No. | A | B | D | m | n | R1 | R2 | G | Q | T |
|---|---|---|---|---|---|---|---|---|---|---|
| 1143 | - | - | $CH_2$ | 3 | 1 | H | Me | $CH_2$ | $CH_2$ | I |
| 1144 | - | - | $CH_2$ | 3 | 1 | H | $C_6H_5CO$ | $CH_2$ | $CH_2$ | I |
| 1145 | - | - | $CH_2$ | 4 | 0 | H | $CH_3CO$ | CO | $(CH_2)_3$ | I |
| 1146 | - | - | $CH_2$ | 4 | 0 | Me | $CH_3CO$ | CO | $(CH_2)_3$ | I |
| 1147 | - | - | $CH_2$ | 4 | 0 | Et | $CH_3CO$ | CO | $(CH_2)_3$ | I |
| 1148 | - | - | $CH_2$ | 4 | 0 | $COCH_3$ | $CH_3CO$ | CO | $(CH_2)_3$ | I |
| 1149 | - | - | $CH_2$ | 4 | 0 | COC6H5 | $CH_3CO$ | CO | $(CH_2)_3$ | I |
| 1150 | - | - | $CH_2$ | 4 | 0 | H | H | CO | $(CH_2)_3$ | I |
| 1151 | - | - | $CH_2$ | 4 | 0 | H | Me | CO | $(CH_2)_3$ | I |
| 1152 | - | - | $CH_2$ | 4 | 0 | H | $C_6H_5CO$ | CO | $(CH_2)_3$ | I |
| 1153 | - | - | $CH_2$ | 4 | 0 | H | $CH_3CO$ | $CH_2$ | $CH_2$ | I |
| 1154 | - | - | $CH_2$ | 4 | 0 | Me | $CH_3CO$ | $CH_2$ | $CH_2$ | I |
| 1155 | - | - | $CH_2$ | 4 | 0 | Et | $CH_3CO$ | $CH_2$ | $CH_2$ | I |
| 1156 | - | - | $CH_2$ | 4 | 0 | $COCH_3$ | $CH_3CO$ | $CH_2$ | $CH_2$ | I |
| 1157 | - | - | $CH_2$ | 4 | 0 | $COC_6H_5$ | $CH_3CO$ | $CH_2$ | $CH_2$ | I |
| 1158 | - | - | $CH_2$ | 4 | 0 | H | H | $CH_2$ | $CH_2$ | I |
| 1159 | - | - | $CH_2$ | 4 | 0 | H | Me | $CH_2$ | $CH_2$ | I |
| 1160 | - | - | $CH_2$ | 4 | 0 | H | $C_6H_5CO$ | $CH_2$ | $CH_2$ | I |

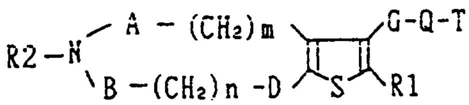

| No. | A | B | D | m | n | R1 | R2 | G | Q | T |
|---|---|---|---|---|---|---|---|---|---|---|
| 1161 | - | - | $CH_2$ | 0 | 4 | H | $CH_3CO$ | CO | $(CH_2)_3$ | I |
| 1162 | - | - | $CH_2$ | 0 | 4 | Me | $CH_3CO$ | CO | $(CH_2)_3$ | I |
| 1163 | - | - | $CH_2$ | 0 | 4 | Et | $CH_3CO$ | CO | $(CH_2)_3$ | I |
| 1164 | - | - | $CH_2$ | 0 | 4 | $COCH_3$ | $CH_3CO$ | CO | $(CH_2)_3$ | I |
| 1165 | - | - | $CH_2$ | 0 | 4 | $COC_6H_5$ | $CH_3CO$ | CO | $(CH_2)_3$ | I |
| 1166 | - | - | $CH_2$ | 0 | 4 | H | H | CO | $(CH_2)_3$ | I |
| 1167 | - | - | $CH_2$ | 0 | 4 | H | Me | CO | $(CH_2)_3$ | I |
| 1168 | - | - | $CH_2$ | 0 | 4 | H | $C_5H_5CO$ | CO | $(CH_2)_3$ | I |
| 1169 | - | - | $CH_2$ | 0 | 4 | H | $CH_3CO$ | $CH_2$ | $CH_2$ | I |
| 1170 | - | - | $CH_2$ | 0 | 4 | Me | $CH_3CO$ | $CH_2$ | $CH_2$ | I |
| 1171 | - | - | $CH_2$ | 0 | 4 | Et | $CH_3CO$ | $CH_2$ | $CH_2$ | I |
| 1172 | - | - | $CH_2$ | 0 | 4 | $COCH_3$ | $CH_3CO$ | $CH_2$ | $CH_2$ | I |
| 1173 | - | - | $CH_2$ | 0 | 4 | $COC_6H_5$ | $CH_3CO$ | $CH_2$ | $CH_2$ | I |
| 1174 | - | - | $CH_2$ | 0 | 4 | H | H | $CH_2$ | $CH_2$ | I |
| 1175 | - | - | $CH_2$ | 0 | 4 | H | Me | $CH_2$ | $CH_2$ | I |
| 1176 | - | - | $CH_2$ | 0 | 4 | H | $C_5H_5CO$ | $CH_2$ | $CH_2$ | I |

| No. | A | B | D | m | n | R1 | R2 | G | Q | T |
|---|---|---|---|---|---|---|---|---|---|---|
| 1177 | - | - | $CH_2$ | 1 | 3 | H | $CH_3CO$ | CO | $(CH_2)_3$ | I |
| 1178 | - | - | $CH_2$ | 1 | 3 | Me | $CH_3CO$ | CO | $(CH_2)_3$ | I |
| 1179 | - | - | $CH_2$ | 1 | 3 | Et | $CH_3CO$ | CO | $(CH_2)_3$ | I |
| 1180 | - | - | $CH_2$ | 1 | 3 | $COCH_3$ | $CH_3CO$ | CO | $(CH_2)_3$ | I |
| 1181 | - | - | $CH_2$ | 1 | 3 | $COC_6H_5$ | $CH_3CO$ | CO | $(CH_2)_3$ | I |
| 1182 | - | - | $CH_2$ | 1 | 3 | H | H | CO | $(CH_2)_3$ | I |
| 1183 | - | - | $CH_2$ | 1 | 3 | H | Me | CO | $(CH_2)_3$ | I |
| 1184 | - | - | $CH_2$ | 1 | 3 | H | $C_6H_5CO$ | CO | $(CH_2)_3$ | I |
| 1185 | - | - | $CH_2$ | 1 | 3 | H | $CH_3CO$ | $CH_2$ | $CH_2$ | I |
| 1186 | - | - | $CH_2$ | 1 | 3 | Me | $CH_3CO$ | $CH_2$ | $CH_2$ | I |
| 1187 | - | - | $CH_2$ | 1 | 3 | Et | $CH_3CO$ | $CH_2$ | $CH_2$ | I |
| 1188 | - | - | $CH_2$ | 1 | 3 | $COCH_3$ | $CH_3CO$ | $CH_2$ | $CH_2$ | I |
| 1189 | - | - | $CH_2$ | 1 | 3 | $COC_6H_5$ | $CH_3CO$ | $CH_2$ | $CH_2$ | I |
| 1190 | - | - | $CH_2$ | 1 | 3 | H | H | $CH_2$ | $CH_2$ | I |
| 1191 | - | - | $CH_2$ | 1 | 3 | H | Me | $CH_2$ | $CH_2$ | I |
| 1192 | - | - | $CH_2$ | 1 | 3 | H | $C_6H_5CO$ | $CH_2$ | $CH_2$ | I |
| 1193 | - | - | $CH_2$ | 2 | 2 | H | $CH_3CO$ | CO | $(CH_2)_3$ | I |
| 1194 | - | - | $CH_2$ | 2 | 2 | Me | $CH_3CO$ | CO | $(CH_2)_3$ | I |
| 1195 | - | - | $CH_2$ | 2 | 2 | Et | $CH_3CO$ | CO | $(CH_2)_3$ | I |
| 1196 | - | - | $CH_2$ | 2 | 2 | $COCH_3$ | $CH_3CO$ | CO | $(CH_2)_3$ | I |
| 1197 | - | - | $CH_2$ | 2 | 2 | $COC_6H_5$ | $CH_3CO$ | CO | $(CH_2)_3$ | I |
| 1198 | - | - | $CH_2$ | 2 | 2 | H | H | CO | $(CH_2)_3$ | I |

| No. | A | B | D | m | n | R1 | R2 | G | Q | T |
|---|---|---|---|---|---|---|---|---|---|---|
| 1199 | - | - | $CH_2$ | 2 | 2 | H | Me | CO | $(CH_2)_3$ | I |
| 1200 | - | - | $CH_2$ | 2 | 2 | H | $C_6H_5CO$ | CO | $(CH_2)_3$ | I |
| 1201 | - | - | $CH_2$ | 2 | 2 | H | $CH_3CO$ | $CH_2$ | $CH_2$ | I |
| 1202 | - | - | $CH_2$ | 2 | 2 | Me | $CH_3CO$ | $CH_2$ | $CH_2$ | I |
| 1203 | - | - | $CH_2$ | 2 | 2 | Et | $CH_3CO$ | $CH_2$ | $CH_2$ | I |
| 1204 | - | - | $CH_2$ | 2 | 2 | $COCH_3$ | $CH_3CO$ | $CH_2$ | $CH_2$ | I |
| 1205 | - | - | $CH_2$ | 2 | 2 | $COC_6H_5$ | $CH_3CO$ | $CH_2$ | $CH_2$ | I |
| 1206 | - | - | $CH_2$ | 2 | 2 | H | H | $CH_2$ | $CH_2$ | I |
| 1207 | - | - | $CH_2$ | 2 | 2 | H | Me | $CH_2$ | $CH_2$ | I |
| 1208 | - | - | $CH_2$ | 2 | 2 | H | $C_6H_5CO$ | $CH_2$ | $CH_2$ | I |
| 1209 | - | - | $CH_2$ | 3 | 1 | H | $CH_3CO$ | CO | $(CH_2)_3$ | I |
| 1210 | - | - | $CH_2$ | 3 | 1 | Me | $CH_3CO$ | CO | $(CH_2)_3$ | I |
| 1211 | - | - | $CH_2$ | 3 | 1 | Et | $CH_3CO$ | CO | $(CH_2)_3$ | I |
| 1212 | - | - | $CH_2$ | 3 | 1 | $COCH_3$ | $CH_3CO$ | CO | $(CH_2)_3$ | I |
| 1213 | - | - | $CH_2$ | 3 | 1 | $COC_6H_5$ | $CH_3CO$ | CO | $(CH_2)_3$ | I |
| 1214 | - | - | $CH_2$ | 3 | 1 | H | H | CO | $(CH_2)_3$ | I |
| 1215 | - | - | $CH_2$ | 3 | 1 | H | Me | CO | $(CH_2)_3$ | I |
| 1216 | - | - | $CH_2$ | 3 | 1 | H | $C_6H_5CO$ | CO | $(CH_2)_3$ | I |
| 1217 | - | - | $CH_2$ | 3 | 1 | H | $CH_3CO$ | $CH_2$ | $CH_2$ | I |
| 1218 | - | - | $CH_2$ | 3 | 1 | Me | $CH_3CO$ | $CH_2$ | $CH_2$ | I |
| 1219 | - | - | $CH_2$ | 3 | 1 | Et | $CH_3CO$ | $CH_2$ | $CH_2$ | I |
| 1220 | - | - | $CH_2$ | 3 | 1 | $COCH_3$ | $CH_3CO$ | $CH_2$ | $CH_2$ | I |

| No. | A | B | D | m | n | R1 | R2 | G | Q | T |
|---|---|---|---|---|---|---|---|---|---|---|
| 1221 | - | - | $CH_2$ | 3 | 1 | $COC_6H_5$ | $CH_3CO$ | $CH_2$ | $CH_2$ | I |
| 1222 | - | - | $CH_2$ | 3 | 1 | H | H | $CH_2$ | $CH_2$ | I |
| 1223 | - | - | $CH_2$ | 3 | 1 | H | Me | $CH_2$ | $CH_2$ | I |
| 1224 | - | - | $CH_2$ | 3 | 1 | H | $C_6H_5CO$ | $CH_2$ | $CH_2$ | I |
| 1225 | - | - | $CH_2$ | 4 | 0 | H | $CH_3CO$ | CO | $(CH_2)_3$ | I |
| 1226 | - | - | $CH_2$ | 4 | 0 | Me | $CH_3CO$ | CO | $(CH_2)_3$ | I |
| 1227 | - | - | $CH_2$ | 4 | 0 | Et | $CH_3CO$ | CO | $(CH_2)_3$ | I |
| 1228 | - | - | $CH_2$ | 4 | 0 | $COCH_3$ | $CH_3CO$ | CO | $(CH_2)_3$ | I |
| 1229 | - | - | $CH_2$ | 4 | 0 | $COC_6H_5$ | $CH_3CO$ | CO | $(CH_2)_3$ | I |
| 1230 | - | - | $CH_2$ | 4 | 0 | H | H | CO | $(CH_2)_3$ | I |
| 1231 | - | - | $CH_2$ | 4 | 0 | H | Me | CO | $(CH_2)_3$ | I |
| 1232 | - | - | $CH_2$ | 4 | 0 | H | $C_6H_5CO$ | CO | $(CH_2)_3$ | I |
| 1233 | - | - | $CH_2$ | 4 | 0 | H | $CH_3CO$ | $CH_2$ | $CH_2$ | I |
| 1234 | - | - | $CH_2$ | 4 | 0 | Me | $CH_3CO$ | $CH_2$ | $CH_2$ | I |
| 1235 | - | - | $CH_2$ | 4 | 0 | Et | $CH_3CO$ | $CH_2$ | $CH_2$ | I |
| 1236 | - | - | $CH_2$ | 4 | 0 | $COCH_3$ | $CH_3CO$ | $CH_2$ | $CH_2$ | I |
| 1237 | - | - | $CH_2$ | 4 | 0 | $COC_5H_5$ | $CH_3CO$ | $CH_2$ | $CH_2$ | I |
| 1238 | - | - | $CH_2$ | 4 | 0 | H | H | $CH_2$ | $CH_2$ | I |
| 1239 | - | - | $CH_2$ | 4 | 0 | H | Me | $CH_2$ | $CH_2$ | I |
| 1240 | - | - | $CH_2$ | 4 | 0 | H | $C_6H_5CO$ | $CH_2$ | $CH_2$ | I |

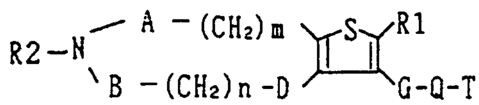

| No. | A | B | D | m | n | R1 | R2 | G | Q | T |
|---|---|---|---|---|---|---|---|---|---|---|
| 1241 | - | - | $CH_2$ | 0 | 2 | H | $CH_3CO$ | CO | $(CH_2)_3$ | I |
| 1242 | - | - | $CH_2$ | 0 | 2 | Me | $CH_3CO$ | CO | $(CH_2)_3$ | I |
| 1243 | - | - | $CH_2$ | 0 | 2 | Et | $CH_3CO$ | CO | $(CH_2)_3$ | I |
| 1244 | - | - | $CH_2$ | 0 | 2 | $COCH_3$ | $CH_3CO$ | CO | $(CH_2)_3$ | I |
| 1245 | - | - | $CH_2$ | 0 | 2 | $COC_6H_5$ | $CH_3CO$ | CO | $(CH_2)_3$ | I |
| 1246 | - | - | $CH_2$ | 0 | 2 | H | H | CO | $(CH_2)_3$ | I |
| 1247 | - | - | $CH_2$ | 0 | 2 | H | Me | CO | $(CH_2)_3$ | I |
| 1248 | - | - | $CH_2$ | 0 | 2 | H | $C_5H_5CO$ | CO | $(CH_2)_3$ | I |
| 1249 | - | - | $CH_2$ | 0 | 2 | H | $CH_3CO$ | $CH_2$ | $CH_2$ | I |
| 1250 | - | - | $CH_2$ | 0 | 2 | Me | $CH_3CO$ | $CH_2$ | $CH_2$ | I |
| 1251 | - | - | $CH_2$ | 0 | 2 | Et | $CH_3CO$ | $CH_2$ | $CH_2$ | I |
| 1252 | - | - | $CH_2$ | 0 | 2 | $COCH_3$ | $CH_3CO$ | $CH_2$ | $CH_2$ | I |
| 1253 | - | - | $CH_2$ | 0 | 2 | $COC_5H_5$ | $CH_3CO$ | $CH_2$ | $CH_2$ | I |
| 1254 | - | - | $CH_2$ | 0 | 2 | H | H | $CH_2$ | $CH_2$ | I |
| 1255 | - | - | $CH_2$ | 0 | 2 | H | Me | $CH_2$ | $CH_2$ | I |
| 1256 | - | - | $CH_2$ | 0 | 2 | H | $C_5H_5CO$ | $CH_2$ | $CH_2$ | I |

| No. | A | B | D | m | n | R1 | R2 | G | Q | T |
|---|---|---|---|---|---|---|---|---|---|---|
| 1257 | - | - | $CH_2$ | 0 | 3 | H | $CH_3CO$ | CO | $(CH_2)_3$ | I |
| 1258 | - | - | $CH_2$ | 0 | 3 | Me | $CH_3CO$ | CO | $(CH_2)_3$ | I |
| 1259 | - | - | $CH_2$ | 0 | 3 | Et | $CH_3CO$ | CO | $(CH_2)_3$ | I |
| 1260 | - | - | $CH_2$ | 0 | 3 | $COCH_3$ | $CH_3CO$ | CO | $(CH_2)_3$ | I |
| 1261 | - | - | $CH_2$ | 0 | 3 | $COC_6H_5$ | $CH_3CO$ | CO | $(CH_2)_3$ | I |
| 1262 | - | - | $CH_2$ | 0 | 3 | H | H | CO | $(CH_2)_3$ | I |
| 1263 | - | - | $CH_2$ | 0 | 3 | H | Me | CO | $(CH_2)_3$ | I |
| 1264 | - | - | $CH_2$ | 0 | 3 | H | $C_6H_5CO$ | CO | $(CH_2)_3$ | I |
| 1265 | - | - | $CH_2$ | 0 | 3 | H | $CH_3CO$ | $CH_2$ | $CH_2$ | I |
| 1266 | - | - | $CH_2$ | 0 | 3 | Me | $CH_3CO$ | $CH_2$ | $CH_2$ | I |
| 1267 | - | - | $CH_2$ | 0 | 3 | Et | $CH_3CO$ | $CH_2$ | $CH_2$ | I |
| 1268 | - | - | $CH_2$ | 0 | 3 | $COCH_3$ | $CH_3CO$ | $CH_2$ | $CH_2$ | I |
| 1269 | - | - | $CH_2$ | 0 | 3 | $COC_6H_5$ | $CH_3CO$ | $CH_2$ | $CH_2$ | I |
| 1270 | - | - | $CH_2$ | 0 | 3 | H | H | $CH_2$ | $CH_2$ | I |
| 1271 | - | - | $CH_2$ | 0 | 3 | H | Me | $CH_2$ | $CH_2$ | I |
| 1272 | - | - | $CH_2$ | 0 | 3 | H | $C_6H_5CO$ | $CH_2$ | $CH_2$ | I |
| 1273 | - | - | $CH_2$ | 0 | 4 | H | $CH_3CO$ | CO | $(CH_2)_3$ | I |
| 1274 | - | - | $CH_2$ | 0 | 4 | Me | $CH_3CO$ | CO | $(CH_2)_3$ | I |
| 1275 | - | - | $CH_2$ | 0 | 4 | Et | $CH_3CO$ | CO | $(CH_2)_3$ | I |
| 1276 | - | - | $CH_2$ | 0 | 4 | $COCH_3$ | $CH_3CO$ | CO | $(CH_2)_3$ | I |
| 1277 | - | - | $CH_2$ | 0 | 4 | $COC_6H_5$ | $CH_3CO$ | CO | $(CH_2)_3$ | I |
| 1278 | - | - | $CH_2$ | 0 | 4 | H | H | CO | $(CH_2)_3$ | I |

| No. | A | B | D | m | n | R1 | R2 | G | Q | T |
|---|---|---|---|---|---|---|---|---|---|---|
| 1279 | - | - | $CH_2$ | 0 | 4 | H | Me | CO | $(CH_2)_3$ | I |
| 1280 | - | - | $CH_2$ | 0 | 4 | H | $C_6H_5CO$ | CO | $(CH_2)_3$ | I |
| 1281 | - | - | $CH_2$ | 0 | 4 | H | $CH_3CO$ | $CH_2$ | $CH_2$ | I |
| 1282 | - | - | $CH_2$ | 0 | 4 | Me | $CH_3CO$ | $CH_2$ | $CH_2$ | I |
| 1283 | - | - | $CH_2$ | 0 | 4 | Et | $CH_3CO$ | $CH_2$ | $CH_2$ | I |
| 1284 | - | - | $CH_2$ | 0 | 4 | $COCH_3$ | $CH_3CO$ | $CH_2$ | $CH_2$ | I |
| 1285 | - | - | $CH_2$ | 0 | 4 | $COC_6H_5$ | $CH_3CO$ | $CH_2$ | $CH_2$ | I |
| 1286 | - | - | $CH_2$ | 0 | 4 | H | H | $CH_2$ | $CH_2$ | I |
| 1287 | - | - | $CH_2$ | 0 | 4 | H | Me | $CH_2$ | $CH_2$ | I |
| 1288 | - | - | $CH_2$ | 0 | 4 | H | $C_6H_5CO$ | $CH_2$ | $CH_2$ | I |

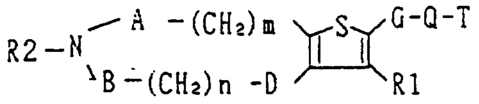

| No. | A | B | D | m | n | R1 | R2 | G | Q | T |
|---|---|---|---|---|---|---|---|---|---|---|
| 1289 | - | - | $CH_2$ | 0 | 2 | H | $CH_3CO$ | CO | $(CH_2)_3$ | I |
| 1290 | - | - | $CH_2$ | 0 | 2 | Me | $CH_3CO$ | CO | $(CH_2)_3$ | I |
| 1291 | - | - | $CH_2$ | 0 | 2 | Et | $CH_3CO$ | CO | $(CH_2)_3$ | I |
| 1292 | - | - | $CH_2$ | 0 | 2 | $COCH_3$ | $CH_3CO$ | CO | $(CH_2)_3$ | I |

| No. | A | B | D | m | n | R1 | R2 | G | Q | T |
|---|---|---|---|---|---|---|---|---|---|---|
| 1293 | - | - | $CH_2$ | 0 | 2 | $COC_6H_5$ | $CH_3CO$ | CO | $(CH_2)_3$ | I |
| 1294 | - | - | $CH_2$ | 0 | 2 | H | H | CO | $(CH_2)_3$ | I |
| 1295 | - | - | $CH_2$ | 0 | 2 | H | Me | CO | $(CH_2)_3$ | I |
| 1296 | - | - | $CH_2$ | 0 | 2 | H | $C_6H_5CO$ | CO | $(CH_2)_3$ | I |
| 1297 | - | - | $CH_2$ | 0 | 2 | H | $CH_3CO$ | $CH_2$ | $CH_2$ | I |
| 1298 | - | - | $CH_2$ | 0 | 2 | Me | $CH_3CO$ | $CH_2$ | $CH_2$ | I |
| 1299 | - | - | $CH_2$ | 0 | 2 | Et | $CH_3CO$ | $CH_2$ | $CH_2$ | I |
| 1300 | - | - | $CH_2$ | 0 | 2 | $COCH_3$ | $CH_3CO$ | $CH_2$ | $CH_2$ | I |
| 1301 | - | - | $CH_2$ | 0 | 2 | $COC_6H_5$ | $CH_3CO$ | $CH_2$ | $CH_2$ | I |
| 1302 | - | - | $CH_2$ | 0 | 2 | H | H | $CH_2$ | $CH_2$ | I |
| 1303 | - | - | $CH_2$ | 0 | 2 | H | Me | $CH_2$ | $CH_2$ | I |
| 1304 | - | - | $CH_2$ | 0 | 2 | H | $C_5H_5CO$ | $CH_2$ | $CH_2$ | I |
| 1305 | - | - | $CH_2$ | 0 | 3 | H | $CH_3CO$ | CO | $(CH_2)_3$ | I |
| 1306 | - | - | $CH_2$ | 0 | 3 | Me | $CH_3CO$ | CO | $(CH_2)_3$ | I |
| 1307 | - | - | $CH_2$ | 0 | 3 | Et | $CH_3CO$ | CO | $(CH_2)_3$ | I |
| 1309 | - | - | $CH_2$ | 0 | 3 | $COCH_3$ | $CH_3CO$ | CO | $(CH_2)_3$ | I |
| 1310 | - | - | $CH_2$ | 0 | 3 | $COC_5H_5$ | $CH_3CO$ | CO | $(CH_2)_3$ | I |
| 1311 | - | - | $CH_2$ | 0 | 3 | H | H | CO | $(CH_2)_3$ | I |
| 1312 | - | - | $CH_2$ | 0 | 3 | H | Me | CO | $(CH_2)_3$ | I |
| 1313 | - | - | $CH_2$ | 0 | 3 | H | $C_6H_5CO$ | CO | $(CH_2)_3$ | I |
| 1314 | - | - | $CH_2$ | 0 | 3 | H | $CH_3CO$ | $CH_2$ | $CH_2$ | I |
| 1315 | - | - | $CH_2$ | 0 | 3 | Me | $CH_3CO$ | $CH_2$ | $CH_2$ | I |

| No. | A | B | D | m | n | R1 | R2 | G | Q | T |
|---|---|---|---|---|---|---|---|---|---|---|
| 1316 | - | - | $CH_2$ | 0 | 3 | Et | $CH_3CO$ | $CH_2$ | $CH_2$ | I |
| 1317 | - | - | $CH_2$ | 0 | 3 | $COCH_3$ | $CH_3CO$ | $CH_2$ | $CH_2$ | I |
| 1318 | - | - | $CH_2$ | 0 | 3 | $COC_6H_5$ | $CH_3CO$ | $CH_2$ | $CH_2$ | I |
| 1319 | - | - | $CH_2$ | 0 | 3 | H | H | $CH_2$ | $CH_2$ | I |
| 1320 | - | - | $CH_2$ | 0 | 3 | H | Me | $CH_2$ | $CH_2$ | I |
| 1321 | - | - | $CH_2$ | 0 | 3 | H | $C_5H_5CO$ | $CH_2$ | $CH_2$ | I |
| 1322 | - | - | $CH_2$ | 0 | 4 | H | $CH_3CO$ | CO | $(CH_2)_3$ | I |
| 1323 | - | - | $CH_2$ | 0 | 4 | Me | $CH_3CO$ | CO | $(CH_2)_3$ | I |
| 1324 | - | - | $CH_2$ | 0 | 4 | Et | $CH_3CO$ | CO | $(CH_2)_3$ | I |
| 1325 | - | - | $CH_2$ | 0 | 4 | $COCH_3$ | $CH_3CO$ | CO | $(CH_2)_3$ | I |
| 1326 | - | - | $CH_2$ | 0 | 4 | $COC_6H_5$ | $CH_3CO$ | CO | $(CH_2)_3$ | I |
| 1327 | - | - | $CH_2$ | 0 | 4 | H | H | CO | $(CH_2)_3$ | I |
| 1328 | - | - | $CH_2$ | 0 | 4 | H | Me | CO | $(CH_2)_3$ | I |
| 1329 | - | - | $CH_2$ | 0 | 4 | H | $C_5H_5CO$ | CO | $(CH_2)_3$ | I |
| 1330 | - | - | $CH_2$ | 0 | 4 | H | $CH_3CO$ | $CH_2$ | $CH_2$ | I |
| 1331 | - | - | $CH_2$ | 0 | 4 | Me | $CH_3CO$ | $CH_2$ | $CH_2$ | I |
| 1332 | - | - | $CH_2$ | 0 | 4 | Et | $CH_3CO$ | $CH_2$ | $CH_2$ | I |
| 1333 | - | - | $CH_2$ | 0 | 4 | $COCH_3$ | $CH_3CO$ | $CH_2$ | $CH_2$ | I |
| 1334 | - | - | $CH_2$ | 0 | 4 | $COC_6H_5$ | $CH_3CO$ | $CH_2$ | $CH_2$ | I |
| 1335 | - | - | $CH_2$ | 0 | 4 | H | H | $CH_2$ | $CH_2$ | I |
| 1336 | - | - | $CH_2$ | 0 | 4 | H | Me | $CH_2$ | $CH_2$ | I |
| 1337 | - | - | $CH_2$ | 0 | 4 | H | $C_6H_5CO$ | $CH_2$ | $CH_2$ | I |

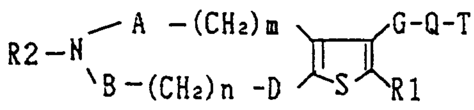

| No. | A | B | D | m | n | R1 | R2 | G | Q | T |
|---|---|---|---|---|---|---|---|---|---|---|
| 1338 | - | - | $CH_2$ | 2 | 0 | Me | $CH_3CO$ | $CH_2$ | $CH_2$ | II |
| 1339 | - | - | $CH_2$ | 2 | 0 | Me | $CH_3CO$ | $CH_2$ | $CH_2$ | III |
| 1340 | - | - | $CH_2$ | 2 | 0 | Me | $CH_3CO$ | $CH_2$ | $CH_2$ | IV |
| 1341 | - | - | $CH_2$ | 2 | 0 | Me | $CH_3CO$ | $CH_2$ | $CH_2$ | V |
| 1342 | - | - | $CH_2$ | 2 | 0 | Me | $CH_3CO$ | $CH_2$ | $CH_2$ | VI |
| 1343 | - | - | $CH_2$ | 2 | 0 | Me | $CH_3CO$ | $CH_2$ | $CH_2$ | VII |
| 1344 | - | - | $CH_2$ | 2 | 0 | Me | $CH_3CO$ | $CH_2$ | $CH_2$ | VIII |
| 1345 | - | - | $CH_2$ | 2 | 0 | Me | $CH_3CO$ | $CH_2$ | $CH_2$ | IX |
| 1346 | - | - | $CH_2$ | 2 | 0 | Me | $CH_3CO$ | $CH_2$ | $CH_2$ | X |
| 1347 | - | - | $CH_2$ | 2 | 0 | Me | $CH_3CO$ | $CH_2$ | $CH_2$ | XI |

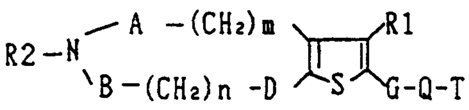

| No. | A | B | D | m | n | R1 | R2 | G | Q | T |
|---|---|---|---|---|---|---|---|---|---|---|
| 1348 | - | CO | $CH_2$ | 0 | 2 | H | H | $CH_2$ | $CH_2$ | II |
| 1349 | - | CO | $CH_2$ | 0 | 2 | H | H | $CH_2$ | $CH_2$ | III |
| 1350 | - | CO | $CH_2$ | 0 | 2 | H | H | $CH_2$ | $CH_2$ | IV |
| 1351 | - | CO | $CH_2$ | 0 | 2 | H | H | $CH_2$ | $CH_2$ | V |
| 1352 | - | CO | $CH_2$ | 0 | 2 | H | H | $CH_2$ | $CH_2$ | VI |
| 1353 | - | CO | $CH_2$ | 0 | 2 | H | H | $CH_2$ | $CH_2$ | VII |
| 1354 | - | CO | $CH_2$ | 0 | 2 | H | H | $CH_2$ | $CH_2$ | VIII |
| 1355 | - | CO | $CH_2$ | 0 | 2 | H | H | $CH_2$ | $CH_2$ | IX |
| 1356 | - | CO | $CH_2$ | 0 | 2 | H | H | $CH_2$ | $CH_2$ | X |
| 1357 | - | CO | $CH_2$ | 0 | 2 | H | H | $CH_2$ | $CH_2$ | XI |

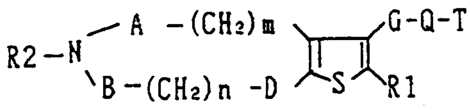

| No. | A | B | D | m | n | R1 | R2 | G | Q | T |
|---|---|---|---|---|---|---|---|---|---|---|
| 1358 | - | CO | $CH_2$ | 0 | 2 | Me | H | $CH_2$ | $CH_2$ | II |
| 1359 | - | CO | $CH_2$ | 0 | 2 | Me | H | $CH_2$ | $CH_2$ | III |
| 1360 | - | CO | $CH_2$ | 0 | 2 | Me | H | $CH_2$ | $CH_2$ | IV |
| 1361 | - | CO | $CH_2$ | 0 | 2 | Me | H | $CH_2$ | $CH_2$ | V |
| 1362 | - | CO | $CH_2$ | 0 | 2 | Me | H | $CH_2$ | $CH_2$ | VI |
| 1363 | - | CO | $CH_2$ | 0 | 2 | Me | H | $CH_2$ | $CH_2$ | VII |
| 1364 | - | CO | $CH_2$ | 0 | 2 | Me | H | $CH_2$ | $CH_2$ | VIII |
| 1365 | - | CO | $CH_2$ | 0 | 2 | Me | H | $CH_2$ | $CH_2$ | IX |
| 1366 | - | CO | $CH_2$ | 0 | 2 | Me | H | $CH_2$ | $CH_2$ | X |
| 1367 | - | CO | $CH_2$ | 0 | 2 | Me | H | $CH_2$ | $CH_2$ | XI |

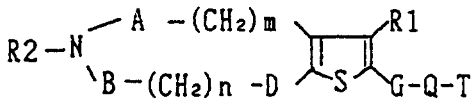

| No. | A | B | D | m | n | R1 | R2 | G | Q | T |
|---|---|---|---|---|---|---|---|---|---|---|
| 1368 | - | - | $CH_2$ | 2 | 0 | Me | $CH_3CO$ | $CH_2$ | $CH_2$ | II |
| 1369 | - | - | $CH_2$ | 2 | 0 | Me | $CH_3CO$ | $CH_2$ | $CH_2$ | III |
| 1370 | - | - | $CH_2$ | 2 | 0 | Me | $CH_3CO$ | $CH_2$ | $CH_2$ | IV |
| 1371 | - | - | $CH_2$ | 2 | 0 | Me | $CH_3CO$ | $CH_2$ | $CH_2$ | V |
| 1372 | - | - | $CH_2$ | 2 | 0 | Me | $CH_3CO$ | $CH_2$ | $CH_2$ | VI |
| 1373 | - | - | $CH_2$ | 2 | 0 | Me | $CH_3CO$ | $CH_2$ | $CH_2$ | VII |
| 1374 | - | - | $CH_2$ | 2 | 0 | Me | $CH_3CO$ | $CH_2$ | $CH_2$ | VIII |
| 1375 | - | - | $CH_2$ | 2 | 0 | Me | $CH_3CO$ | $CH_2$ | $CH_2$ | IX |
| 1376 | - | - | $CH_2$ | 2 | 0 | Me | $CH_3CO$ | $CH_2$ | $CH_2$ | X |
| 1377 | - | - | $CH_2$ | 2 | 0 | Me | $CH_3CO$ | $CH_2$ | $CH_2$ | XI |
| 1378 | - | - | $CH_2$ | 2 | 0 | Me | $CH_3CH_2CO$ | $CH_2$ | $CH_2$ | I |
| 1379 | - | - | $CH_2$ | 2 | 0 | Me | $(CH_3)_2CHCO$ | $CH_2$ | $CH_2$ | I |
| 1380 | - | - | $CH_2$ | 2 | 0 | Me | Cyclopropyl-CO | $CH_2$ | $CH_2$ | I |
| 1381 | - | - | $CH_2$ | 2 | 0 | Me | $C_5H_5CO$ | $CH_2$ | $CH_2$ | I |
| 1382 | - | - | $CH_2$ | 2 | 0 | Me | Cyclohexyl-CO | $CH_2$ | $CH_2$ | I |
| 1383 | - | - | $CH_2$ | 2 | 0 | Me | H | $CH_2$ | $CH_2$ | I |
| 1384 | - | - | $CH_2$ | 2 | 0 | Me | Me | $CH_2$ | $CH_2$ | I |

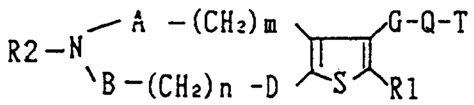

| No. | A | B | D | m | n | R1 | R2 | G | Q | T |
|---|---|---|---|---|---|---|---|---|---|---|
| 1385 | - | - | $CH_2$ | 0 | 2 | Me | H | $CH_2$ | $CH_2$ | I |
| 1386 | - | - | $CH_2$ | 0 | 2 | Me | H | CO | $(CH_2)_3$ | I |
| 1387 | - | - | $CH_2$ | 1 | 1 | Me | H | $CH_2$ | $CH_2$ | I |
| 1388 | - | - | $CH_2$ | 1 | 1 | Me | H | CO | $(CH_2)_3$ | I |
| 1389 | - | - | $CH_2$ | 2 | 0 | Et | $CH_3CO$ | $CH_2$ | $CH_2$ | V |

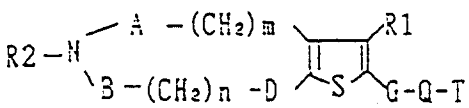

| No. | A | B | D | m | n | R1 | R2 | G | Q | T |
|---|---|---|---|---|---|---|---|---|---|---|
| 1390 | - | - | $CH_2$ | 2 | 0 | H | $COCH_3$ | CO | $(CH_2)_3$ | V |
| 1391 | - | CO | $CH_2$ | 0 | 2 | H | H | $CH_2$ | $CH_2$ | V |

The pharmacological activities of the compound of formula (I) were examined by a series of receptor binding tests, anti-apomorphine action and anti-ergometrine action as described below.

Experiment Example 1: Affinity for dopamine 2 receptor

A specific binding of dopamine 2 ($D_2$) receptor was tested according to the method described in Eur. J. Pharmacol. 46, 377 (1977).

A synaptosome fraction was separated from corpus striatum of 9-10 week-old Wistar rats, and suspended in 50 mM Tris-HCl buffer (pH 7.1) containing 120 mM sodium chloride, 5 mM potassium chloride, 2 mM calcium chloride, 1 mM magnesium chloride, 10 µM pargyline and 0.1% ascorbic acid for the experiment.

The test compound at several concentrations and tritiated spiperone (final concentration 0.2 nM) were added to the synaptosome suspension, and each mixture was incubated at 37°C for 20 minutes. After the incubation, the mixture was filtered with suction through Whatman GF/B (trade mark) glass filter. The filter was washed with 50 mM Tris-HCl buffer (pH 7.7), and the radioactivity of the filter was measured by a liquid scintillation counter. Non-specific binding was determined in the presence of $10^{-4}$ M (±)-sulpiride. The

concentration necessary for 50% inhibition ($IC_{50}$) was determined on a graph, and inhibition constant (Ki value) was calculated. The results are shown in Table 1.

Experiment Example 2 : Affinity for serotonin 2 receptor

A specific binding of serotonin 2 ($5\text{-}HT_2$) receptor was tested according to the method described in Mol. Pharmacol. 21, 301 (1981).

A crude synaptosome fraction was separated from hippocampus of 9-10 week-old Wistar rats, and suspended in 50 mM Tris-HCl buffer (pH 7.7) for the experiment. The test compound at several concentration and tritiated ketanserin (final concentration 0.2 nM) were added to the synaptosome suspension, and each mixture was incubated at 37° C for 20 minutes. After the incubation, the mixture was filtered with suction through Whatman GF/B (trade mark) glass filter. The filter was washed with 50 mM Tris-HCl buffer (pH 7.7), and the radioactivity of the filter was measured by a liquid scintillation counter. Non-specific binding was determined in the presence of $10^{-5}$ M mianserin. The concentration necessary for 50% inhibition ($IC_{50}$) was determined on a graph, and inhibition constant (Ki value) was calculated. The result are shown in Table 1.

Experiment Example 3 : Affinity for serotonin 1A receptor

A specific binding of serotonin 1A ($5\text{-}HT_{1A}$) receptor was tested according to the method described in J. Neurochem. 44, 1685 (1985).

A crude synaptosome fraction was separated from hippocampus of 9-10 week-old Wistar rats, and suspended in 50 mM Tris-HCl buffer (pH 7.4) containing 1 mM manganese chloride for the experiment. The test compound at several concentrations and tritiated 8-hydroxy-2-dipropylaminotetralin (8-OH-DPAT, final concentration 0.2 nM) were added to the synaptosome suspension, and each mixture was incubated at 37° C for 12 minutes. After the incubation, the mixture was filtered with suction through Whatman GF/B (trade mark) glass filter. The filter was washed with 50 mM Tris-HCl buffer (pH 7.7), and the radioactivity of the filter was measured by a liquid scintillation counter. Non-specific binding was determined in the presence of $10^{-5}$ M serotonin (5-HT). The concentration necessary for 50% inhibition ($IC_{50}$) was determined on a graph, and inhibition constant (Ki value) was calculated. The results are shown in Table 1.

Table 1

| Test compound (Example No.) | Ki (nM) | | |
|---|---|---|---|
| | $D_2$ | $5\text{-}HT_2$ | $5\text{-}HT_{1A}$ |
| 15 | 0.065 | 0.32 | 1.6 |
| 22 | 0.52 | 0.21 | 15 |
| 24 | 0.46 | 0.062 | 32 |
| 32 | 0.10 | 0.086 | 54 |
| 42 | 0.23 | 0.20 | 1.1 |
| 43 | 1.5 | 0.11 | 87 |
| 53 | 0.15 | 0.043 | 3.7 |

Experiment Example 4: Anti-apomorphine action (in mouse)

Male dd mice at 3 per group were used for the experiment. The test compound was orally administered to the mice, and 60 minutes later, 0.5 mg/kg of apomorphine hydrochloride was subcutaneously administered. The movement for 20 minutes from immediately after the administration of apomorphine hydrochloride was measured using Valimex (Columbus, USA). Each group was tested four times, and the amount of the test compound necessary to lower the movement by 50% than that of a control group was calculated from a graph and taken as $ED_{50}$. The results are shown in Table 2.

Experiment Example 5: Anti-ergometrine action

Male ddY mice at 12 per group were used for the experiment. The test compound was orally administered to the mice, and 60 minutes later, 20 mg/kg of ergometrine maleate was intraperitoneally administered. The amount of the test compound necessary to vanish the head-twitch reaction for 10 minutes from immediately after the administration of ergometrine maleate was calculated as $ED_{50}$ value by Probit method. The results are shown in Table 2.

Table 2

| Test compound (Example No.) | $ED_{50}$ (mg/kg, p.o.) | |
|---|---|---|
| | anti-apomorphine | anti-ergometrine |
| 15 | 0.063 | 0.089 |
| 22 | 1.1 | 1.5 |
| 24 | 0.39 | 0.37 |
| 42 | 0.20 | 0.19 |

Experiment Example 6: Acute toxicity

Male ddY mice weighing 20-28g were used at 10 per group. They were orally administered with the compound of Example 24 and observed for 5 days after the administration. The $LD_{50}$ (50% lethal dose) was 205mg/kg.

The compounds of the formula (I) possess various pharmacological actions required of an antipsychotic such as motility suppressing action, anti-apomorphine action, methamphetamine antagonistic action, tetrabenazine-induced blepharoptosis enhancing action, and the like, and additionally possess strong inhibitory action on ergometrine induced head-twitch. In the determination of affinity for receptors using tritium-labeled ligands, the compounds of the invention showed high affinity for dopamine ($D_2$) receptor and serotonin 2 ($5\text{-}HT_2$) receptor. Furthermore, catalepsy inducing action, which is an index of extrapyramidal side-effects, was examined using rats, and the compounds of the invention showed extremely weak action. From the result, the compounds of the invention can be expected to improve negative symptoms in addition to positive symptoms and have been proved to be useful as an antipsychotic with high safety exhibiting less extrapyramidal symptoms and less endocrinium side-effects. Also, the compounds of the invention have been found to show high affinity for serotonin 1A ($5\text{-}HT_{1A}$) receptor, and have antianxiety activity and are also useful as non-benzodiazepine type antianxiety agent. The compounds of formula (II) are useful as synthetic intermediate for the compounds of formula (I).

When the compounds of formula (I) of the present invention are used as pharmaceuticals, a therapeutically effective amount of the compounds and adequate pharmacologically acceptable additives such as excipient, carrier, diluent and so on are mixed to be formulated into a form such as tablets, capsules, granules, syrups, injectable solutions, suppositories, dispersible powders or the like and are administered in the form mentioned above. The dosage may generally range about 5 to about 500 mg per day for an adult in a single dose or divided doses in the case of oral administration.

Best Mode for Carrying Out the Invention

The present invention will be explained in more detail by the following examples, but these examples are not to be construed as limiting the present invention. The following compounds obtained can be identified by the various analysis such as NMR, IR, elemental analysis, mass spectrometry and so on.

Reference Example 1

To a solution of 171 g of 2-methylthiophene in 1500 ml of methylene chloride were added dropwise 500 g of tin(IV) chloride and 300 g of ethyl succinyl chloride under ice-cooling and the mixture was stirred for 1.5 hours. The mixture was poured into water, extracted with chloroform and the organic layer was washed with water, dried over magnesium sulfate and distilled away. To the residue was added isopropyl ether and

the precipitated crystals were collected by filtration to give 299 g of white crystals. The resulting crystals were dissolved in 1600 ml of diethylene glycol and 264 g of 100% hydrazine monohydrate was add thereto and the mixture was stirred at 110°C for 45 minutes. After cooling to 60°C, 445 g of potassium hydroxide was added thereto and the mixture was stirred at 140°C for 1.5 hours. The mixture was poured into water, acidified with hydrochloric acid and extracted with ethyl acetate. The organic layer was dried over magnesium sulfate and the solvent was distilled away. The residue was dissolved in 500 ml of acetic anhydride, 5 ml of phosphoric acid was added thereto and the mixture was refluxed with stirring for 80 minutes. The mixture was poured into water, made alkaline with potassium carbonate and extracted with ethyl acetate. The organic layer was washed with water, dried over magnesium sulfate and concentrated in vacuo. The mixture of 164 g of the residue, 103 g of hydroxylamine hydrochloride and 124 g of sodium hydrogencarbonate in 500ml of ethanol was refluxed with stirring for 1.5 hours. The mixture was concentrated in vacuo, water was added to the residue and extracted with ethyl acetate. The organic layer was washed with water, dried and distilled away. The residue was recrystallised from ethanol to give 152 g of white crystals. The resulting crystals were portionwise added to polyphosphoric acid at 60°C. The mixture was poured into water and extracted with chloroform. The extract was washed with water, dried over magnesium sulfate and concentrated in vacuo. The residue was chromatographed on a silica gel to give 30 g of 2-methyl-4,6,7,8-tetrahydro-5H-thieno[3,2-b]azepin-5-one (m.p. 133-135°C) and 93 g of 2-methyl-5,6,7,8-tetrahydro-4H-thieno[3,2-c]azepin-4-one (m.p. 119-120°C).

Reference Example 2

To a mixture of 61 g of thiophene in 800ml of tetrahydrofuran was added 500 ml of n-butyllithium dropwise at -25°C and the mixture was stirred for an hour. To the mixture was added 24.4g of sulfur at -25°C, the mixture was stirred for 1.5 hours and 111.1g of 3-bromopropionic acid in 50.1 g of potassium carbonate solution was added dropwise thereto, and then the mixture was stirred at room temperature for 4 hours. The aqueous layer was collected fractionally, washed with toluene, acidified with hydrochloric acid and extracted with chloroform. The organic layer was washed with water, dried and concentrated to give 135 g of a pale yellow oil. To a solution of 125 g of the oil in 1200ml of toluene was added 153.6 g of trifluoroacetic anhydride dropwise, the mixture was stirred at room temperature for an hour and stirred at 40°C for further 2 hours. The mixture was poured into ice-cold water and neutralized with sodium hydroxide solution. The toluene layer was washed with water, dried and concentrated in vacuo to give 103 g of a brown oil. To a solution of 103 g of the oil in 1000 ml of ethanol were added 49.9 g of hydroxylamine hydrochloride and 60.3 g of sodium hydrogencarbonate and the mixture was refluxed with stirring for 2 hours. Inorganic substances were filtered off and the filtrate was concentrated. To the residue was added isopropyl ether and the crystals were collected by filtration to give 77 g of white crystals. The resulting crystals were added portionwise to 100% polyphosphoric acid at 70°C and the mixture was stirred at 80°C for an hour. After the mixture was poured into water, the precipitated crystals were collected by filtration and recrystallized from ethanol to give 63 g of 2,3-dihydrothieno[3,2-f][1,4]thiazepin-5(4H)-one as pale brown crystals, m.p. 157-158°C.

Example 1

To a mixture of 13 g of 2,3-dihydrothieno[3,2-f][1,4]thiazepin-5(4H)-one and 21.5 g of 4-chlorobutyryl chloride in 200 ml of dichloroethane was added 21.5 g of aluminum chloride under cooling and the mixture was stirred for 3 hours. The mixture was poured into ice-cold water and extracted with chloroform. The organic layer was washed with saline solution, dried over magnesium sulfate and concentrated. The residue was recrystallized from ethanol to give 12 g of 7-(4-chlorobutyryl)-2,3-dihydrothieno[3,2-f][1,4]thiazepin-5-(4H)-one as white crystals, m.p. 124-125°C.

Example 2

To a mixture of 5 g of 2-methyl-5,6,7,8-tetrahydro-4H-thieno[3,2-c]azepin-4-one and 5 g of 4-chlorobutyryl chloride in 50 ml of dichloroethane was added 8.6 g of aluminum chloride under ice-cooling and the mixture was stirred for 3 hours. The mixture was poured into ice-cold water and extracted with chloroform. The organic layer was washed with saline solution, dried over magnesium sulfate and concentrated. The residue was recrystallized from ethanol to give 1.2 g of 3-(4-chlorobutyryl)-2-methyl-5,6,7,8-tetrahydro-4H-thieno[3,2-c]azepin-4-one as white crystals, m.p. 113-115°C.

Example 3

To a mixture of 5 g of 2-methyl-5,6,7,8-tetrahydro-4H-thieno[3,2-b]azepin-5-one and 4.5 g of 3-chloropropionyl chloride in 50 ml of dichloroethane was added 9.2 g of aluminum chloride and the mixture was stirred for 3 hours. The mixture was poured into ice-cold water and extracted with chloroform. The organic layer was washed with saline solution, dried over magnesium sulfate and concentrated. The residue was recrystallized from ethanol to give 5.6 g of 3-(3-chloropropionyl)-2-methyl-5,6,7,8-tetrahydro-4H-thieno-[3,2-b]azepin-5-one as white crystals, m.p. 86-87 °C.

Example 4

The mixture of 1.0 g of 4-(1,2-benzisothiazol-3-yl)piperazine hydrochloride, 1.5 g of 7-(4-chlorobutyryl)-2,3-dihydrothieno[3,2-f][1,4]thiazepin-5(4H)-one, 2.5 g of potassium carbonate and 0.8 g of potassium iodide in 25 ml of dimethylformamide and 25 ml of toluene was stirred at 100 °C for 24 hours. After the mixture was cooled in a water bath, water was added thereto and the mixture was extracted with ethyl acetate. The organic layer was washed with saline solution, dried over magnesium sulfate and concentrated. The residue was purified by column chromatography on a silica gel, dissolved in isopropyl alcohol and oxalic acid was added thereto to make oxalate. The resulting crystals were recrystallized from isopropyl alcohol to give 0.12 g of 7-(4-(4-(1,2-benzisothiazol-3-yl)piperazin-1-yl)butyryl)-2,3-dihydrothieno[3,2-f][1,4]thiazepin-5(4H)-one oxalate 1/2 hydrate as white crystals, m.p. 118-120 °C.

Example 5

The mixture of 2.0 g of 4-(1,2-benzisothiazol-3-yl)piperazine hydrochloride, 2.0 g of 3-(4-chlorobutyryl)-2-methyl-5,6,7,8-tetrahydro-4H-thieno[3,2-c]azepin-4-one, 2.1 g of potassium carbonate and 1.2 g of potassium iodide in 15 ml of dimethylformamide and 15 ml of toluene was stirred at 60 °C for 3 hours. After the mixture was cooled in a water bath, water was added thereto and the mixture was extracted with ethyl acetate. The organic layer was washed with saline solution, dried over magnesium sulfate and concentrated. The residue was purified by column chromatography on a silica gel, dissolved in isopropyl alcohol and ethanolmaleic acid was added thereto to make meleate. The resulting crystals were recrystallized from ethanol to give 0.90 g of 3-(4-(4-(1,2-benzisothiazol-3-yl)piperazin-1-yl)butyryl)-2-methyl-5,6,7,8-tetrahydro-4H-thieno[3,2-c]azepin-4-one maleate as white crystals, m.p. 150-152 °C.

Example 6

The mixture of 2.1 g of 4-(1,2-benzisothiazol-3-yl)piperazine hydrochloride, 2.0 g of 3-(3-chloropropionyl)-2-methyl-4,6,7,8-tetrahydro-5H-thieno[3,2-b]azepin-5-one, 2,2 g of potassium carbonate and 1.2 g of potassium iodide in 15 ml of dimethylformamide and 15 ml of toluene was stirred at 60 °C for 5 hours. After the mixture was cooled in a water bath, water was added thereto and the mixture was extracted with ethyl acetate. The organic layer was washed with saline solution, dried over magnesium sulfate and concentrated. The residue was purified by column chromatography on a silica gel, dissolved in isopropyl alcohol and crystallized from isopropyl alcohol-isopropyl ether. The resulting crystals were recrystallized from ethanol to give 1.30 g of 3-(3-(4-(1,2-benzisothiazol-3-yl)piperazin-1-yl)propionyl)-2-methyl-4,6,7,8-tetrahydro-5H-thieno[3,2-b]azepin-5-one as white crystals, m.p. 146-147 °C.

Example 7

The mixture of 2.4 g of 4-(bis(4-chlorophenyl)methyl)piperazine, 2.0 g of 7-(4-chlorobutyryl)-2,3-dihydrothieno[3,2-f][1,4]thiazepin-5(4H)-one, 1.9 g of potassium carbonate and 1.2g of potassium iodide in 15 ml of dimethylformamide and 15 ml of toluene was stirred at 60 °C for 5 hours. After the mixture was cooled in a water bath, water was added thereto. The precipitated crystals were filtered off and the filtrate was extracted with toluene. The organic layer was washed with saline solution, dried over magnesium sulfate and concentrated. The residue was purified by column chromatography on a silica gel and the resulting crystals were recrystallized from isopropyl alcohol-isopropyl ether to give 0.18 g of 7-(4-(4-(bis (4-chlorophenyl)methyl)piperazin-1-yl)butyryl)-2,3-dihydrothieno[3,2-f][1,4]thiazepin-5(4H)-one as white crystals, m.p. 185-187 °C (decomposition).

The following compounds can be prepared in a similar manner as the above.

Example 8

2-(4-(4-(2-methoxyphenyl)piperazin-1-yl)butyryl)-4,6,7,8-tetrahydro-5H-thieno[3,2-b]azepin-5-one, m.p. 161-162°C

Example 9

2-(4-(4-(4-chlorophenyl)-4-hydroxypiperidin-1-yl)butyryl)-4,6,7,8-tetrahydro-5H-thieno[3,2-b]azepin-5-one, m.p. 192-194°C

Example 10

2-(3-(4-(2-methoxyphenyl)piperazin-1-yl)propionyl)-4,6,7,8-tetrahydro-5H-thieno[3,2-b]azepin-5-one, m.p. 161-162°C

Example 11

2-(3-(4-(3-trifluoromethylphenyl)piperazin-1-yl)propionyl)-4,6,7,8-tetrahydro-5H-thieno[3,2-b]azepin-5-one, m.p. 137-138°C

Example 12

7-(4-(4-(2-methoxyphenyl)piperazin-1-yl)butyryl)-2,3-dihydrothieno[3,2-f][1,4]thiazepin-5(4H)-one, m.p. 197-198°C

Example 13

2-methyl-3-(4-(4-(2-methoxyphenyl)piperazin-1-yl)butyryl)-5,6,7,8-tetrahydro-4H-thieno[3,2-c]azepin-4-one hydrochloride, m.p. 220-222°C

Example 14

7-(4-(4-(6-fluoro-1,2-benzisoxazol-3-yl)piperidin-1-yl)butyryl)-2,3-dihydrothieno[3,2-f][1,4]thiazepin-5(4H)-one, m.p. 186-187°C

Example 15

2-(2-(4-(6-fluoro-1,2-benzisoxazol-3-yl)piperidin-1-yl)ethyl)-4,6,7,8-tetrahydro-5H-thieno[3,2-b]azepin-5-one, m.p. 210-212°C (decomposition)

Example 16

2,4-dimethyl-3-(2-(4-(6-fluoro-1,2-benzisoxazol-3-yl)piperidin-1-yl)ethyl)-4,6,7,8-tetrahydro-5H-thieno[3,2-b]azepin-5-one hydrochloride, m.p. 250°C (decomposition)

Example 17

3-(4-(4-(6-fluoro-1,2-benzisoxazol-3-yl)piperidin-1-yl)butyryl)-2-methyl-4,6,7,8-tetrahydro-5H-thieno[3,2-b]azepin-5-one oxalate 3/2hydrate, m.p. 115-118°C

Example 18

2-(4-(4-(6-fluoro-1,2-benzisoxazol-3-yl)piperidin-1-yl)butyryl)-3-methyl-4,6,7,8-tetrahydro-5H-thieno[3,2-b]azepin-5-one, m.p. 172-175°C

Example 19

3-(2-(4-(6-fluoro-1,2-benzisoxazol-3-yl)piperidin-1-yl)ethyl)-2-methyl-4,6,7,8-tetrahydro-5H-thieno[3,2-b]-azepin-5-one hydrochloride, m.p. 184-186 ° C (decomposition)

Example 20

3-(4-(4-(6-fluoro-1,2-benzisoxazol-3-yl)piperidin-1-yl)butyryl)-2-methyl-5,6,7,8-tetrahydro-4H-thieno[3,2-c]azepin-4-one oxalate, m.p. 114-117 ° C

Example 21

3-(4-(4-(6-fluoro-1,2-benzisoxazol-3-yl)piperidin-1-yl)acetyl)-2-methyl-4,6,7,8-tetrahydro-5H-thieno[3,2-b]-azepin-5-onehydrochloride 1/2hydrate, m.p. 208-210 ° C (decomposition)

Example 22

3-(2-(4-(6-fluoro-1,2-benzisoxazol-3-yl)piperidin-1-yl)ethyl)-2-methyl-4,6,7,8-tetrahydro-5H-thieno[3,2-b]-azepin-5-one,m.p. 165-167 ° C

Example 23

2-(2-(4-(6-fluoro-1,2-benzisoxazol-3-yl)piperidin-1-yl)ethyl)-5,6,7,8-tetrahydro-4H-thieno[3,2-c]azepin-4-one, m.p. 182-184 ° C

◎ 2-(4-(4-(6-fluoro-1,2-benzisoxazol-3-yl)piperidin-1-yl)butyryl)-4,6,7,8-tetrahydro-5H-thieno[3,2-c]azepin-4-one

◎ 2-(4-(4-(6-fluoro-1,2-benzisoxazol-3-yl)piperidin-1-yl)butyryl)-4,6,7,8,-tetrahydro-5H-thieno[3,2-b]azepin-5-one

◎ 7-(4-(4-(6-fluoro-1,2-benzisoxazol-3-yl)piperidin-1-yl)butyryl)-2,3-dihydrothieno[3,2-f][1,4]thiazepin-5(4H)-one

◎ 7-(4-(4-(6-fluoro-1,2-benzisoxazol-3-yl)piperidin-1-yl)butyryl)-3,4-dihydrothieno[2,3-b][1,4]thiazepin-2(1H)-one

◎ 2-(4-(4-(6-fluoro-1,2-benzisoxazol-3-yl)piperidin-1-yl)-1-hydroxybutyl)-5,6,7,8-tetrahydro-4H-thieno[3,2-c]-azepin-4-one

◎ 2-(4-(4-(6-fluoro-1,2-benzisoxazol-3-yl)piperidin-1-yl)butyl)-5,6,7,8-tetrahydro-4H-thieno[3,2-c]azepin-4-one

◎ 2-(4-(4-(6-fluoro-1,2-benzisoxazol-3-yl)piperidin-1-yl)butyl)-5,6,7,8-tetrahydro-4H-thieno[3,2-c]azepin-4-one

◎ 2-(4-(4-(6-fluoro-1,2-benzisoxazol-3-yl)piperidin-1-yl)acetyl)-4,6,7,8-tetrahydro-5H-thieno[3,2-b]azepin-5-one

◎ 2-(4-(4-(6-fluoro-1,2-benzisoxazol-3-yl)piperidin-1-yl)ethyl)-5,6,7,8-tetrahydro-4H-thieno[3,2-c]azepin-4-one

◎ 2-(4-(4-(6-fluoro-1,2-benzisoxazol-3-yl)piperidin-1-yl)ethyl)-4,6,7,8-tetrahydro-5H-thieno[3,2-c]azepin-5-one

◎ 2-(4-N,N-dimethylamino)butyryl)-5,6,7,8-tetrahydro-4H-thieno[3,2-c]azepin-4-one

◎ 2-(4-(N,N-dimethylamino)butyryl)-4,6,7,8-tetrahydro-5H-thieno[3,2-b]azepin-5-one

◎ 2-(2-(N,N-dimethylamino)acetyl)-5,6,7,8-tetrahydro-4H-thieno[3,2-c]azepin-4-one

◎ 2-(2-(N,N-dimethylamino)acetyl)-4,6,7,8-tetrahydro-5H-thieno[3,2-b]azepin-5-one

◎ 2-(3-(4-(6-fluoro-1,2-benzisoxazol-3-yl)piperidin-1-yl)propylthio)-5,6,7,8-tetrahydro-4H-thieno[3,2-c]azepin-4-one

◎ 2-(3-(4-(6-fluoro-1,2-benzisoxazol-3-yl)piperidin-1-yl)propylsulfinyl)-5,6,7,8-tetrahydro-4H-thieno[3,2-c]-azepin-4-one

◎ 2-(3-(4-(6-fluoro-1,2-benzisoxazol-3-yl)piperidin-1-yl)propylsulfonyl)-5,6,7,8-tetrahydro-4H-thieno[3,2-c]-azepin-4-one

◎ 2-(3-(4-(6-fluoro-1,2-benzisoxazol-3-yl)piperidin-1-yl)propylthio)-4,6,7,8-tetrahydro-5H-thieno[3,2-b]azepin-5-one

◎ 2-(3-(4-(6-fluoro-1,2-benzisoxazol-3-yl)piperidin-1-yl)propylsulfinyl)-4,6,7,8-tetrahydro-5H-thieno[3,2-b]-azepin-5-one

◎ 2-(3-(4-(6-fluoro-1,2-benzisoxazol-3-yl)piperidin-1-yl)propylsulfonyl)-4,6,7,8-tetrahydro-5H-thieno[3,2-b]-azepin-5-one
◎ 2-(3-(4-(6-fluoro-1,2-benzisoxazol-3-yl)piperidin-1-yl)butyryl)-4,5-dihydrothieno[2,3-b]pyridin-6(7H)-one
◎ 2-(4-(4-(6-fluoro-1,2-benzisoxazol-3-yl)piperidin-1-yl)butyryl)-4,5-dihydrothieno[2,3-c]pyridin-7(6H)-one
◎ 2-(4-(4-(6-fluoro-1,2-benzisoxazol-3-yl)piperidin-1-yl)butyryl)-4,5-dihydro-7H-thieno[3,2-c]pyridin-6-one
◎ 2-(4-(4-(6-fluoro-1,2-benzisoxazol-3-yl)piperidin-1-yl)butyryl)-6,7-dihydro-4H-thieno[2,3-c]pyridin-5-one
◎ 2-(2-(4-(6-fluoro-1,2-benzisoxazol-3-yl)piperidin-1-yl)acetyl)-4,5-dihydro-7H-thieno[3,2-c]pyridin-6-one
◎ 2-(2-(4-(6-fluoro-1,2-benzisoxazol-3-yl)piperidin-1-yl)acetyl)-6,7-dihydro-4H-thieno[2,3-c]pyridin-5-one
◎ 2-(3-(4-(6-fluoro-1,2-benzisoxazol-3-yl)piperidin-1-yl)propylthio)-6,7-dihydro-4H-thieno[2,3-c]pyridin-5-one
◎ 2-(3-(4-(6-fluoro-1,2-benzisoxazol-3-yl)piperidin-1-yl)propylsulfinyl)-6,7-dihydro-4H-thieno[2,3-c]pyridin-5-one
◎ 2-(3-(4-(6-fluoro-1,2-benzisoxazol-3-yl)piperidin-1-yl)propylsulfonyl)-6,7-dihydro-4H-thieno[2,3-c]pyridin-5-one

Reference Example 3

A mixture of 50 g of 5-methyl-2-thiophenealdehyde and 42 g of aminoacetoaldehyde dimethyl acetal was stirred at 60-70 °C for an hour and concentrated to give 71 g of oil. The resulting oil was dissolved in 450 ml of methanol, 25 g of sodium borohydride was added thereto under cooling and the mixture was stirred for 10-20 minutes, and then concentrated. To the residue was added water and extracted with chloroform. The extract was washed with water, dried over magnesium sulfate and concentrated. To the residue were added 264 ml of water and 132 ml of conc. hydrochloric acid and the mixture was stirred at 50 °C for 2 hours. After cooling, 50ml of conc. hydrochloric acid and 265 g of tin(II) chloride dihydrate were added thereto and the mixture was stirred at 60 °C for 2 hours. The mixture was poured into water, made alkaline with 40% sodium hydroxide solution and extracted with chloroform. The organic layer was washed with water, dried over magnesium sulfate and concentrated. The residue was dissolved in 200 ml of tetrahydrofuran, and 80 ml of triethylamine and 40 ml of acetic anhydride were added thereto under cooling, and then the mixture was stirred for an hour. The mixture was poured into water, extracted with ethyl acetate and the extract was washed with water, dried and concentrated. The residue was subjected to column chromatography to give 27 g of 6-acetyl-4,5,6,7-tetrahydro-2-methylthieno[2,3-c]pyridine.

Reference Example 4

To a solution of 20 g of 4,5,6,7-tetrahydrothieno[3,2-c]pyridine as an oil obtained by a known method in 100 ml of tetrahydrofuran were added 30 ml of triethylamine and 15 ml of acetic anhydride under cooling. The mixutre was stirred for an hour, poured into water and extracted with ethyl acetate. The extract was washed with water, dried and concentrated to give 10 g of 5-acetyl-4,5,6,7-tetrahydrothieno[3,2-c]pyridine.

Preparative Example 1

To a mixture of 27 g of 6-acetyl-4,5,6,7-tetrahydro-2-methylthieno[2,3-c]pyridine and 16 g of acetyl chloride in 250 ml of dichloroethane was added 42 g of aluminum chloride under cooling and the mixture was stirred for 30 minutes. The mixture was poured into water and extracted with chloroform. The organic layer was washed with water, dried over magnesium sulfate and concentrated. The residue was dissolved in 300 ml of methanol, 20 g of bromine was added thereto at 30 °C and the mixture was stirred for 2 hours. The mixture was poured into water and extracted with ethyl acetate. The organic layer was washed with water, dried and concentrated to give 28 g of 6-acetyl-3-bromoacetyl-4,5,6,7-tetrahydro-2-methylthieno[2,3-c]pyridine.

Preparative Example 2

A mixture of 28 g of 6-acetyl-3-bromoacetyl-4,5,6,7-tetrahydro-2-methylthieno[2,3-c]pyridine in 42 ml of triethylsilane and 140 ml of trifluoroacetic acid was stirred at room temperature for 5 hours. The mixture was poured into water, made alkaline and extracted with ethyl acetate. The organic layer was washed with water, dried and concentrated. To the residue was added isopropyl ether and the precipitated crystals were collected by filtration to give 14 g of 6-acetyl-3-(2-bromoethyl)-4,5,6,7-tetrahydro-2-methylthieno[2,3-c]-pyridine as white crystals, m.p. 93-95 °C.

The following compounds were prepared in a similar manner as the above. Figures mean δ value of $^1H$-NMR ($CDCl_3$).

◎ 5-acetyl-2-(2-bromoethyl)-4,5,6,7-tetrahydrothieno[3,2-c]pyridine 6.66(s,1H), 4.60(d,2H), 4.00-3.00(m,6H), 2.80(b,2H), 2.15(s,3H)

◎ 6-acetyl-3-(3-chloropropyl)-4,5,6,7-tetrahydro-2-methylthieno[2,3-c]pyridine
4. 66(d,2H), 4.00-3.30(m,4H), 2.50(b,4H), 2.33(s,3H), 2.15(s,3H), 1.50(m,2H)

◎ 6-acetyl-2-(2-chloropropyl)-4,5,6,7-tetrahydro-3-methylthieno[2,3-c]pyridine
4.60(d,2H), 4.00-3.40(m,4H), 3.15(t,2H), 2.50(b,4H), 2. 15(s,3H), 2.05(s,3H)

◎ 6-acetyl-3-(4-chloropropyl)-4,5,6,7-tetrahydro-2-methylthieno[2,3-c]pyridine
4.66(d,2H), 4.00-3.40(m,4H), 2.70-2.30(b,4H), 2.33(s,3H), 2.15(s,3H), 2.00-1.30(m,4H),

◎ 6-acetyl-2-(2-bromoethyl)-4,5,6,7-tetrahydrothieno[2,3-c]pyridine
6.70(s,1H), 4.60(d,2H), 4.00-3.00(m,6H), 2.66(b,2H), 2.10(s,3H)

◎ 6-acetyl-3-(2-chloroethyl)-4,5,6,7-tetrahydro-2-methylthieno[2,3-c]pyridine
4. 65(d,2H), 4.05-3.40(m + t,4H), 2.95(t,2H), 2.65(b,2H), 2.40(s,3H), 2.20(s,3H)

◎ 3,6-bisacetyl-2-(2-chloroethyl)-4,5,6,7-tetrahydro[2,3-c]pyridine
4. 66(d,2H), 4.00-3.50(m + t,4H), 3.40(t,2H), 2,85(b,2H), 2.50(s,3H), 2.15(s,3H)

◎ 6-acetyl-3-(2-chloroethyl)-4,5,6,7-tetrahydro-2-propylthieno[2,3-c]pyridine
4. 66(d,2H), 4.00-3.33(m + t,4H), 3.00-2.33(m,6H), 2.15(s,3H), 1.65(m,2H), 1.00(t,3H)

◎ 6-acetyl-3-chloroacetyl-4,5,6,7-tetrahydro-2-methylthieno[2,3-c]pyridine
4.75(d,2H), 4.50(s,2H), 3.75(t,2H), 2.65(t,2H), 2.50(s,3H),2.20(s,3H)

Example 24

A mixture of 5.9 g of 6-acetyl-3-(2-bromoethyl)-4,5,6,7-tetrahydro-2-methylthieno[2,3-c]pyridine, 5 g of 4-(6-fluoro-1,2-benzisoxazol-3-yl)piperidine hydrochloride, 6.7 g of potassium carbonate and 3.2 g of potassium iodide in 50ml of dimethylformamide and 50 ml of toluene was stirred at 50 °C for 7 hours and poured into water. The toluene layer was washed with water, dried over magnesium sulfate and concentrated. The residue was purified by column chromatography on a silica gel and crystallized from isopropyl ether. The resulting crystals were recrystallized from acetonitrile to give 6-acetyl-3-(2-(4-(6-fluoro-1,2-benzisoxazol-3-yl)piperidin-1-yl)ethyl)-4,5,6,7-tetrahydro-2-methylthieno[2,3-c]pyridine, m.p. 70-75 °C.

Example 25

A mixture of 4 g of 6-acetyl-3-(2-(4-(6-fluoro-1,2-benzisoxazol-3-yl)piperidin-1-yl)ethyl)-4,5,6,7-tetrahydro-2-methylthieno[2,3-c]pyridine in 4ml of conc. sulfuric acid and 80 ml of water was refluxed under heating for 3 hours. The mixture was poured into water, made alkaline and extracted with chloroform. The organic layer was washed with water, dried over magnesium sulfate and concentrated. To the residue was added isopropyl ether and the crystals were collected by filtration to give 3-(2-(4-(6-fluoro-1,2-benzisoxazol-3-yl)piperidin-1-yl)ethyl)-4,5,6,7-tetrahydro-2-methylthieno[2,3-c]pyridine, m.p. 88-90 °C.

Example 26

To a mixture of 0.5 g of 3-(2-(4-(6-fluoro-1,2-benzisoxazol-3-yl)piperidin-1-yl)ethyl)-4,5,6,7-tetrahydro-2-methylthieno[2,3-c]pyridine in 0.5 ml of triethylamine and 10 ml of chloroform was added 0.18g of benzoyl chloride under cooling and the mixture was stirred for an hour, and then poured into water. The organic layer was washed with water, dried and concentrated. The residue was purified by column chlomatogaphy to give 0.4 g of 6-benzoyl-3-(2-(4-(6-fluoro-1,2-benzisoxazol-3-yl)piperidin-1-yl) ethyl)-4,5,6,7-tetrahydro-2-methylthieno[2,3-c]pyridine as an oil, m.p. 192-195 °C as oxalate 1/4hydrate thereof.

Example 27

The reaction and procedure were conducted in a similar manner as in Example 24 using 0.48 g of 6-acetyl-3-(2-bromoethyl)-4,5,6,7-tetrahydro-2-methylothieno[2,3-c]pyridine and 0.5 g of 4-((6,7-dimethoxy-1,2-benzisoxazol-3-yl)methyl)piperidine hydrochloride to give 0.4 g of 6-acetyl-3-(2-(4-((6,7-dimethoxy-1,2-benzisoxazol-3-yl)methyl)piperidin-1-yl)ethyl)-4,5,6,7-tetrahydro-2-methylthieno[2,3-c]pyridine as an oil, m.p. 197-200 °C as oxalate thereof.

Example 28

The reaction and procedure were conducted in a similar manner as in Example 26 using 0.19 g of cyclohexanecarbonyl chloride in stead of benzoyl chloride to give 0.06g of 6-cyclohexylcarbonyl-3-(2-(4-(6-fluoro-1,2-benzisoxazol-3-yl)piperidin-1-yl)ethyl)-4,5,6,7-tetrahydro-2-methylthieno[2,3-c]pyridine as an oil, m.p. 205-208 ° C as oxalate 1/2hydrate thereof.

Example 29

The reaction and procedure were conducted in a similar manner as in Example 24 using 1.4 g of 6-acetyl-3-(4-chlorobutyryl)-4,5,6,7-tetrahydro-2-methylthieno[2,3-c]pyridine and 1.3 g of 4-(6-fluoro-1,2-benzisoxazol-3-yl)piperidine hydrochloride to give 1.4 g of 6-acetyl-3-(4-(4-(6-fluoro-1,2-benzisoxazol-3-yl)-piperidin-1-yl)butyryl)-4,5,6,7-tetrahydro-2-methylthieno[2,3-c]pyridine as an oil.

$^1$H-NMR($CDCl_3$) $\delta$:1.85-2.30(m,8H), 2.15(s,3H), 2.30-2.70(m,2H), 2.60(s,3H), 2.70-3.30(m,5H), 3.65-(m,4H), 4.60(d,2H), 7.05(dt,1H), 7.25(dd,1H), 7.70(dd,1H)

Example 30

The reaction and procedure were conducted in a similar manner as in Example 24 using 1.5 g of 6-acetyl-3-(4-chlorobutyryl-4,5,6,7-tetrahydro-2-ethylthieno[2,3-c]pyridine and 1.2 g of 4-(6-fluoro-1,2-benzisoxazol-3-yl)piperidine hydrochloride to give 0.7 g of 6-acetyl-3-(4-(4-(6-fluoro-1,2-benzisoxazol-3-yl)piperidin-1-yl)butyryl)-4,5,6,7-tetrahydro-2-ethylthieno[2,3-c]pyridine as an oil, m.p. 93-95 ° C as oxalate 1/4hydrate thereof.

Example 31

The reaction and procedure were conducted in a similar manner as in Example 24 using 4.0 g of 6-acetyl-3-(2-bromoethyl)-4,5,6,7-tetrahydrothieno[2,3-c]pyridine and 3.0 g of 4-(6-fluoro-1,2-benzisoxazol-3-yl)piperidine hydrochloride to give 3.3 g of 6-acetyl-2-(2-(4-(6-fluoro-1,2-benzisoxazol-3-yl)piperidin-1-yl)-ethyl)-4,5,6,7-tetrahydrothieno[2,3-c]pyridine as white crystals, m.p. 130-132 ° C.

Example 32

The reaction and procedure were conducted in a similar manner as in Example 24 using 2.5 g of 6-acetyl-3-(2-bromoethyl)-2-ethyl-4,5,6,7-tetrahydrothieno[2,3-c]pyridine and 2.0 g of 4-(6-fluoro-1,2-benzisoxazol-3-yl)piperidine hydrochloride to give 2.2 g of 6-acetyl-2-ethyl-3-(2-(4-(6-fluoro-1,2-benzisoxazol-3-yl)-piperidin-1-yl)ethyl)-4,5,6,7-tetrahydrothieno[2,3-c]pyridine as an oil, m.p. 191-201 ° C as oxalate 1/2hydrate thereof.

Example 33

The reaction and procedure were conducted in a similar manner as in Example 24 using 0.7 g of 5-acetyl-2-(2-bromoethyl)-4,5,6,7-tetrahydrothieno[2,3-c]pyridine and 0.61 g of 4-(6-fluoro-1,2-benzisoxazol-3-yl)piperidine hydrochloride to give 0.7 g of 5-acetyl-2-(2-(4-(6-fluoro-1,2-benzisoxazol-3-yl)piperidin-1-yl)-ethyl)-4,5,6,7-tetrahydrothieno[3,2-c]pyridine as an oil, m.p. 170-172 ° C as oxalate thereof.

Example 34

The reaction and procedure were conducted in a similar manner as in Example 24 using 0.7 g of 6-acetyl-3-(2-chloroethyl)-4,5,6,7-tetrahydro-2-methylthieno[2,3-c]pyridine and 0.6 g of 4-(bis(4-fluorophenyl)-methylene)piperidine to give 0.5 g of 6-acetyl-3-(2-(4-(bis(4-fluorophenyl)methylene)piperidin-1-yl)ethyl)-4,5,6,7-tetrahydro-2-methylthieno[2,3-c]pyridine as an oil, m.p. 209-211 ° C as axalate thereof.

Example 35

The reaction and procedure were conducted in a similar manner as in Example 24 using 1.0 g of 6-acetyl-3-chloroacetyl-4,5,6,7-tetrahydro-2-methylthieno[2,3-c]pyridine and 0.95 g of 4-(6-fluoro-1,2-benzisoxazol-3-yl)piperidine hydrochloride to give 0.9 g of 6-acetyl-3-(2-(4-(6-fluoro-1,2-benzisoxazol-3-yl)piperidin-1-yl)-1-oxoethyl)-4,5,6,7-tetrahydro-2-methylthieno[2,3-c]pyridine as an oil, m.p. 92-94 °C as 3/2oxalate 1/2hydrate thereof.

Example 36

The reaction and procedure were conducted in a similar manner as in Example 24 using 1.7 g of 6-acetyl-3-(2-chloroethyl)-4,5,6,7-tetrahydro-2-propylthieno[2,3-c]pyridine and 1.0 g of 4-(6-fluoro-1,2-benzisoxazol-3-yl)piperidine hydrochloride to give 0.7 g of 6-acetyl-2-propyl-3-(2-(4-(6-fluoro-1,2-benzisoxazol-3-yl)-piperidin-1-yl)ethyl)-4,5,6,7-tetrahydrothieno[2,3-c]pyridine as an oil, m.p. 178-180 °C (decomposition) as oxalate 1/2hydrate thereof.

Example 37

The reaction and procedure were conducted in a similar manner as in Example 24 using 1.0 g of 6-acetyl-3-(4-chlorobutyl)-4,5,6,7-tetrahydro-2-methylthieno[2,3-c]pyridine and 0.9 g of 4-(6-fluoro-1,2-benzisoxazol-3-yl)piperidine hydrochloride to give 0.5 g of 6-acetyl-3-(4-(4-(6-fluoro-1,2-benzisoxazol-3-yl)-piperidin-1-yl)butyl)-4,5,6,7-tetrahydro-2-methylthieno[2,3-c]pyridine as an oil, m.p. 158-160 °C as 3/2oxalate thereof.

Example 38

The reaction and procedure were conducted in a similar manner as in Example 24 using 2.0 g of 6-acetyl-(3-chloropropyl)-4,5,6,7-tetrahydro-2-methylthieno[2,3-c]pyridine and 2.0 g of 4-(6-fluoro-1,2-benzisoxazol-3-yl)piperidine hydrochloride to give 0.5 g of 6-acetyl-3-(3-(4-(6-fluoro-1,2-benzisoxazol-3-yl)piperidin-1-yl)propionyl)-4,5,6,7-tetrahydro-2-methylthieno[2,3-c]pyridine as an oil, m.p. 180-182 °C as oxalate thereof.

Examle 39

To a solution of 0.5 g of 3-(2-(4-(6-fluoro-1,2-benzisoxazol-3-yl)piperidin-1-yl)ethyl)-4,5,6,7-tetrahydro-2-methylthieno[2,3-c]pyridine hydrochloride in ethanol was added 0.3 ml of 37% formaldehyde solution and the mixture was stirred at room temperature for an hour. To the mixture was added 0.2 g of sodium cyanoborohydride, the mixture was stirred for an hour, and then concentrated. To the residue was added water and extracted with chloroform. The extract was washed with water, dried and concentrated in vacuo. The residue was purified by column chromatography and recrystallized from isopropyl ether to give 2,6-dimethyl-3-(2-(4-(6-fluoro-1,2-benzisoxazol-3-yl)piperidin-1-yl)ethyl)-4,5,6,7-tetrahydrothieno[2,3-c]pyridine 1/2hydrate as white crystals, m.p. 100-103 °C.

Example 40

A mixture of 0.8 g of 6-acetyl-3-(2-chloroethyl)-4,5,6,7-tetrahydro-2-methylthieno[2,3-c]pyridine and 20 ml of 16.8% dimethylamine-ethanol solution in a pressure bottle was stirred at 80 °C for 5 hours and then the solvent was distilled away. To the residue was added water and extracted with ethyl acetate. The extract was washed with water, dried and concentrated in vacuo to give 0.4 g of 6-acetyl-3-(2-dimethylaminoethyl)-4,5,6,7-tetrahydro-2-methylthieno[2,3-c]pyridine as an oil.

$^1$H-NMR($CDCl_3$) δ:2.17(s,3H), 2.30(s,6H), 2.40(s,3H), 2.50-2.80 (m,6H), 3. 80(m,2H), 4.66(d,2H)

Example 41

The reaction and procedure were conducted in a similar manner as in Example 40 using 2.3 g of 5-acetyl-2-(2-chloroethyl)-4,5,6,7-tetrahydrothieno [3,2-c]pyridine to give 5-acetyl-2-(2-dimethylaminoethyl)-4,5,6,7-tetrahydrothieno[3,2-c]pyridine hydrochloride 1/4hydrate, m.p. 201-203 °C (decomposition).

Example 42

The reaction and procedure were conducted in a similar manner as in Example 24 using 1.0 g of 6-acetyl-2-(2-chloroethyl)-4,5,6,7-tetrahydro-3-methylthieno[2,3-c]pyridine and 1.0 g of 4-(6-fluoro-1,2-benzisoxazol-3-yl)piperidine hydrochloride to give 6-acetyl-2-(2-(4-(6-fluoro-1,2-benzisoxazol-3-yl)piperidin-1-yl)-ethyl)-4,5,6,7-tetrahydro-3-methylthieno[2,3-c]pyridine as an oil.

$^1H$-NMR($CDCl_3$) δ:1.80-3.50(m,15H), 2.10(s,3H), 2.25(s,3H), 3.80(m,2H), 4.70(d,2H), 7.10(dt,1H), 7.30-(dd,1H), 7.75(dd,1H)

Example 43

The reaction and procedure were conducted in a similar manner as in Example 24 using 2.2 g of 6-acetyl-2-(4-chlorobutyryl)-4,5,6,7-tetrahydrothieno[2,3-c]pyridine and 2.0 g of 4-(6-fluoro-1,2-benzisoxazol-3-yl)piperidine hydrochloride to give 1.3 g of 6-acetyl-2-(4-(4-(6-fluoro-1,2-benzisoxazol-3-yl)piperidin-1-yl)-butyryl)-4,5,6,7-tetrahydrothieno[2,3-c]pyridine as white crystals, m.p. 145-147 °C.

Example 44

The reaction and procedure were conducted in a similar manner as in Example 26 using 0.12 g of propionyl chloride instead of benzoyl chloride to give 0.4 g of 3-(2-(4-(6-fluoro-1,2-benzisoxazol-3-yl)-piperidin-1-yl)ethyl)-4,5,6,7-tetrahydro-2-methyl-6-propionylthieno[2,3-c]pyridine as an oil, m.p. 192-195 °C (decomposition) as oxalate 1/2 hydrate thereof.

Example 45

The reaction and procedure were conducted in a similar manner as in Example 26 using 0.15 g of isobutyryl chloride instead of benzoyl chloride to give 0.4 g of 3-(2-(4-(6-fluoro-1,2-benzisoxazol-3-yl)-piperidin-1-yl)ethyl)-4,5,6,7-tetrahydro-6-isobutyryl-2-methylthieno[2,3-c]pyridine as art oil, m.p.200 °C (decomposition) as oxalate 1/2hydrate thereof.

Example 46

The reaction and procedure were conducted in a similar manner as in Example 26 using 0.14 g of cyclopropylcarbonyl chloride instead of benzoyl chloride to give 0.4 g of 6-cyclopropylcarbonyl-3-(2-(4-(6-fluoro-1,2-benzisoxazol-3-yl)piperidin-1-yl)ethyl)-4,5,6,7-tetrahydro-2-methylthieno[2,3-c]pyridine as an oil, m.p. 188-190 °C (decomposition) as oxalate 1/4hydrate thereof.

Example 47

The reaction and procedure were conducted in a similar manner as in Example 24 using 1.5 g of 3,6-diacetyl-2-(2-chloroethyl)-4,5,6,7-tetrahydrothieno[2,3-c]pyridine and 1.3 g of 4-(6-fluoro-1,2-benzisoxazol-3-yl)piperidine hydrochloride to give 3,6-diacetyl-2-(2-(4-(6-fluoro-1,2-benzisoxazol-3-yl)piperidin-1-yl)ethyl)-4,5,6,7-tetrahydrothieno[2,3-c] pyridine as an oil.

$^1H$-NMR($CDCl_3$) δ:1.80-2.60(m,7H), 2.20(s,3H), 2.50(s,3H), 2.60-3.40 (m,8H), 3.65(m,2H), 4.60(d,2H), 7.05(dt,1H), 7.25(dd,1H), 7.70(dd,1H)

Example 48

The reaction and procedure were conducted in a similar manner as in Example 24 using 2.0 g of 5-acetyl-3-(2-chloroethyl)-4,5,6,7-tetrahydro-2-methylthieno[3,2-c]pyridine and 1.0 g of 4-(6-fluoro-1,2-benzisoxazol-3-yl)piperidine hydrochloride to give 0.25 g of 5-acetyl-3-(2-(4-(6-fluoro-1,2-benzisoxazol-3-yl)-piperidin-1-yl)ethyl)-4,5,6,7-tetrahydro-2-methylthieno[3,2-c]pyridine as an oil.

$^1H$-NMR($CDCl_3$) δ:1.60-2.50(m,8H), 2.20(s,3H), 2.40(s,3H), 2.50-2.90 (m,4H), 3.16(m,3H), 3.80(m,2H), 4.50-(d,2H), 7.05(dt,1H), 7.25(dd,1H), 7.70(dd,1H)

Example 49

The reaction and procedure were conducted in a similar manner as in Example 24 using 1.4 g of 6-acetyl-3-(2-chloroethyl)-4,5,6,7-tetrahydro-2-methylthieno[2,3-c]pyridine and 0.9 g of 4-(1,2-benzisothiazol-3-yl)piperazine to give 1.0 g of 6-acetyl-3-(2-(4-(1,2-benzisothiazol-3-yl)piperazin-1-yl)ethyl)-4,5,6,7-tetrahydro-2-methylthieno[2,3-c]pyridine as an oil, m.p. 190-192 °C (decomposition) as oxalate thereof.

Example 50

The reaction and procedure were conducted in a similar manner as in Example 24 using 1.6 g of 5-acetyl-3-(4-chlorobutyryl)-4,5,6,7-tetrahydro-2-methylthieno[3,2-c]pyridine and 1.4 g of 4-(6-fluoro-1,2-benzisoxazol-3-yl)piperidine hydrochloride to give 0.6 g of 5-acetyl-3-(4-(4-(6-fluoro-1,2-benzisoxazol-3-yl)-piperidin-1-yl)butyryl)-4,5,6,7-tetrahydro-2-methylthieno[3,2-c]pyridine as an oil.

$^1$H-NMR($CDCl_3$) $\delta$:1.80-3.30(m,16H), 2.02(s,3H), 2.18(s,3H), 3.75(m,3H), 4.70(d,2H), 7.04(dd,2H), 7.70-(dd,1H)

Example 51

A mixture of 334 mg of 6-acetyl-3-(2-bromoethyl)-4,5,6,7-tetrahydro-2-methylthieno[2,3-c]pyridine, 420 mg of 4-(6-fluoro-1-(4-fluorophenyl)-1H-indazol-3-yl)piperidine hydrochloride, 500 mg of potassium carbonate and 216 mg of potassium iodide in 10 ml of dimethylformamide and 10 ml of toluene was stirred at 85°C for 8 hours and concentrated in vacuo. To the residue were added ethyl acetate and water, and separated. The ethyl acetate layer was washed with water, dried over magnesium sulfate and concentrated in vacuo. The residue was purified by column chromatography on a silica gel to give 360 mg of 6-acetyl-3-(2-(4-(6-fluoro-1-(4-fluorophenyl)-1H-indazol-3-yl)piperidin-1-yl)ethyl)-4,5,6,7-tetrahydro-2-methylthieno[2,3-c]pyridine as an oil, m.p. 193-196°C as oxalate thereof.

Example 52

A mixture of 334 mg of 6-acetyl-3-(2-bromoethyl)-4,5,6,7-tetrahydro-2-methylthieno[2,3-c]pyridine, 308 mg of 4-(5-fluoro-1,2-benzisoxazol-3-yl)piperidine hydrochloride, 500 mg of potassium carbonate and 220mg of potassium iodide in 10 ml of dimethylformamide and 10 ml of toluene was stirred at 90°C for 25 hours and concentrated in vacuo. To the residue were added ethyl acetate and water and separated. The ethyl acetate layer was washed with water, dried over magnesium sulfate and concentrated in vacuo. The residue was purified by column chromatography on a silica gel to give 100 mg of 6-acetyl-3-(2-(4-(5-fluoro-1,2-benzisoxazol-3-yl)piperidin-1-yl)ethyl)-4,5,6,7-tetrahydro-2-methylthieno[2,3-c]pyridine as an oil, m.p. 167-172°C as oxalate thereof.

Example 53

A mixture of 210 mg of 6-acetyl-3-(2-bromoethyl)-4,5,6,7-tetrahydro-2-methylthieno[2,3-c]pyridine, 200 mg of 4-(6-fluorobenzo(b)thiophen-3-yl)piperidine hydrochloride, 250 mg of potassium carbonate and 140 mg of potassium iodide in 5 ml of dimethylformamide and 5 ml of toluene was stirred at 90°C for 22 hours and concentrated in vacuo. To the residue were added ethyl acetate and water, and separated. The ethyl acetate layer was washed with water, dried over magnesium sulfate and concentrated in vacuo. The residue was purified by column chromatography on a silica gel to give 160 mg of 6-acetyl-3-(2-(4-(6-fluorobenzo(b)-thiophen-3-yl)piperidin-1-yl)ethyl)-4,5,6,7-tetrahydro-2-methylthieno[2,3-c]pyridine as an oil.

$^1$H-NMR($CDCl_3$) $\delta$:2.20(s,3H), 2.40(s,3H), 3.10(m,2H), 1.8-2.8 (m,13H), 3.75(m,2H), 4.60(d,2H), 7.02-(s,1H), 7.10(dt,1H), 7.50 (dd,1H), 7.70(dd,1H)
m.p. 223-225°C as oxalate thereof.

Example 54

A mixture of 514 mg of 6-acety-3-(2-bromoethyl)-4,5,6,7-tetrahydro-2-methylthieno[2,3-c]pyridine, 511 mg of 4-(6-fluoro-1H-indazol-3-yl)piperidine hydrochloride 690 mg of potassium carbonate and 365 mg of potassium iodide in 10 ml of dimethylformamide and 10 ml of toluene was stirred at 90°C for 22 hours and concentrated in vacuo. To the residue were added ethyl acetate and water, and separated. The ethyl acetate layer was washed with water, dried over magnesium sulfate and concentrated in vacuo. The residue was purified by column chromatography on a silica gel and recrystallized from the mixture of diisopropyl ether and isopropyl alcohol to give 170 mg of 6-acetyl-3-(2-(4-(6-fluoro-1H-indazol-3-yl)piperidin-1-yl)ethyl-4,5,6,7-tetrahydro-2-methylthieno[2,3-c]pyridine monohydrate, m.p. 116-119°C.

Example 55

A mixture of 590 mg of 6-acetyl-3-(2-bromoethyl)-4,5,6,7-tetrahydro-2-methylthieno[2,3-c]pyridine, 580 mg of 4-(6-fluoro-1-methyl-1H-indazol-3-yl) piperidine, 780 mg of potassium carbonate and 415 mg of potassium iodide in 10 ml of dimethylformamide and 10 ml of toluene was stirred at 90 °C for 22 hours and concentrated in vacuo. To the residue were added ethyl acetate and water, and separated. The ethyl acetate layer was washed with water, dried over magnesium sulfate and concentrated in vacuo. The residue was purified by column chromatography on a silica gel to give 540 mg of 6-acetyl-3-(2-(4-(6-fluoro-1-methyl-1H-indazol-3-yl)piperidin-1-yl) ethyl)-4,5,6,7-tetrahydro-2-methylthieno[2,3-c]pyridine as an oil, m.p. 213-216 °C as oxalate thereof.

Example 56

To a solution of 2 g of 4,5,6,7-tetrahydro-8H-thieno[2,3-c]azepin-8-one and 2 g of chloroacetyl chloride in 25 ml of dichloromethane was added 4.8 g of aluminum chloride. The mixture was stirred for 30 minutes, refluxed under heating for an hour and then poured into water. The precipitated crystals were collected by filtration and washed with isopropyl alcohol to give 2 g of 2-chloroacetyl-4,5,6,7-tetrahydro-8H-thieno[2,3-c]-azepin-8-one, m.p. 221-223 °C (decomposition).

Example 57

A mixture of 1.9 g of 2-chloroacetyl-4,5,6,7-tetrahydro-8H-thieno[2,3-c]azepin-8-one in 5.4 ml of triethylsilane and 15 ml of trifluoroacetic acid was stirred at 50 °C for 12 hours. The mixture was poured into water, alkalified and extracted with ethyl acetate. The exract was washed with water, dried and concentrated. The residue was purified by column chromatography on a silica gel to give 0.85 g of 2-chloroethyl-4,5,6,7-tetrahydro-8H-thieno[2,3-c]azepin-8-one as white crystals, m.p. 156-158 °C.

Example 58

A mixture of 800 mg of 2-chloroethyl-4,5,6,7-tetrahydro-8H-thieno[2,3-c]azepin-8-one, 920 mg of 4-(6-fluoro-1,2-benzisoxazol-3-yl)piperidine hydrochloride, 1.45 g of potassium carbonate and 580 mg of potassium iodide in 10 ml of dimethylformamide and 10 ml of toluene was stirred at 90 °C for 22 hours and concentrated in vacuo. To the residue were added ethyl acetate and water, and separated. The ethyl acetate layer was washed with water, dried over magnesium sulfate and concentrated in vacuo. The residue was purified by column chromatography on a silica gel and recrystallized from ethyl acetate to give 0.65 g of 2-(2-(4-(6-fluoro-1,2-benzisoxazol-3-yl)piperidin-1-yl)ethyl)-4,5,6,7-tetrahydro-8H-thieno[2,3-c]azepin-8-one, m.p. 192-194 °C.

Example 59

To a solution of 2 g of 4,5,6,7-tetrahydro-8H-thieno[2,3-c]azepin-8-one and 2.5 g of 4-chlorobutyryl chloride in 25 ml of dichloromethane was added 4.8 g of aluminum chloride under cooling. The mixture was stirred for 30 minutes, refluxed under heating and poured into water, and then extracted with chloroform. The extract was washed with water, dried over magnesium sulfate and concentrated. The residue was crystallized from ethyl acetate-isopropyl ether to give 1.2 g of 2-(4-chlorobutyryl)-4,5,6,7-tetrahydro-8H-thieno[2,3-c]azepin-8-one as white crystals, m.p. 163-165 °C.

Example 60

A mixture of 1.1 g of 2-(4-chlorobutyryl)-4,5,6,7-tetrahydro-8H-thieno[2,3-c]azepin-8-one, 1.1 g of 4-(6-fluoro-1,2-benzisoxazol-3-yl)piperidine hydrochloride, 1.68 g of potassium carbonate and 670 mg of potassium iodide in 10 ml of dimethylformamide and 10 ml of toluene was stirred at 90 °C for 22 hours and concentrated in vacuo. To the residue were added ethyl acetate and water, and separated. The ethyl acetate layer was washed with water, dried over magnesium sulfate and concentrated in vacuo. The residue was purified by column chromatography on a silica gel and recrystallized from ethyl acetate-isopropyl ether to give 0.55 g of 2-(4-(4-(6-fluoro-1,2-benzisoxazol-3-yl)piperidin-1-yl)butyryl)-4,5,6,7-tetrahydro-8H-thieno-[2,3-c]azepin-8-one 1/2hydrate, m.p. 186-187 °C.

Example 61

To a mixture of 6.7 g of 4,5,6,7-tetrahydro-8H-thieno[2,3-c]azepin-8-one and 4.5 g of sodium borohydride in 70 ml of tetrahydrofuran was added boron trifluoride-ether complex dropwise under ice-cooling and the mixture was refluxed under heating for an hour. The mixture was poured into water, washed with chloroform, made alkaline with 10% potassium hydroxide solution and extracted with chloroform. The extract was washed with water, dried and concentrated to give 4.1 g of 5,6,7,8-tetrahydro-4H-thieno[2,3-c]-azepine as an oil.

Example 62

To a mixture of 4 g of 5,6,7,8-tetrahydro-4H-thieno[2,3-c]azepine and 4 g of pyridine in 20 ml of chloroform was added 4.1 g of acetic anhydride under ice-cooling and the mixture was stirred at room temperature for an hour. The mixture was poured into water and extracted with chloroform. The extract was washed with water, dried and concentrated to give 4 g of 7-acetyl-5,6,7,8-tetrahydro-4H-theino[2,3-c]azepine as an oil.

Example 63

To a solution of 2 g of 7-acetyl-5,6,7,8-tetrahydro-4H-thieno[2,3-c]azepine and 1.7 g of chloroacetyl chloride in 25ml of dichloromethane was added 4.8 g of aluminum chloride under ice-cooling. The mixture was stirred for 30 minutes and refluxed under heating for an hour. The mixture was poured into water and extracted with chloroform. The extract was washed with water, dried over magnesium sulfate and concentrated to give 2.2 g of 7-acetyl-2-chloroacetyl-5,6,7,8-tetrahydro-4H-thieno[2,3-c] azepine as an oil.

Example 64

A mixture of 2.1 g of 7-acetyl-2-chloroacetyl-5,6,7,8-tetrahydro-4H-thieno[2,3-c]azepine in 5.4 ml of triethylsilane and 15 ml of trifluoroacetic acid was stirred at room temperature for 12 hours. The mixture was poured into water, made alkaline and extracted with ethyl acetate. The exract was washed with water, dried and concentrated. The residue was purified by column chromatography on a silica gel to give 1.8 g of 7-acetyl-2-chloroethyl-5,6,7,8-tetrahydro-4H-thieno[2,3-c]azepine as an oil.

Example 65

A mixture of 1.6 g of 7-acetyl-2-chloroethyl-5,6,7,8-tetrahydro-4H-thieno[2,3-c]azepine, 1.8 g of 4-(6-fluoro-1,2-benzisoxazol-3-yl)piperidine hydrochloride, 2.6 g of potassium carbonate and 1g of potassium iodide in 15 ml of dimethylformamide and 15 ml of toluene was stirred at 90 °C for 5 hours and concentrated in vacuo. To the residue were added ethyl acetate and water, and separated. The ethyl acetate layer was washed with water, dried over magnesium sulfate and concentrated in vacuo. The residue was purified by column chromatograhy on a silica gel to give 1.5 g of 7-acetyl-2-(2-(4-(6-fluoro-1,2-benzisoxazol-3-yl)piperidin-1-yl)ethyl)-5,6,7,8-tetrahydro-4H-thieno[2,3-c]azepine as an oil. The oil was treated with hydrochloric acid in isopropyl alcohol and recrystallized from ethanol-isopropyl ether to give 7-acetyl-2-(2-(4-(6-fluoro-1,2-benzisoxazol-3-yl)piperidin-1-yl)ethyl)-5,6,7,8-tetrahydro-4H-thieno[2,3-c]azepine hydrochloride 3/2hydrate, m.p. 210 °C (decomposition).

Example 66

To a solution of 2.5 g of 7-acetyl-5,6,7,8-tetrahydro-4H-thieno[2,3-c]azepine and 2.7 g of 4-chlorobutyryl chloride in 25 ml of dichloromethane was added 6.0 g of aluminum chloride under ice-cooling. The mixture was stirred for 30 minutes, poured into water and extracted with chloroform. The extract was washed with water, dried over magnesium sulfate and concentrated. The residue was purified by column chromatography on a silica gel to give 2.9 g of 7-acetyl-2-(4- chlorobutyryl)-5,6,7,8-tetrahydro-4H-thieno[2,3-c]azepine as an oil.

Example 67

A mixture of 2.8 g of 7-acetyl-2-(4-chlorobutyryl)-5,6,7,8-tetrahydro-4H-thieno[2,3-c]azepine, 2.6 g of 4-(6-fluoro-1,2-benzisoxazol-3-yl)piperidine hydrochloride, 3.9 g of potassium carbonate and 1.55 g of potassium iodide in 30 ml of dimethylformamide and 30 ml of toluene was stirred at 70°C for 20 hours and concentrated in vacuo. To the residue were added ethyl acetate and water, and separated. The ethyl acetate layer was washed with water, dried over magnesium sulfate and concentrated in vacuo. The residue was purified by column chromatography on a silica gel to give 3.0 g of 7-(acetyl-2-(4-(4-(6-fluoro-1,2-benzisoxazol-3-yl)piperidin-1-yl)butyryl)-5,6,7,8-tetrahydro-4H-thieno[2,3-c]azepine as an oil. The oil was treated with hydrochloric acid in isopropyl alcohol and recrystallized from ethanol-ethyl acetate to give 7-acetyl-2-(4-(4-(6-fluoro-1,2-benzisoxazol-3-yl)piperidin-1-yl)butyryl)-5,6,7,8-tetrahydro-4H-thieno[2,3-c]-azepine hydrochloride, m.p. 230°C (decomposition).

Example 68

The reaction and procedure were conducted in a similar manner as in Example 24 using 1.6 g of 5-acetyl-3-(4-chlorobutyryl)-4,5,6,7-tetrahydro-2-methylthieno[3,2-c]pyridine and 1.4 g of 4-(6-fluoro-1,2-benzisoxazol-3-yl)piperidine hydrochloride to give 0.6 g of 5-acetyl-3-(4-(4-(6-fluoro-1,2-benzisoxazol-3-yl)-piperidin-1-yl)butyryl)-4,5,6,7-tetrahydro-2-methylthieno[3,2-c]pyridine as an oil, m.p. 116-118°C as an oxalate monohydrate thereof.

Example 69

The reaction and procedure were conducted in a similar manner as in Example 14 using 1.0 g of 6-acetyl-3-(2-chloroethyl)-4,5,6,7-tetrahydro-2-methylthieno[2,3-c]pyridine and 0.88 g of 1-(1,2-benzoisothiazol-3-yl)piperazine to give 1.0 g of 6-acetyl-3-(2-(4-(1,2-benzisothiazol-3-yl)piperazin-1-yl)ethyl)-4,5,6,7-tetrahydro-2-methylthieno[2,3-c]pyridine as an oil, m.p. 190-192°C (decomposition) as an oxalate 1/2hydrate thereof.

Example 70

The reaction and procedure were conducted in a similar manner as in Example 24 using 0.9 g of 6-acetyl-2-(2-chloroethyl)-4,5,6,7-tetrahydrothieno[2,3-c]pyridine and 1.0 g of 4-(5-chloro-1,2-benzisoxazol-3-yl)piperidine hydrochloride to give 0.7 g of 6-acetyl-2-(2-(4-(5-chloro-1,2-benzisoxazol-3-yl)piperidin-1-yl)-ethyl)-4,5,6,7-tetrahydrothieno[2,3-c]pyridine as an oil, m.p. 122-124°C (decomposition) as a hydrochloride thereof.

Example 71

The reaction and procedure were conducted in a similar manner as in Example 24 using 0.2 g of 6-acetyl-3-(2-chloroethyl)-4,5,6,7-tetrahydro-2-methylthieno[2,3-c]pyridine and 0.2 g of 1-(6-chlorobenzothiazol-2-yl)piperazine to give 0.04 g of 6-acetyl-3-(2-(4-(6-chlorobenzothiazol-2-yl)piperazin-1-yl)ethyl)-4,5,6,7-tetrahydro-2-methylthieno[2,3-c]pyridineas an oil, m.p. 180°C (decomposition) as an oxalate thereof.

Example 72

The reaction and procedure were conducted in a similar manner as in Example 24 using 2.0 g of 5-acetyl-3-(2-chloroethyl)-4,5,6,7-tetrahydro-2-methylthieno[3,2-c]pyridine and 1.0 g of 4-(6-fluoro-1,2-benzisoxazol-3-yl)piperidine hydrochloride to give 0.25 g of 5-acetyl-3-(2-(4-(6-fluoro-1,2-benzisoxasol-3-yl)-piperidin-1-yl)ethyl)-4,5,6,7-tetrahydro-2-methylthieno[3,2-c]pyridine as an oil, m.p. 198-200°C (decomposition) as an oxalate thereof.

Example 73

The reaction and procedure were conducted in a similar manner as in Example 24 using 0.7 g of 6-acetyl-2-(2-chloroethyl)-3-ethyl-4,5,6,7-tetrahydrothieno[2,3-c]pyridine and 0.67 g of 4-(6-fluoro-1,2-benzisoxazol-3-yl)piperidine hydrochloride to give 0.5 g of 6-acetyl-2-(2-(4-(6-fluoro-1,2-benzisoxazol-3-yl)piperidin-1-yl)ethyl)-3-ethyl-4,5,6,7-tetrahydrothieno[2,3-c]pyridine as an oil, m.p. 165-167°C (decomposition) as hydrochloride dihydrate thereof.

Example 74

The reaction and procedure were conducted in a similar manner as in Example 24 using 1.8 g of 5-acetyl-2-(4-chlorobutyryl)-4,5,6,7-tetrahydrothieno[3,2-c]pyridine and 1.6 g of 4-(6-fluoro-1,2-benzisoxazol-3-yl)piperidine hydrochloride to give 1.0 g of 5-acetyl-2-(4-(4-(6-fluoro-1,2-benzisoxasol-3-yl)piperidin-1-yl)-butyryl)-4,5,6,7-tetrahydro thieno[3,2-c]pyridine as an oil, m.p. 235 ° C as hydrochloride 1/2hydrate thereof.

Example 75

The reaction and procedure were conducted in a similar manner as in Example 24 using 1.1 g of 2-(4-chlorobutyryl)-5,6,7,8-tetrahydro-4H-thieno[3,2-c]azepin-4-one and 1.0 g of 4-(6-fluoro-1,2-benzisoxazol-3-yl)piperidine hydrochloride to give 0.21 g of 2-(4-(4-(6-fluoro-1,2-benzisoxazol-3-yl)piperidin-1-yl)butyryl)-5,6,7,8-tetrahydro-4H-thieno[3,2-c]azepin-4-one, m.p. 142-143 ° C.

Example 76

To a solution of 0.4 g of 3-(2-(4-(6-fluoro-1,2-benzisoxazol-3-yl)piperidin-1-yl)acetyl)-2-methyl-5,6,7,8-tetrahydro-4H-thieno[3,2-c]azepin-4-one (m.p. 175-176 ° C as oxalate thereof) in 20 ml of methanol was added 0.5 g of sodium borohydride under cooling. The mixture was stirred for 10 minutes and concentrated. Water was added thereto and the solution was extracted with ethyl acetate. The extract was washed with water, dried and the solvent was distilled away. The residue was treated with fumaric acid in ethanol and recrystallized from ethanol to give 0.22 g of 3-(2-(4-(6-fluoro-1,2-benzisoxazol-3-yl)piperidin-1-yl)-1-hydroxyethyl)-2-methyl-5,6,7,8-tetrahydro-4H-thieno[3,2-c]azepin-4-one difumarate monohydrate, m.p. 116-118 ° C.

Examle 77

The reaction and procedure were conducted in a similar manner as in Example 24 using 0.5 g of 6-acetyl-3-bromo-2-(2-chloroethyl)-4,5,6,7-tetrahydrothieno[2,3-c]pyridine and 0.4 g of 4-(6-fluoro-1,2-benzisoxazol-3-yl)piperidine hydrochloride to give 0.05 g of 6-acetyl-3-bromo-2-(2-(4-(6-fluoro-1,2-benzisoxazol-3-yl)piperidin-1-yl)ethyl)-4,5,6,7-tetrahydrothieno[2,3-c]pyridine as an oil, m.p. 114-117 ° C as an oxalate thereof.

Example 78

The reaction and procedure were conducted in a similar manner as in Example 24 using 0.5 g of 2-(2-chloroethyl)-4,6,7,8-tetrahydro-5H-thieno[3,2-b]azepin-5-one and 0.5 g of 4-(6-fluorobenzo(b)thiophen-3-yl)-piperidine hydrochloride to give 0.2 g of 2-(2-(4-(6-fluorobenzo(b)thiophen-3-yl)piperidin-1-yl)ethyl)-4,6,7,8-tetrahydro-5H-thieno[3,2-b]azepin-5-one m.p. 191-193 ° C.

Example 79

The reaction and procedure are conducted in a similar manner as in Example 24 using 6-acetyl-3-(2-chloroethyl)-4,5,6,7-tetrahydro-2-ethylthieno[2,3-c]pyridine and 4-(6-fluorobenzo(b)thiophen-3-yl)piperidine hydrochloride to give 6-acetyl-3-(2-(4-(6-fluorobenzo(b)thiophen-3-yl)piperidin-1-yl)ethyl)-4,5,6,7-tetrahydro-2-ethylthieno[2,3-c]pyridine.

Example 80

The reaction and procedure are conducted in a similar manner as in Example 24 using 6-acetyl-2-(4-chlorobutyryl)-4,5,6,7-tetrahydrothieno[2,3-c]pyridine and 4-(6-fluorobenzo(b)thiophen-3-yl)piperidine hydrochloride to give 6-acetyl-2-(4-(4-(6-fluorobenzo(b)thiophen-3-yl)piperidin-1-yl)butyryl)-4,5,6,7-tetrahydrothieno[2,3-c]pyridine.

Example 81

The reaction and procedure are conducted in a similar manner as in Example 24 using 6-acetyl-2-(2-chloroethyl)-4,5,6,7-tetrahydro-3-methylthieno[2,3-c]pyridine and 4-(6-fluorobenzo(b)thiophen-3-yl)piperidine hydrochloride to give 6-acetyl-2-(2-(4-(6-fluorobenzo(b)thiophen-3-yl)piperidin-1-yl)ethyl)-4,5,6,7-tetrahydro-3-methylthieno[2,3-c]pyridine.

Formulation Example 1

Tablets containing 10 mg of a compound of the formula (I) are prepared in accordance with the following formulation:

| | |
|---|---|
| Compound of formula (I) | 10.0 mg |
| Lactose | 58.5 mg |
| Corn starch | 25.0 mg |
| Crystalline cellulose | 20.0 mg |
| Polyvinylpyrrolidone K-30 | 2.0 mg |
| Talc | 4.0 mg |
| Magnesium stearate | 0.5 mg |
| | 120.0 mg |

The compound of the formula (I) is pulverized by an atomizer into fine powders below 10 $\mu$ in average particle diameter, which are admixed with lactose, corn starch and crystalline cellulose sufficiently in a kneading machine, and further kneaded with polyvinylpyrrolidone paste. The kneaded mixture is passed through a sieve of 200 mesh, dried at 50 ° C and passed through a sieve of 24 mesh. Talc and magnesium stearate are mixed therewith and the mixture is compressed into 120.0 mg tablets with a punch of 8mm in diameter. These tablets are, if desired, subjected to sugar-coating or film-coating.

While the present invention has been adequately and sufficiently described in the foregoing specification including examples, the description can be changed or modified within the spirit and scope of this invention.

## Claims

**1.** A condensed thiophene compound of the formula:

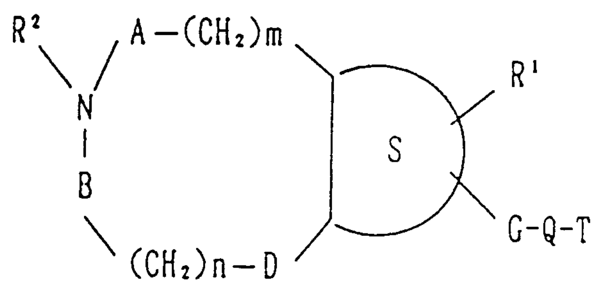

( I )

or a pharmaceutical acceptable salt thereof.

In the above formula, the ring S means following fused thiophenes.

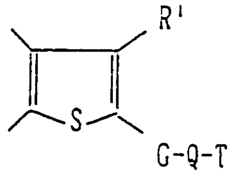
,
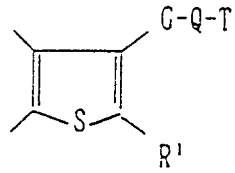

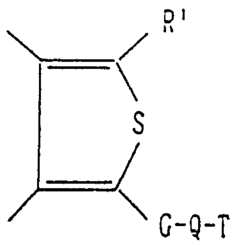
,
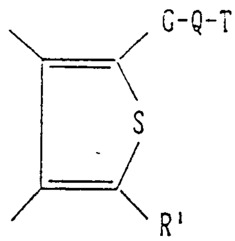

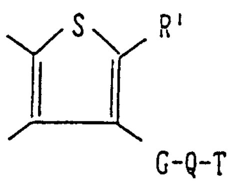
,
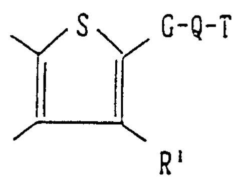

$R^1$ is hydrogen, halogen, nitro, amino, cyano, hydroxyl, formyl, alkyl, alkoxy, haloalkyl, arylalkyl, acyl, acyloxy, alkoxyalkyl, acyloxyalkyl, hydroxyalkyl, acyloxyalkanoyl, alkoxyalkanoyl, hydroxyalkanoyl, aryloxyalkanoyl, haloalkanoyl, alkylthio, alkylsulfinyl, alkylsulfonyl, arylthio, arylsulfinyl, arylsulfonyl, hydroxysulfonyl, halosulfonyl, sulfamoyl, substituted sulfamoyl, carboxyl, acylamino, alkoxycarbonyl, carbamoyl, substituted carbamoyl or substituted amino.

$R^2$ is hydrogen, alkyl, acyl, aryl or arylalkyl.

G is $-CH_2-$, $-CH(OR^3)-$ ($R^3$ is hydrogen, alkyl or acyl), -CO-, $-C(=NOR^4)-$ ($R^4$ is hydrogen, alkyl or acyl), $-CH(NH_2)-$ or -S(O)t- (t is 0, 1 or 2).

Q is straight alkylene or branched chain alkylene.

T is a primary amino, a secondary amino or a tertiary amino.

D is $-CH_2-$ or -S(O)u- (u is 0, 1 or 2).

A and B are (i) the same or different and each is carbonyl or thiocarbonyl, (ii) one is absent and the other is carbonyl or thiocarbonyl, or (iii) both absent.

When A, B are the case of above-mentioned (i) or (ii), m is 0 or 1, and n is 0, 1 or 2. When A, B are the case of above-mentioned (iii), m and n are the same or different and each is 0 or an integer of 1 to 4 with the proviso that m + n is an integer of below 4.

**2.** The compound of claim 1 or a pharmaceutically acceptable salt thereof, wherein T is a primary amino of $-NH_2$, a secondary amino of -NHRa (Ra is alkyl, cycloalkyl, arylalkyl or heteroarylalkyl), or a tertiary amino of -N(Rb)(Rc) [Rb and Rc are the same or different and each is alkyl, cycloalkyl, arylalkyl or heteroarylalkyl, or Rb and Rc together with the adjacent nitrogen atom form a cyclic amino of the formula:

(1)

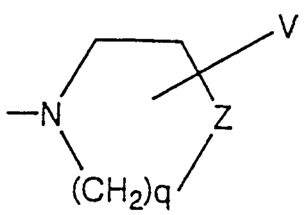

or

(2)

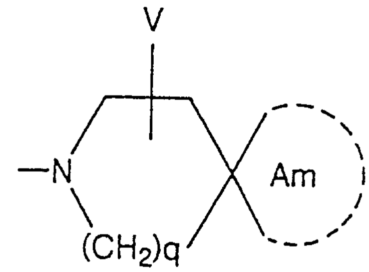

wherein q is an integer of 1 to 4, Z is methylene, oxygen atom, sulfur atom or N-$R^5$ ($R^5$ is hydrogen, carbamoyl, substituted carbamoyl, alkyl, cyanoalkyl, hydroxyalkyl, aryl, arylalkyl, alkoxycarbonyl, diarylalkyl, heteroaryl, heteroarylalkyl, cycloalkyl, cycloalkylalkyl, acyl, cinnamyl or adamantanemethyl), substituent V is hydrogen, hydroxyl, amino, carbamoyl, mono or di-substituted amino, cyclic amino, acyl, aryl, arylalkyl, arylalkylamino, alkyl, alkoxy, hydroxyalkyl, alkoxycarbonyl, heteroaryl, heteroarylalkyl, phenoxyalkyl, anilinoalkyl, alkylaminoalkyl, alkanoylaminoalkyl or bisarylmethylene and the number of V is 1 to 4. Cyclic amino of formula (1) may contain carbonyl group in the cycle and further may be condensed with aryl or heteroaryl. Ring Am of formula (2) contain amido bond in the cycle and further may contain oxygen atom, sulfur atom, carbonyl and/or N-$R^6$ ($R^6$ is hydrogen, alkyl or phenyl). Further, the ring Am can be condensed with 5 to 7-membered saturated or unsaturated ring.].

**3.** The compound of claim 1 or a pharmaceutically acceptable salt thereof, wherein $R^1$ is hydrogen, halogen, nitro, amino, alkyl, alkoxy, acyl, acyloxy, alkylthio, alkylsulfinyl, alkylsulfonyl or acylamino, G is $-CH_2-$, $-CH(OR^3)-$ ($R^3$ is hydrogen), -CO- or -S(O)t- ( t is 0, 1 or 2), T is a primary amino of $-NH_2$, a secondary amino of -NHRa (Ra is arylalkyl or heteroarylalkyl) or a tertiary amino of -N(Rb)(Rc) [Rb and Rc are the same or different and each is alkyl, arylalkyl or heteroarylalkyl, or Rb and Rc together with the adjacent nitrogen atom form a cyclic amino of the formula:

(1)

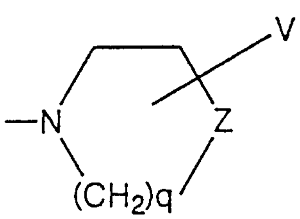

or

(2)

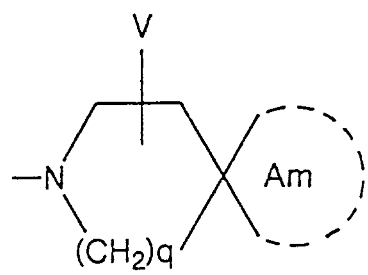

wherein q is an integer of 1 to 4, Z is methylene oxygen atom, sulfur atom or N-$R^5$ ($R^5$ is hydrogen, carbamoyl, substituted carbamoyl, alkyl, aryl, diarylalkyl, heteroaryl, heteroarylalkyl or acyl), substituent V is hydrogen, hydroxyl, heteroaryl, heteroarylalkyl or bisarylmethylene and the number of V is 1 to 4. Cyclic amino of formula (1) may contain carbonyl group in the cycle and further may be condensed with aryl or heteroaryl. Ring Am of formula (2) contain amido bond in the cycle and further may contain oxygen atom, sulfur atom, carbonyl and/or N-$R^6$ ($R^6$ is hydrogen, alkyl or phenyl). Further, the ring Am can be condensed with 5 to 7-membered saturated or unsaturated ring.].

**4.** The compound of claim 1 or a pharmaceutically acceptable salt thereof, wherein $R^1$ is hydrogen, halogen, alkyl or acyl, G is $-CH_2-$, $-CH(OR^3)-$($R^3$ is hydrogen), -CO- or -S(O)t- (t is 0, 1 or 2), T is a tertiary amino of -N(Rb)(Rc) [Rb and Rc are the same or different and each is alkyl, or Rb and Rc together with the adjacent nitrogen atom form a cyclic amino of the formula:

(1)

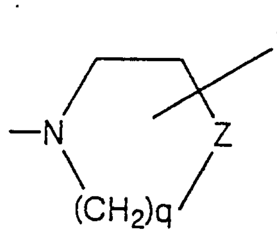

or

(2)

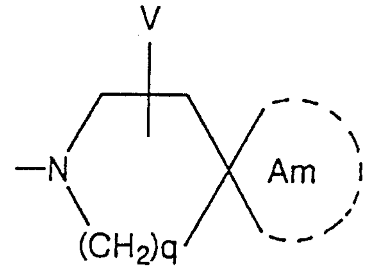

wherein q is an integer of 1 to 4, Z is methylene, or N-$R^5$ ($R^5$ is aryl, diarylalkyl, heteroaryl or heteroarylalkyl), substituent V is hydrogen, hydroxyl, heteroaryl, heteroarylalkyl or bisarylmethylene and the number of V is 1 to 4. Cyclic amino of formula (1) may contain carbonyl group in the cycle and further may be condensed with aryl or heteroaryl. Ring Am of formula (2) contain amido bond in the cycle and further may contain oxygen atom, sulfur atom, carbonyl and/or N-$R^6$ ($R^6$ is hydrogen, alkyl or phenyl). Further, the ring Am can be condensed with 5 to 7-membered saturated or unsaturated ring.].

**5.** The compound of claim 1 selected from the group consisting of:
2-(2-(4-(6-fluoro-1,2-benzisoxazol-3-yl)piperidin-1-yl)ethyl)-4,6,7,8-tetrahydro-5H-thieno[3,2-b]azepin-5-one,
3-(2-(4-(6-fluoro-1,2-benzisoxazol-3-yl)piperidin-1-yl)ethyl)-2-methyl-4,6,7,8-tetrahydro-5H-thieno[3,2-b]-azepin-5-one,
2-(2-(4-(6-fluoro-1,2-benzisoxazol-3-yl)piperidin-1-yl)ethyl)-4,5,6,7-tetrahydro-8H-thieno[2,3-c]azepin-8-one,
2-(4-(4-(6-fluoro-1,2-benzisoxasol-3-yl)piperidin-1-yl)butyryl)-4,5,6,7-tetrahydro-8H-thieno[2,3-c]azepin-8-one,
6-acetyl-3-(2-(4-(6-fluoro-1,2-benzisoxazol-3-yl)piperidin-1-yl)ethyl)-4,5,6,7-tetrahydro-2-methylthieno-[2,3-c]pyridine,
6-acetyl-2-ethyl-3-(2-(4-(6-fluoro-1,2-benzisoxasol-3-yl)piperidin-1-yl)ethyl)-4,5,6,7-tetrahydrothieno[2,3-c]pyridine,
6-acetyl-2-(2-(4-(6-fluoro-1,2-benzisoxazol-3-yl)piperidin-1-yl)ethyl)-4,5,6,7-tetrahydro-3-methylthieno-[2,3-c]pyridine,
6-acetyl-2-(4-(4-(6-fluoro-1,2-benzisoxazol-3-yl)piperidin-1-yl)butyryl)-4,5,6,7-tetrahydrothieno[2,3-c]-pyridine,
3-(2-(4-(6-fluoro-1,2-benzisoxazol-3-yl)piperidin-1-yl)ethyl)-4,5,6,7-tetrahydro-2-methyl-6-propionylthieno-[2,3-c]pyridine,
6-cyclopropylcarbonyl-3-(2-(4-(6-fluoro-1,2-benzisoxazol-3-yl)piperidin-1-yl)ethyl)-4,5,6,7-tetrahydro-2-methylthieno[2,3-c]pyridine,
3, 6-diacetyl-2-(2-(4-(6-fluoro-1,2-benzisoxazol-3-yl)piperidin-1-yl)ethyl)-4,5,6,7-tetrahydrothieno[2,3-c]-pyridine, and
6-acetyl-3-(2-(4-(6-fluorobenzo(b)thiophen-3-yl)piperidin-1-yl)ethyl)-4,5,6,7-tetrahydro-2-methylthieno-[2,3-c]pyridine
or a pharmaceutically acceptable salt thereof.

**6.** A condensed thiophene compound of the formula:

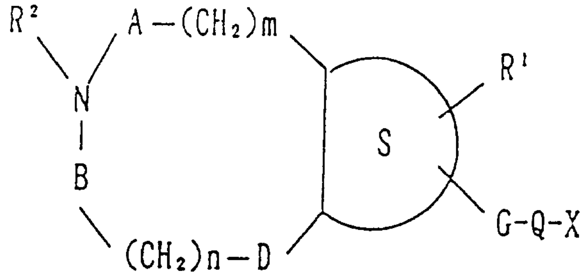

(II)

wherein X is hydroxyl, reactive atom or group derived from hydroxyl (halogen, methanesulfonyloxy, p-toluenesulfonyloxy and so on), and other symbols are as defined in claim 1.

**7.** A pharmaceutical composition comprising a compound of claim 1 and pharmaceutical additives.

**8.** An antipsychotic drug containing a compound of claim 1 as an effective ingredient.

**9.** An antianxietic drug containing a compound of claim 1 as an effective ingredient.

**INTERNATIONAL SEARCH REPORT**

International application No.
PCT/JP92/01695

A. CLASSIFICATION OF SUBJECT MATTER

Int. Cl$^5$ C07D495/04, A61K31/395

According to International Patent Classification (IPC) or to both national classification and IPC

B. FIELDS SEARCHED

Minimum documentation searched (classification system followed by classification symbols)

Int. Cl$^5$ C07D495/00-495/04, A61K31/395-31/405, 31/435-31/465, 31/54, 31/55

Documentation searched other than minimum documentation to the extent that such documents are included in the fields searched

Electronic data base consulted during the international search (name of data base and, where practicable, search terms used)

CAS ONLINE

C. DOCUMENTS CONSIDERED TO BE RELEVANT

| Category* | Citation of document, with indication, where appropriate, of the relevant passages | Relevant to claim No. |
|---|---|---|
| A | JP, A, 60-161979 (BASF AG), August 23, 1985 (23. 08. 85), & EP, A, 150034 | 1-9 |
| A | JP, A, 57-150687 (Dr. Karl Thomae GmbH), September 17, 1982 (17. 09. 82), & EP, A, 58341 | 1-9 |
| A | JP, A, 48-57994 (Yoshitomi Pharmaceutical Industries, Ltd.), August 14, 1973 (14. 08. 73), (Family: none) | 1-9 |
| A | JP, B1, 46-39350 (Yoshitomi Pharmaceutical Industries, Ltd.), November 19, 1971 (19. 11. 71), (Family: none) | 1-9 |

[X] Further documents are listed in the continuation of Box C. [ ] See patent family annex.

* Special categories of cited documents:

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| March 30, 1993 (30. 03. 93) | April 20, 1993 (20. 04. 93) |

| Name and mailing address of the ISA/ | Authorized officer |
|---|---|
| Japanese Patent Office | |
| Facsimile No. | Telephone No. |